# DEMANDE DE BREVET EUROPEEN

(11) **EP 1 394 159 A1**
(43) Date de publication de la demande: **03.03.2004**
(21) Numéro de dépôt: 02292037.5
(22) Date de dépôt: 13.08.2002
(51) Int. Cl.: C07D 333/38, A61K 31/38, C07D 409/10, C07D 491/10, C07D 413/10, C07D 409/12, C07D 417/10

(54) **Nouveaux dérivés de thiophène, leur procédé de préparation et les compositions pharmaceutiques qui les contiennent**

(71) Demandeur: Warner-Lambert Company LLC, Morris Plains, New Jersey 07950 (US)
(72) Inventeur: Dublanchet, Anne-Claude, 92160 Antony (FR); Compere, Delphine, 75015 Paris (FR); Cluzeau, Philippe, 91380 Chilly Mazarin (FR); Blais, Stéphane, 91120 Palaiseau (FR)
(74) Mandataire: Hirsch, Denise

(57) **Abrégé**

Composés de formule (I) : 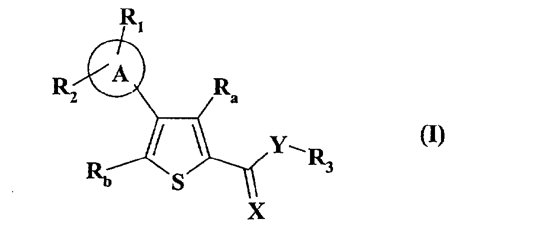 caractérisés en ce que :
- X représente oxygène ou soufre,
- Y représente oxygène,-NH ou -N(C₁-C₆)alkyle,
- Rₐ représente hydrogène, halogène, (C₁-C₃)alkyle, hydroxy, ou (C₁-C₃)alkoxy,
- R_{b} représente hydrogène, halogène, ou (C₁-C₃)alkyle,
- A représente phényle, (C₅-C₆)cycloalkyle ou (C₅-C₆)cycloalkènyle,
- R₁ et R₂ représentent chacun un groupement choisi parmi hydrogène, halogène, cyano, nitro, halogénoalkyle, halogénoalkoxy, alkyle, alkènyle, alkynyle, -OR₄, -NR₄R₅, - S(O)ₙR₄, -C(O)R₄, -CO₂R₄, -O-C(O)R₄, -C(O)NR₄R₅, -NR₅-C(O)R₄, -NR₅-SO₂R₄, -T-CN, -T-OR₄, -T-OCF₃, -T-NR₄R₅, -T-S(O)ₙR₄, -T-C(O)R₄, -T-CO₂R₄, -T-O-C(O)R₄, -T-C(O)NR₄R₅, -T-NR₄-C(O)R₅, -T-NR₄-SO₂R₅, -R₆, et -T-R₆ dans lesquels n, T, R₄, R₅ et R₆ sont tels que définis dans la description,
- R₃ représente un groupement -R₇ ou -U-R₁₁, dans lesquels R₇ représente hydrogène, alkyle, aryle, cycloalkyle ou hétérocycle, U représente une chaîne alkylène linéaire ou ramifiée, et R₁₁ est tel que défini dans la description,
leurs isomères optiques ou leurs sels d'addition à un acide ou à une base pharmaceutiquement acceptable,
et leur utilisation en tant qu'inhibiteur de la métalloprotéinase-12.

## Description

La présente invention concerne de nouveaux dérivés de thiophène, leur procédé de préparation et les compositions pharmaceutiques qui les contiennent.

Les composés de la présente invention sont particulièrement intéressants d'un point de vue pharmacologique pour leur interaction spécifique avec les métalloprotéinases et plus spécifiquement avec la macrophage métalloélastase-12 (MMP-12), trouvant leur application dans la prévention et le traitement des pathologies respiratoires telles que les maladies pulmonaires obstructives chroniques (COPD), l'emphysème, les bronchites chroniques, les inflammations pulmonaires chroniques, l'asthme, la fibrose cystique, la détresse respiratoire aigüe (ARDS), les allergies respiratoires dont la rhinite allergique, ainsi que les maladies liées à la production de TNFα incluant les maladies pulmonaires fibrotiques sévères, la sarcoïdose pulmonaire, et la silicose.

Les métalloprotéinases (MMPs) constituent une large famille de proteinases dégradant la matrice extracellulaire et sont sécrétées notamment par les cellules mésenchymales, les macrophages et les leucocytes polynucléaires. Les métalloprotéinases sont classées en plusieurs sous-familles selon leur structure primaire et leur spécificité. Ces familles incluent notamment les collagénases (MMP-1, MMP-8 et MMP-13), les stromélysines (MMP-3 et MMP-10), les gélatinases (MMP-2 et MMP-9), la matrilysine (MMP-7), la macrophage métalloélastase (MMP-12) ainsi que les MMPs de type membranaire (MMP-14, MMP-15, MMP-16 et MMP-17).

Les MMPs sont des zinc-métalloprotéinases ayant la capacité de dégrader la quasi-totalité des composants de la matrice extracellulaire, c'est-à-dire l'interstitium et les membranes basales. Une synthèse accrue de ces enzymes est retrouvée dans de nombreuses maladies destructrices (arthrite inflammatoire, athérosclérose, invasion tumorale, angiogénèse). Les MMPs (en particulier celles ayant une puissante activité élastolytique) sont impliquées dans la physiopathologie de l'asthme et des broncho-pneumopathies chroniques obstructives dont l'emphysème pulmonaire lié au tabac (BPCO).

L'élastase macrophagique humaine (HME ou MMP-12) présente toutes les caractéristiques des autres MMPs. Elle dégrade de nombreuses macromolécules de la matrice extracellulaire (gélatine, fibronectine et laminine) et surtout l'élastine. La MMP-12 n'est pas synthétisée par les cellules monocytaires circulantes, mais uniquement par les macrophages ou encore les monocytes différenciés *in vitro* en macrophages. La pathologie de l'emphysème est caractérisée par la destruction de l'élastine présente dans les parois des alvéoles pulmonaires. La mise en évidence de l'augmentation du taux de MMP-12 lors de la manifestation de cette pathologie, suggère ainsi un rôle prédominant de cette enzyme dans la survenue et le développement de cette maladie. De même des études ont démontré l'absence de développement d'emphysème chez des souris déficientes en MMP-12, ces souris étant exposées longuement à la fumée de cigarette *(Science* **1997**, 277, 2002-2004.). Plus récemment, en utilisant également des souris déficientes en MMP-12, dans un modèle d'asthme, un groupe a suggéré l'implication de la MMP-12 dans le développement de l'asthme chronique *(FASEB,* **2002**, 16, A590). Ces résultats démontrent clairement que des inhibiteurs de l'élastase macrophagique humaine (MMP-12) pourraient être très utiles pour la prévention et le traitement de pathologies respiratoires chroniques telles que les maladies pulmonaires obstructives chroniques (BPCO), l'emphysème, les bronchites chroniques, les inflammations pulmonaires chroniques, mais également les pathologies respiratoires dûes à un phénomène d'inflammation telles que l'asthme, la mucoviscidose, la détresse respiratoire aigüe (ARDS), les allergies respiratoires dont la rhinite allergique, ainsi que les maladies liées à la production de TNFα incluant les maladies pulmonaires fibrotiques sévères, la sarcoïdose pulmonaire, et la silicose.

Toutes les métalloprotéinases présentent un domaine catalytique constitué de 162 à 173 acides aminés contenant le site actif de l'enzyme. Un ion Zn²⁺ est présent dans le site actif auquel il est fixé par l'intermédiaire de résidus histidines. Ce site constitue l'un des points d'ancrage privilégié des inhibiteurs synthétiques des MMPs car il permet notamment de créer un centre de chélation stable, puissant et aisément accessible pour les petites molécules. Ainsi tous les inhibteurs puissants décrits dans la littérature possèdent une fonction chimique telle qu'un acide hydroxamique permettant une chélation entre l'atome de zinc du site catalytique de la métalloprotéinase et ledit inhibiteur. Cette chélation assure un blocage du site actif et entraîne l'inhibition de ladite enzyme.

L'un des problèmes majeurs de ce type d'inhibition est l'absence de sélectivité ou le faible taux de sélectivité, toutes les MMPs possédant en effet un ion zinc au sein de leur site actif. Le second problème lié à ces inhibiteurs puissants mais généralement faiblement sélectif est la toxicité (dont des problèmes de pharmacocinétiques) liée à la présence d'une fonction chimique telle qu'un acide hydroxamique.

L'un des objets de l'invention est donc de fournir de nouveaux composés possédant des propriétés inhibitrices de la métalloprotéinases de type 12 (MMP-12). Une solution a été trouvée par l'élaboration de nouveaux dérivés thiophène, ainsi que par l'utilisation desdits composés dans des compositions pharmaceutiques aptes à être utilisées dans la prévention et le traitement des pathologies liées à une inhibition de la MMP-12.

Plusieurs demandes de brevets ou articles scientifiques décrivent des composés comportant un motif central thiophène. Parmi cette littérature il peut être cité la demande de brevet WO 98/23605 qui décrit des dérivés thién-2-yl-carboxamides substitués en position 4 par un système cyclique et en position 5 par un groupement trifluorométhyle. Ces composés sont revendiqués pour leur activité bactéricide et fongicide. La demande de brevet WO 96/16954 décrit également des composés comportant éventuellement un système 4-arylthién-2-yl carboxamide dans lequel la fonction amide peut être substituée par un groupement phényle, utiles pour leur propriété anti-fongiques.

La demande de brevet WO 01/06821 revendique des composés utiles pour le traitement des pathologies psychotiques. Ces composés, qui constituent des agonistes des récepteurs nicotiniques à acétylcholine, peuvent notamment présenter un motif central thièn-2-yl-carboxamide dans lequel la fonction amide est substituée par un groupement 1-azabicyclo[2,2,2]oct-3-yl. Il peut également être cité la demande de brevet JP 63175853 ou l'article *Chem. Commun.* **2001**, 8, 759-760, qui décrivent des composés comportant un groupement thiophène substitué, ces composés constituant des photo-régulateurs de fluorescence ou des révélateurs photographiques.

Aucun de ces documents ne décrit ou ne suggère pour ces composés une activité inhibitrice de la MMP-12 et une utilisation potentielle de ce type de produits dans le traitement de pathologies respiratoires, propriété originale des composés revendiqués par la demanderesse.

Plus particulièrement, la présente invention concerne les composés de formule (I): 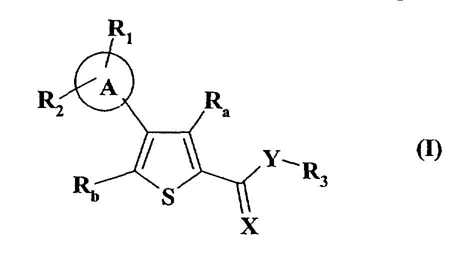 dans laquelle :
- X représente un atome d'oxygène ou un atome de soufre,
- Y représente un atome d'oxygène, un groupement -NH ou un groupement -N(C₁-C₆)alkyle,
- Rₐ représente un groupement choisi parmi hydrogène, halogène, (C₁-C₃)alkyle, hydroxy, et (C₁-C₃)alkoxy,
- R_{b} représente un groupement choisi parmi hydrogène, halogène, et (C₁-C₃)alkyle,
- A représente un groupement choisi parmi phényle, (C₅-C₆)cycloalkyle et (C₅-C₆)cycloalkènyle,
- R₁ et R₂, identiques ou différents, indépendamment l'un de l'autre, représentent chacun un groupement choisi parmi :
   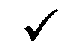 hydrogène, halogène, cyano, nitro, halogéno(C₁-C₆)alkyle, halogéno(C₁-C₆)alkoxy, (C₁-C₆)alkyle, (C₂-C₆)alkènyle, (C₂-C₆)alkynyle,
   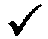 -OR₄, -NR₄R₅, -S(O)ₙR₄, -C(O)R₄, -CO₂R₄, -O-C(O)R₄, -C(O)NR₄R₅, -NR₅-C(O)R₄, -NR₅-SO₂R₄, -T-CN, -T-OR₄, -T-OCF₃, -T-NR₄R₅, -T-S(O)ₙR₄, -T-C(O)R₄, -T-CO₂R₄, -T-O-C(O)R₄, -T-C(O)NR₄R₅, -T-NR₄-C(O)R₅, -T-NR₄-SO₂R₅, -R₆, et -T-R₆ dans lesquels :
      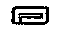 n représente un entier compris entre 0 et 2 inclus,
      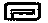 T représente une chaîne (C₁-C₆)alkylène linéaire ou ramifiée, éventuellement substituée par un groupement choisi parmi oxo, atome d'halogène, (C₁-C₆)alkoxy, hydroxy, amino, mono(C₁-C₆)alkylamino et di(C₁-C₆)alkylamino, et/ou dans laquelle éventuellement un des atomes de carbone est remplacé par un atome d'oxygène, un atome de soufre, un groupement -NH-, ou -N(C₁-C₆)alkyle-, (étant entendu que dans le cas où un des atomes de carbone est remplacé par un groupement tel que défini précédemment alors ladite chaîne alkylène comporte au moins un enchaînement de deux atomes)
      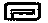 R₄ représente un atome d'hydrogène, un groupement (C₁-C₆)alkyle, aryle, cycloalkyle, ou un hétérocycle,
      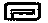 R₅ représente un atome d'hydrogène ou un groupement (C₁-C₆)alkyle,
      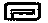 R₆ représente un groupement choisi parmi aryle, cycloalkyle, et un hétérocyle, chacun de ces groupements étant éventuellement substitués par un à cinq groupements, identiques ou différents, indépendamment l'un de l'autre choisis parmi halogène, cyano, nitro, halogéno(C₁ -C₆)alkyle, halogéno(C₁-C₆)alkoxy, (C₁-C₆)alkyle, (C₁-C₆)alkènyle, -OR_{40,} -NR₄₀R_{50,} -S(O)ₙ₁R₄₀, -C(O)R_{40,} - CO₂R₄₀, -O-C(O)R_{40,} -C(O)NR₄₀R_{50,} -NR₅₀-C(O)R_{40,} -NR₅₀-SO₂R_{40,} -T₁-CN, - T₁-OR₄₀, -T₁-OCF₃, -T₁-NR₄₀R_{50,} -T₁-S(O)ₙR₄₀, -T₁-C(O)R₄₀, -T₁-CO₂R_{40,} - T₁-O-C(O)R₄₀, -T₁-C(O)NR₄₀R₅₀, -T₁-NR₅₀-C(O)R₄₀, -T₁-NR₅₀-SO₂R_{40,} -G₁ et - T₁-G₁ dans lesquels :
         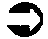 R_{40,} R_{50,} T₁ et n₁ ont respectivement les mêmes significations que R_{4,} R_{5,} T et n tels que définis précédemment,
         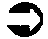 G₁ représente un groupement choisi parmi aryle, cycloalkyle, et un hétérocycle, chacun de ces groupements étant éventuellement substitués par 1 à 5 groupements, identiques ou différents, indépendamment l'un de l'autre choisis parmi halogène, cyano, nitro, halogéno(C₁-C₆)alkyle, halogéno(C₁-C₆)alkoxy, (C₁-C₆)alkyle, hydroxy, (C₁-C₆)alkoxy, phénoxy, benzyloxy, amino, mono(C₁-C₆)alkylamino, di(C₁-C₆)alkylamino, mercapto, (C₁-C₆)alkylthio, (C₁-C₇)acyle, (C₁-C₆)alkylsulfoxyde, carboxy, (C₁-C₆)alkoxycarbonyl, phényl et un hétérocycle,
- R₃ représente un groupement -R₇ ou -U-R₁₁, dans lesquels :
   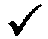 R₇ représente un groupement choisi parmi hydrogène, (C₁-C₆)alkyle, aryle, cycloalkyle et hétérocycle, chaque système cyclique étant éventuellement substitué par un à cinq groupements, identiques ou différents, indépendamment l'un de l'autre choisis parmi halogène, cyano, nitro, halogéno(C₁-C₆)alkyle, halogéno(C₁-C₆)alkoxy, (C₁-C₆)alkyle, -OR_{8,} -NR₈R_{9,} -S(O)ₘR_{8,} -C(O)R_{8,} -CO₂R_{8,} -O-C(O)R_{8,} -C(O)NR₈R_{9,} -NR₈-C(O)R_{9,} -NR₈-SO₂R_{9,} -V-CN, -V-OR_{8,} -V-NR₈R_{9,} - V-S(O)ₘR_{8,} -V-C(O)R_{8,} -V-CO₂R_{8,} -V-O-C(O)R_{8,} -V-C(O)NR₈R_{9,} -V-NR₈-C(O)R₉, -V-NR₈-SO₂R_{9,} -R_{10,} et -V-R₁₀ dans lesquels :
      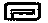 m représente un entier compris entre 0 et 2 inclus,
      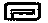 V représente une chaîne (C₁-C₆)alkylène linéaire ou ramifiée, une chaîne (C₂-C₆)alkènylène linéaire ou ramifiée, un groupement cyclopropylène ou une chaîne (C₂-C₆)alkylène linéaire ou ramifié dans laquelle un des atomes de carbone est remplacé par un atome d'oxygène, un atome de soufre, un groupement -NH-, ou -N(C₁-C₆)alkyl-,
      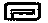 R₈ représente un atome d'hydrogène, un groupement (C₁-C₆)alkyle, aryle, cycloalkyle ou un hétérocycle,
      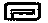 R₉ représente un atome d'hydrogène ou un groupement (C₁-C₆)alkyle,
      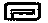 R₁₀ représente un groupement aryle, cycloalkyle ou un hétérocyle,
   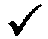 U représente une chaîne (C₁-C₆)alkylène linéaire ou ramifiée, ou (C₂-C₆)alkylène linéaire ou ramifiée dans laquelle un des atomes de carbone est remplacé par un atome d'oxygène, un atome de soufre, un groupement -NH-, ou -N(C₁-C₆)alkyle,
   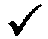 R₁₁ représente un groupement choisi parmi halogène, -OR₁₂, -NR₁₂R_{13,} -S(O)ₚR_{12,} -C(O)R_{12,} -CO₂R_{12,} -O-C(O)R_{12,} -C(O)NR₁₂R_{13,} -NR₁₃-C(O)R_{12,} -NR₁₃-SO₂R_{12,} et - R₁₄ ce dernier groupement étant éventuellement substitué par un ou deux groupements, identiques ou différents, indépendamment l'un de l'autre choisis parmi halogène, cyano, nitro, halogéno(C₁-C₆)alkyle, halogéno(C₁-C₆)alkoxy, (C₁-C₆)alkyle, (C₂-C₆)alkènyle, (C₂-C₆)alkynyle. -OR₁₅, -NR₁₅R₁₆, -S(O)_{q}R₁₅, -C(O)R₁₅, -O-C(O)R₁₅, -CO₂R_{15,} -C(O)NR₁₅R_{16,} -NR₁₆-C(O)R₁₅, -NR₁₆-SO₂R₁₅, - R₁₇, -W-CN, -W-OR₁₅, -W-NR₁₅R_{16,} -W-S(O)_{q}R₁₅, -W-C(O)R_{15,} -W-CO₂R₁₅, -W-O-C(O)R_{15,} -W-C(O)NR₁₅R_{16,} -W-NR₁₆-C(O)R_{15,} -W-NR₁₆-SO₂R₁₅, -W-R₁₇, et -C(O)-W₁-CO₂R_{15,} dans
      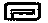 R₁₂ représente un atome d'hydrogène ou un groupement (C₁-C₆)alkyle, aryle, cycloalkyle, ou un hétérocycle
      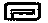 R₁₃ représente un atome d'hydrogène ou un groupement (C₁-C₆)alkyle,
      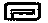 R₁₄ représente un groupement aryle, cycloalkyle ou un hétérocycle,
      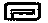 R₁₅ représente un atome d'hydrogène ou un groupement (C₁-C₆)alkyle, aryle, cycloalkyle, ou un hétérocycle,
      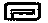 R₁₆ représente un atome d'hydrogène ou un groupement (C₁-C₆)alkyle,
      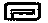 R₁₇ représente un groupement aryle, cycloalkyle ou un hétérocycle,
      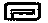 p représente un entier compris entre 0 et 2 inclus,
      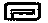 q représente un entier compris entre 0 et 2 inclus,
      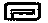 W représente une chaîne (C₁-C₆)alkylène linéaire ou ramifiée, une chaîne (C₂-C₆)alkènylène linéaire ou ramifiée, un groupement cyclopropylène, ou (C₂-C₆)alkylène linéaire ou ramifié dans laquelle un des atomes de carbone est remplacé par un atome d'oxygène, un atome de soufre, un groupement -NH-, ou -N(C₁-C₆)alkyle-,
      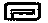 W₁ représente une chaîne (C₁-C₆)alkylène linéaire ou ramifiée,
leurs isomères, ainsi que leurs sels d'addition à un acide ou à une base pharmaceutiquement acceptable,
étant entendu que les composés de formule (I) ne sont pas choisis parmi le :
■ 5-méthyl-4-phényl-thiophèn-2-yl carboxylate d'éthyle,
■ acide 5-méthyl-4-phényl-thiophèn-2-yl carboxylique,
■ 3-hydroxy-4-phényl-thiophèn-2-yl-carboxylate de méthyle,
■ 3-méthoxy-4-phényl-thiophèn-2-yl-carboxylate de méthyle,
■ acide 3-méthoxy-4-phényl-thiophèn-2-yl-carboxylique,
■ 4-(4-méthoxyphényl)-thiophèn-2-yl-carboxylate de méthyle,
■ 4-phényl-thiophèn-2-yl-carboxylate de méthyle,
■ acide 4-(4-méthoxyphényl)-thiophèn-2-yl-carboxylique,
■ acide 4-phényl-thiophèn-2-yl-carboxylique,
■ acide 4-(4-*tert*-butylphényl)-thiophèn-2-yl-carboxylique,
■ 5-chloro-3-hydroxy-4-phényl-thiophèn-2-yl-carboxylate de méthyle,
■ acide 4-(3,5-diméthyl-phényl)-thiophèn-2-yl-carboxylique,
■ 4-[(4-acétylamino)-phényl]-thiophèn-2-yl-carboxylate de méthyle,
■ 4-phényl-thiophèn-2-yl-carboxylate d'éthyle,
■ 4-phényl-thiophèn-2-yl-carboxamide,
■ *N*-méthyl 4-phényl-thiophèn-2-yl-carboxamide,
■ *N,N*-diméthyl 4-phényl-thiophèn-2-yl-carboxamide,
■ 5-méthyl-4-phényl-thiophèn-2-yl-carboxamide,
■ *N*-méthyl 5-méthyl-4-phényl-thiophèn-2-yl-carboxamide,
■ *N,N*-diméthyl 5-méthyl-4-phényl-thiophèn-2-yl-carboxamide,
■ 2-{[4-(4-méthoxy-phényl)-thiophèn-2-yl]-carboxamido}benzoate de méthyle,
■ et le *N*-[3-(trifluorométhyl)phényl] 4-(4-méthoxyphényl)-thiophèn-2-yl-carboxamide,
étant entendu également que :
- si Rₐ représente un atome d'hydrogène, A représente un groupement cyclopentèn-1-yl substitué en position 2 par un groupement R₁ prenant la définition thiényle éventuellement substitué alors R_{b} représente un groupement choisi parmi hydrogène, halogène, et (C₂-C₃)alkyle,
- si R₃ représente un groupement R₇ prenant la définition hétérocycle alors ledit hétérocycle ne peut pas représenter un groupement 1-azabicyclo[2,2,2]oct-3-yl,
- et si R₃ représente un groupement R₇ prenant la définition phényle substitué en position *para* par un groupement R₁₀ alors ledit groupement R₁₀ ne peut pas représenter un groupement 5-méthyl-4,5-dihydro-3-oxo-2*H*-pyridazin-6-yle,
étant entendu aussi que dans la définition générale des différents groupements des composés de formule (I):
- par groupement aryle on comprend un système monocyclique ou bicyclique aromatique comprenant de 4 à 10 atomes de carbone, étant compris que dans le cas d'un système bicyclique l'un des cycles présente un caractère aromatique et l'autre cycle est aromatique ou insaturé ; à titre indicatif il peut être cité les groupements suivants : phényle, naphthyle, indényle, benzocyclobutènyle,...
- par groupement cycloalkyle on comprend un système monocyclique ou bicyclique fusionné ou ponté, saturé ou partiellement insaturé, comprenant de 3 à 12 atomes de carbone; à titre indicatif il peut être cité les groupements suivants: cyclopropyle, cyclobutyle, cyclopentyle, cyclohexyle, cyclooctyle, cycloheptyle, adamantyle, décalinyle, norbornyle, cyclopentènyle, cyclohexènyl, cyclohexèndiyl, ...
- par un groupement hétérocycle on comprend un système monocyclique ou bicyclique fusionné ou ponté, de 3 à 12 chaînons, saturé, insaturé ou aromatique, comprenant de 1 à 4 hétéroatomes, identiques ou différents, choisis parmi oxygène, soufre et azote, et contenant éventuellement 1 ou 2 groupements oxo ou thioxo, étant compris que dans le cas d'un système bicyclique l'un des cycles peut présenter un caractère aromatique et l'autre cycle est aromatique ou insaturé, ou les deux cycles sont saturés, ou l'un des cyles est saturé et l'autre cycle est insaturé, ou les deux cycles sont insaturés ; à titre indicatif il peut être cité les groupements suivants : furyle, thiènyle, pyrrolyle, pyrazolyle, pyridyle, pyrimidyle, pyrazinyle, benzofuryle, benzothiènyle, indolyle, quinolyle, isoquinolyle, imidazolyle, benzodioxolyle, benzodioxinyle, benzo[1,2,5]thiadiazolyle, benzo[1,2,5]oxadiazolyle, [1,2,3]triazolyle, [1,2,4]triazolyle,...
- par groupement (C₁-C₆)alkyle on comprend une chaîne carbonée linéaire ou ramifiée comprenant de 1 à 6 atomes de carbone; à titre indicatif il peut être cité les groupements suivants: méthyle, éthyle, propyle, isopropyle, tert-butyle, néopentyle, hexyle,...
- par groupement (C₂-C₆)alkènyle on comprend une chaîne carbonée linéaire ou ramifiée comprenant de 2 à 6 atomes de carbone et une ou plusieurs double liaisons ; à titre indicatif il peut être cité les groupements suivants : vinyle, allyle, 3-butèn-1-yl, 2-méthyl-butèn-1-yl, hexènyl,...
- par groupement (C₂-C₆)alkynyl on comprend une chaîne carbonée linéaire ou ramifiée comprenant de 2 à 6 atomes de carbone et une ou plusieurs triples liaisons ; à titre indicatif il peut être cité les groupements suivants : éthynyle, propynyle, 3-butyn-1-yl, 2-méthyl-butyn-1-yl, hexynyl,...
- par groupement (C₁-C₆)alkoxy on comprend un groupement alkyle tel que défini précédemment lié au travers d'un atome d'oxygène ; à titre indicatif il peut être cité les groupements suivants : méthoxy, éthoxy, *n*-propyloxy, *tert*-butyloxy,...
- par groupement halogéno(C₁-C₆)alkyle on comprend une chaîne carbonée linéaire ou ramifiée comprenant de 1 à 6 atomes de carbone et substituée par 1 à 6 atomes d'halogène; à titre indicatif il peut être cité les groupements suivants : trifluorométhyle, 2,2,2-trifluoroéthyle,...
- par groupement halogéno(C₁-C₆)alkoxy on comprend une chaîne carbonée linéaire ou ramifiée comprenant de 1 à 6 atomes de carbone et substituée par 1 à 6 atomes d'halogène, ladite chaîne étant reliée au composé de formule (I) par un atome d'oxygène ; à titre indicatif il peut être cité les groupements suivants : trifluorométhoxy, 2,2,2-trifluoroéthoxy, ...
   par atome d'halogène on comprend un atome choisi parmi brome, chlore, fluor et iode,
- par groupement (C₁-C₇)acyle on comprend un atome d'hydrogène, un groupement alkyle tel que défini précédemment ou un groupement phényle lié au travers d'un groupement oxo ; à titre indicatif il peut être cité les groupements suivants : formyle, acétyle, éthylcarbonyle, benzoyle,...
- les isomères optiques se réfèrent aux racémates, énantiomères et diastéréoisomères.

Selon une variante avantageuse de l'invention, les composés préférés de l'invention sont les composés de formule (LA.) : 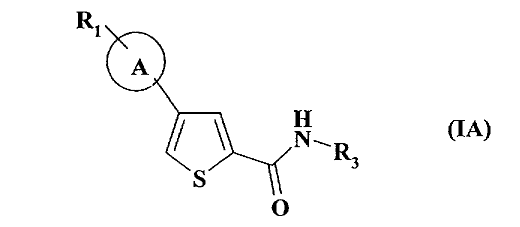 dans laquelle A, R₁, et R₃ sont tels que définis dans la formule (1).

D'une façon avantageuse A représente préférentiellement un groupement phényle dans les composés de formule (I) ou de formule (IA).

Selon une variante particulièrement intéressante de l'invention, ledit groupement A prenant la définition phényle est substitué par un groupement R₁ tel que défini dans la formule (I) situé en position *para*.

Selon une autre variante intéressante de l'invention, ledit groupement A prenant la définition phényle est substitué par un groupement R₁ tel que défini dans la formule (I) situé en position *ortho* ou en position *méta.*

Les groupements R₁ préférés selon l'invention sont les groupements choisis parmi trifluorométhyle, trifluorométhoxy, (C₁-C₆)alkyle, -OR₄, -SR₄, -NR₄R₅, -CO₂R₄, -O-C(O)R₄, -T-CO₂R₄ et -R₆ dans lesquels :
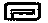 R₄ représente un atome d'hydrogène, un groupement (C₁-C₆)alkyle, aryle, cycloalkyle ou un hétérocycle,
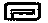 R₅ représente un atome d'hydrogène ou un groupement (C₁-C₆)alkyle,
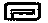 R₆ représente un groupement choisi parmi aryle, cycloalkyle, et un hétérocyle, chacun de ces groupements étant éventuellement substitués par un groupement choisi parmi halogène, cyano, nitro, halogéno(C₁-C₆)alkyle, halogéno(C₁-C₆)alkoxy, (C₁-C₆)alkyle, -OR₄₀, -NR₄₀R_{50,} -S(O)ₙ₁R_{40,} -C(O)R_{40,} -CO₂R_{40,} -O-C(O)R_{40,} -C(O)NR₄₀R_{50,} -NR₅₀-C(O)R_{40,} et -NR₅₀-SO₂R₄₀ dans lesquels R_{40,} R_{50,} et n1 sont tels que définis dans la formule (I),
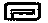 T est tel que défini dans la formule (I).

Selon une variante de l'invention les composés de formule (I) sont des composés dans lesquels A représente un groupement phényle substitué en position *ortho* ou en position *méta* par un groupement R₁ choisi parmi trifluorométhyle, trifluorométhoxy, (C₁-C₆)alkyle, - OR₄, et -NR₄R₅ dans lesquels :
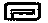 R₄ représente un atome d'hydrogène ou un groupement (C₁-C₃)alkyle,
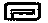 R₅ représente un atome d'hydrogène ou un groupement (C₁-C₃)alkyle.

D'une façon préférée R₁ représente un groupement choisi parmi (C₂-C₄)alkyle, (C₁-C₄)alkylthio, (C₁-C₄)alkoxy, trifluorométhoxy, et -R₆ dans lequel R₆ représente un groupement choisi parmi phényle, (C₅-C₆)cycloalkyle saturé ou partiellement insaturé, et un hétérocycle à 5 ou 6 chaînons comprenant de 1 à 3 hétéroatomes choisis parmi oxygène, azote et soufre, chacun desdits groupement R₆ étant éventuellement substitué par un groupement tel que défini dans la formule (I).

Selon une autre variante particulièrement avantageuse de l'invention, R₁ représente un groupement choisi parmi :
- phényle éventuellement substitué par un groupement choisi parmi atome d'halogène, hydroxy, (C₁-C₄)alkoxy linéaire ou ramifié, (C₁-C₄)acyle, (C₁-C₄)alkylsulfone, -CO₂R₄₀ et -C(O)NR₄₀R₅₀ dans lesquels R₄₀ et R₅₀ sont tels que définis dans la formule (I),
- cyclohexyle,
- 4-pyridyle, 3-pyridyle, N-pyrrolydinyle, 1-méthylpyrrol-3-yl, et 3,6-dihydro-2H-pyridin-1-yl.

Selon un autre mode préféré de l'invention les composés de formule (I) sont des composés dans lesquels R₁ représente un groupement cyclohexyle éventuellement substitué par un groupement choisi parmi hydroxy, (C₁-C₄)alkoxy linéaire ou ramifié, (C₁-C₄)acyle, (C₁-C₄)alkylsulfone, (C₁-C₄)alkyle, amino, (C₁-C₄)alkylamino et di(C₁-C₄)alkylamino.

D'une façon avantageuse R₃ représente un groupement R₇ choisi parmi phényle, cyclohexyle, et pyridyle, chacun de ces groupements étant éventuellement substitué par un ou deux groupements, identiques ou différents, indépendamment l'un de l'autre choisis parmi (C₁-C₆)alkyle, -OR_{8,} -NR₈R_{9,} -CO₂R_{8,} -V-OR_{8,} -V-NR₈R₉ et -V-CO₂R₈ dans lesquels V représente une chaîne (C₁-C₄)alkylène linéaire ou ramifiée ou une chaîne (C₂-C₄)alkènylène linéaire ou ramifiée, R₈ représente un atome d'hydrogène ou un groupement (C₁-C₆)alkyle, et R₉ représente un atome d'hydrogène.

Selon une variante préférée de l'invention, R₃ représente un groupement -U-R₁₁ dans lequel U représente une chaîne (C₁-C₄)alkylène linéaire et R₁₁ représente un groupement choisi parmi -CO₂R₁₂ et -R₁₄ dans lesquels :
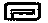 R₁₂ représente un atome d'hydrogène ou un groupement (C₁-C₆)alkyle,
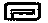 R₁₄ représente un groupement choisi parmi phényle, cyclohexyle, morpholinyle, et pyridyle chacun de ces groupements étant éventuellement substitué par un ou deux groupements, identiques ou différents, indépendamment l'un de l'autre choisis parmi halogène, (C₁-C₆)alkyle, -CO₂R₁₅, et -W-CO₂R₁₅, dans lesquels R₁₅ représente un atome d'hydrogène ou un groupement (C₁-C₆)alkyle, et W représente une chaîne (C₁-C₆)alkylène linéaire ou ramifiée ou une chaîne (C₂-C₆)alkènylène linéaire ou ramifiée.

Les isomères ainsi que les sels d'addition à un acide ou à une base pharmaceutiquement acceptable des variantes et des composés préférés font partie intégrante de l'invention.

L'invention concerne aussi les sels pharmaceutiquement acceptables des composés de formule (I). Une revue des sels pharmaceutiquement acceptables est notamment décrite dans *J. Pharm. Sci.,* **1977**, 66, 1-19.

Les acides pharmaceutiquement acceptables signifient les acides non toxiques organiques ou minéraux. Parmi les acides pharmaceutiquement acceptables, on peut citer à titre non limitatif les acides chlorhydrique, bromhydrique, sulfurique, phosphonique, nitrique, acétique, trifluoroacétique, lactique, pyruvique, malonique, succinique, glutarique, fumarique, tartrique, maléïque, citrique, ascorbique, oxalique, méthane sulfonique, camphorique, benzoique, toluènesulfonique, etc...

Les bases pharmaceutiquement acceptables signifient les bases non toxiques organiques ou minérales.

Parmi les bases pharmaceutiquement acceptables, on peut citer à titre non limitatif l'hydroxyde de sodium, l'hydroxyde de potassium, l'hydroxyde de lithium, l'hydroxyde de calcium, la triéthylamine, la tertbutylamine, la 2-diéthylaminoéthanol, l'éthanolamine, l'éthylènediamine, la dibenzyléthylènediamine, la piperidine, la pyrrolidine, la morpholine, la piperazine, la benzylamine, l'arginine, la lysine, l'histidine, la glucamine, la glucosamine, les hydroxides d'ammonium quaternaire, etc...

D'une façon générale on comprend par isomères des composés de l'invention, les isomères optiques tels que les énantiomères et les diastéréoisomères. Plus particulièrement, les formes énantiomères pures des composés de l'invention peuvent être séparées à partir des mélanges d'énantiomères qui sont mis à réagir avec un agent libérable de dédoublement des racémiques, ledit agent existant quant à lui sous la forme d'un énantiomère pur, permettant d'obtenir les diastéréoisomères correspondants. Ces diastéréoisomères sont ensuite séparés selon les techniques de séparation bien connues de l'homme de l'art, telles que la cristallisation ou la chromatographie, puis l'agent de dédoublement est éliminé en utilisant les techniques classiques de la chimie organique, pour conduire à l'obtention d'un énantiomère pur. D'une autre façon les formes énantiomères pures des composés de l'invention peuvent être séparées par chromatographie sur colonne chirale.

Les composés de l'invention qui sont présents sous la forme d'un mélange de diastéréoisomères, sont isolés sous forme pure par l'utilisation des techniques classiques de séparation telles que les chromatographies.

Dans certains cas particuliers, le procédé de séparation des composés de l'invention peut conduire à la formation prédominante d'un énantiomère ou d'un diastéréoisomère par rapport à l'autre.

L'invention s'étend également au procédé de préparation des composés de formule (I). Plus particulièrement les composés de formule (I) peuvent être obtenus à partir des composés de formule (II): 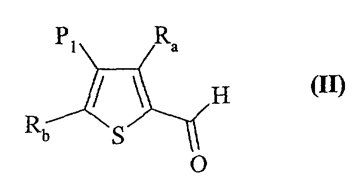 dans laquelle Rₐ et R_{b} sont tels que définis dans la formule (I) et P₁ représente un atome d'halogène ou un groupement triflate,
composés de formule (II) qui sont soumis à des conditions d'oxydations en présence, par exemple, de nitrate d'argent en milieu basique et polaire, pour donner les composés de formule (III) : 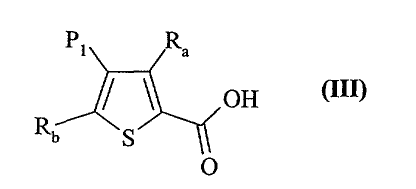 dans laquelle Rₐ, R_{b} et P₁ sont tels que définis précédemment,
composés de formule (III) qui sont éventuellement transformés en chlorures d'acide correspondant (IV) par action du chlorure d'oxalyle par exemple, 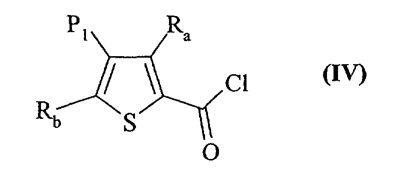 dans laquelle Rₐ, R_{b} et P₁ sont tels que définis précédemment,
ou qui sont traités directement, dans des conditions de couplage peptidique en présence par exemple de HATU et dans un milieu basique et polaire, avec un composé de formule (V) :

HY-R₃ (V)

dans laquelle Y et R₃ ont les mêmes significations que dans les composés de formule (I), pour conduire aux composés de (VI): 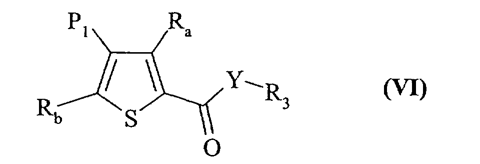 dans laquelle Rₐ, R_{b}, R₃, Y et P₁ sont tels que définis précédemment,
composés de formule (VI) qui sont :
- soit mis à réagir, dans des conditions basiques de couplage au palladium, avec un composé de formule (VII) : 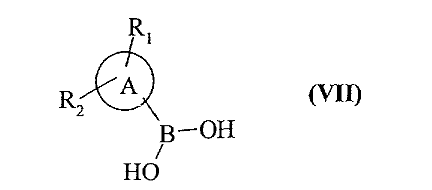
dans laquelle A, R₁ et R₂ ont les mêmes significations que dans les composés de formule (I),
pour conduire aux composés de formule (I/a), cas particulier des composés de formule (I): 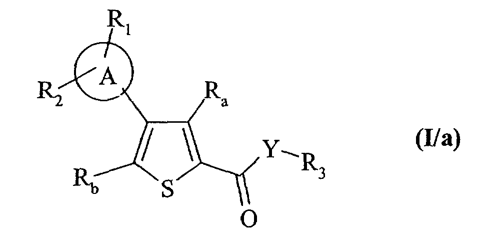 dans laquelle Rₐ, R_{b}, R₁, R₂, R₃, Y et A sont tels que définis précédemment,
- soit traités avec de l'hexaméthyldiétain, en présence d'un catalyseur au palladium, pour conduire aux composés de formule (VIII) : 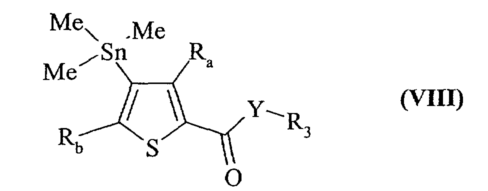
dans laquelle Rₐ, R_{b}, R₃ et Y sont tels que définis précédemment,
composés de formule (VIII) qui sont mis à réagir :
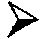 avec un composé de formule (IX) : 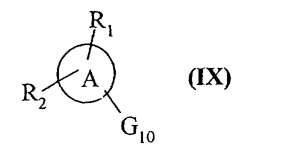
dans laquelle R₁, R₂ et A ont les mêmes significations que dans la formule (I) et G₁₀ représentent un atome d'halogène choisi parmi chlore et brome ou un groupement triflate,
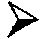 soit en présence de triphénylphosphine arsenic et d'un catalyseur au palladium, dans le cas où G₁₀ représente un groupement triflate,
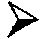 soit en présence d'un composé halogénocuivrique tel que CuBr₂ et d'un catalyseur au palladium, dans des conditions de solvant polaire, dans le cas où G₁₀ représente un atome d'halogène,
pour conduire également aux composés de formule (I/a) tels que décrits précédemment,
- soit traités par du bis(pmacolato)di-borane suivi par une réaction d'oxydation par action par exemple de periodate de sodium, pour conduire aux composés de formule (VIa): 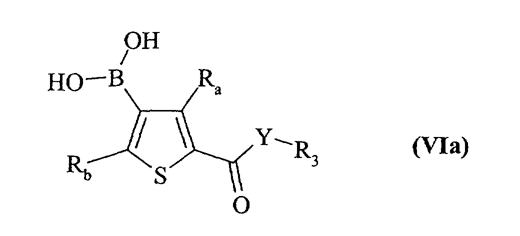
dans laquelle Rₐ, R_{b}, R₃ et Y sont tels que définis précédemment,
composés de formule (VIa) qui est mis à réagir dans des conditions basiques de couplage au palladium avec un composé de formule (IX) : 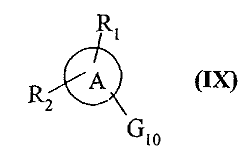 dans laquelle A, R_{1,} R₂ et G₁₀ sont tels que définis précédemment,
pour conduire également aux composés de formule (I/a), tel que défini précédemment : 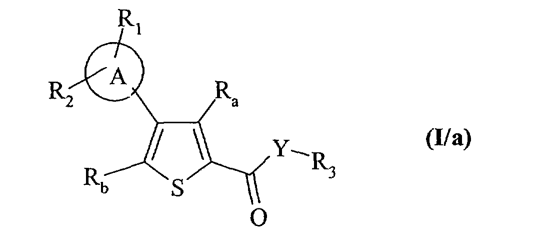 dans laquelle R_{a,} R_{b,} R₁, R_{2,} R_{3,} Y et A sont tels que définis précédemment,
- soit mis à réagir avec un composé de formule (IXa), (lesdits composés de formule (IXa) étant obtenus par traitement des composés de formule (IX) tels que définis précédemment, avec de l'hexaméthyldiétain en présence d'un catalyseur au palladium) : 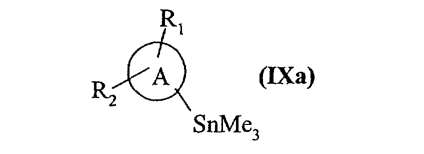
dans laquelle A, R₁, et R₂ sont tels que définis dans la formule (I),
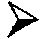 soit en présence de triphénylphosphine arsenic et d'un catalyseur au palladium, dans le cas où P₁ dans les composés de formule (VI) représente un groupement triflate,
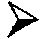 soit en présence d'un composé halogénocuivrique tel que CuBr₂ et d'un catalyseur au palladium, dans des conditions de solvant polaire, dans le cas où P₁ dans les composés de formule (VI) représente un atome d'halogène,
pour conduire également aux composés de formule (I/a) tels que décrits précédemment, 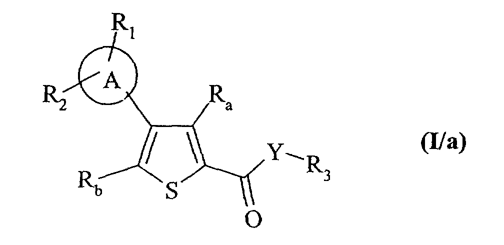 dans laquelle R_{a,} R_{b,} R_{1,} R_{2,} R_{3,} Y et A sont tels que définis précédemment,
composés de formule (I/a) dans le cas particulier où ils représentent des composés de formule (I/b) dans lesquels A représente un groupement phényle, R₂ représente un atome d'hydrogène, R₁ représente un groupement hydroxy ou un atome d'halogène (Hal) et R_{a,} R_{b,} Y et R₃ ont la même signification que dans la formule (I): 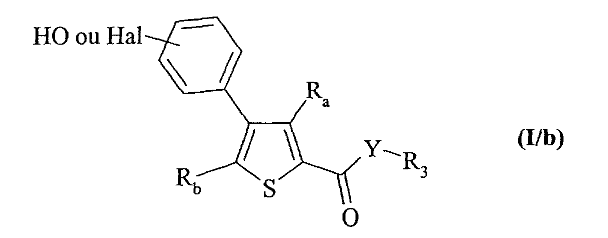 composés de formule (I/b) qui peuvent alors être traités préalablement par de l'anhydride trifluorométhanesulfonique en présence d'une base forte, dans le cas où R₁ représente un groupement hydroxy, pour obtenir le dérivé activé phényltriflate,
lesdits composés portant un groupement R₁ prenant la définition halogène ou triflate pouvant alors :
o soit être mis à réagir en condition basique et en présence d'un catalyseur au palladium avec un composé de formule (X): 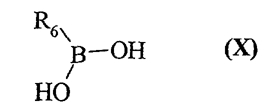
dans laquelle R₆ est tel que défini dans la formule (I), c'est à dire qu'il représente un groupement choisi parmi aryle, cycloalkyle, et un hétérocycle, et dans le cas présent R₆ représente préférentiellement un groupement aryle,
pour conduire aux composés de formule (I/c), cas particulier des composés de formule (I): 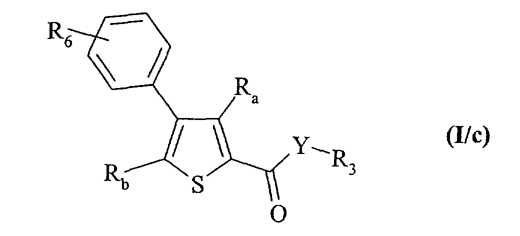 dans lesquels R₆,Rₐ, R_{b}, Y et R₃ sont tels que définis précédemment,
o soit être traités par du bis(pinacolato)di-borane suivi par une réaction d'oxydation par action par exemple de periodate de sodium, pour conduire aux composés de formule (XI): 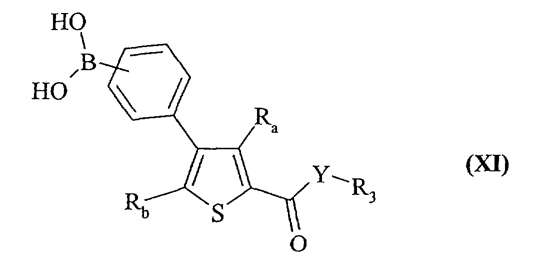
dans lesquels R_{a,} R_{b,} Y et R₃ sont tels que définis précédemment,
composés de formule (XI) qui sont mis à réagir en condition basique et en présence d'un catalyseur au palladium avec un composé de formule (Xa) :

R₆―P₂ (Xa)

dans laquelle R₆ est tel que défini dans la formule (I), c'est à dire qu'il représente un groupement choisi parmi aryle, cycloalkyle, et un hétérocycle, et dans le cas présent R₆ représente préférentiellement un groupement aryle, et P₂ représente un atome d'halogène ou un groupement triflate,
pour conduire également aux composés de formule (I/c), tels que définis précédemment : 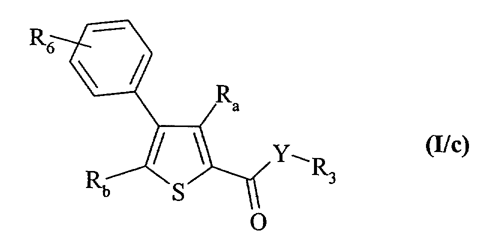 dans lesquels R₆,Rₐ, R_{b}, Y et R₃ sont tels que définis précédemment,
o soit être traités avec de l'hexaméthyldiétain, en présence d'un catalyseur au palladium, pour conduire aux composés de formule (XIa) : 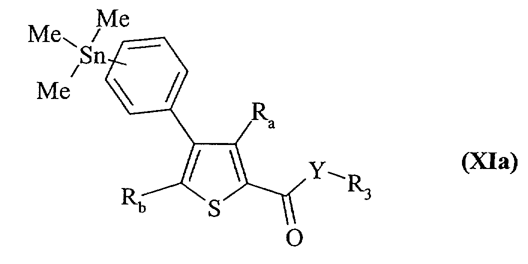 dans laquelle Rₐ, R_{b}, R₃ et Y sont tels que définis précédemment,
   composés de formule (XIb) qui sont mis à réagir avec un composé de formule (Xa) tel que défini précédemment :

   R₆―P₂ (Xa)
dans laquelle R₆ est tel que défini dans la formule (I), P₂ représente un atome d'halogène ou un groupement triflate,
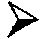 soit en présence de triphénylphosphine arsenic et d'un catalyseur au palladium, dans le cas où **P**₂ représente un groupement triflate,
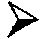 soit en présence d'un composé halogénocuivrique tel que CuBr₂ et d'un catalyseur au palladium, dans des conditions de solvant polaire, dans le cas où P₂ représente un atome d'halogène,
pour conduire également aux composés de formule (I/c), tels que définis précédemment : 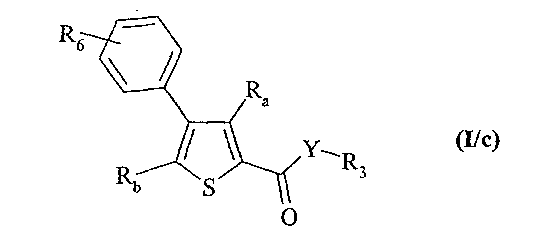 dans lesquels R₆,Rₐ, R_{b}, Y et R₃ sont tels que définis précédemment,
o soit être mis à réagir avec un composé de formule (Xb) (lesdits composés de formule (Xb) étant obtenus par traitement des composés de formule (Xa) tels que définis précédemment, avec de l'hexaméthyldiétain en présence d'un catalyseur au palladium) :

   R₆―SnMe₃ (Xb)
dans laquelle R₆ est tel que défini précédemment,
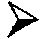 soit en présence de triphénylphosphine arsenic et d'un catalyseur au palladium, dans le cas où les composés de formule (I/b) comportent un groupement triflate,
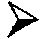 soit en présence d'un composé halogénocuivrique tel que CuBr₂ et d'un catalyseur au palladium, dans des conditions de solvant polaire, dans le cas où les composés de formule (I/b) comportent un atome d'halogène,
   pour conduire également aux composés de formule (I/c) tels que décrits précédemment, 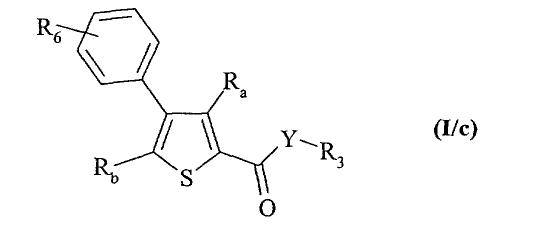 dans lesquels R₆,Rₐ, R_{b}, Y et R₃ sont tels que définis précédemment,

o soit être mis à réagir dans des conditions de couplage au palladium, en milieu basique, par un composé de formule (XII):

   R_{6'}-H (XII)
dans laquelle R₆, représente un groupement hétérocycle azoté éventuellement substitué par un ou plusieurs groupements tels qu'ils sont définis pour les substituants du groupement R₆ au sein des composés de formule (I),
pour conduire aux composés de formule (I/d), cas particulier des composés de formule (I) : 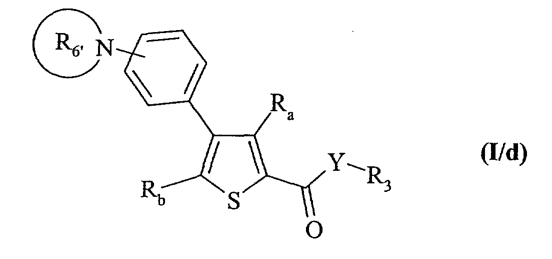 dans lesquels R_{a,} R_{b,} Y et R₃ sont tels que définis précédemment, et R₆, représente un hétérocycle azoté éventuellement substitué tel que défini dans la formule (I),
composés de formule (I/a), (I/b), (I/c) et (I/d) formant ensemble les composés de formule(I/e) 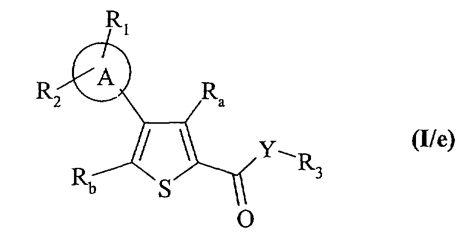 dans lesquels R_{a,} R_{b,} R₁, R₂, A, Y et R₃ sont tels que définis dans la formule (I),
composés de formule (I/e) qui peuvent être traités par exemple par du [2,4-bis(4-méthoxyphényl) 1,3-dithia-2,4-diphosphetane-2,4-disulfide] pour conduire aux composés de formule (I/f), cas particulier des composés de formule (I): 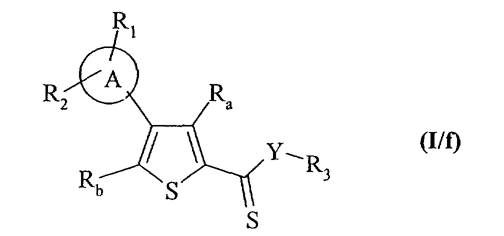 dans lesquels Rₐ, R_{b,} R_{1,} R_{2,} A, Y et R₃ sont tels que définis dans la formule (I),
les composés (I/a) à (I/f) formant l'ensemble des composés de l'invention, qui sont purifiés, le cas échéant, selon une technique classique de purification, qui peuvent, si on le désire, être séparés en leurs différents isomères selon une technique classique de séparation, et que l'on transforme, le cas échéant, en leurs sels d'addition à un acide ou à une base pharmaceutiquement acceptable.

Les composés de formule (II), (V), (VII), (IX), (XI), (XIa) et (XII) sont soit des composés commerciaux, soit obtenus selon des méthodes connues de la synthèse organique aisément accessibles et compréhensibles à l'homme du métier.

Selon une variante de l'invention les composés de formule (I) peuvent également être obtenus par un second procédé caractérisé en ce qu'on utilise comme produit de départ un composé de formule (II) : 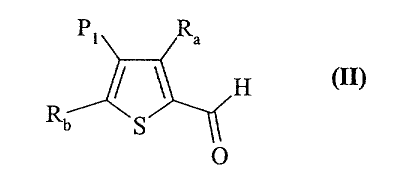 dans laquelle Rₐ et R_{b} sont tels que définis dans la formule (I) et P₁ représente un atome d'halogène ou un groupement triflate,
composés de formule (II) qui sont soumis à des conditions d'oxydations en présence, par exemple, de nitrate d'argent en milieu basique et polaire, pour donner les composés de formule (III) : 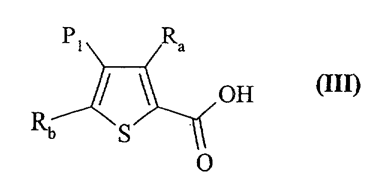 dans laquelle Rₐ, R_{b} et P₁ sont tels que définis précédemment,
composés de formule (III) dont la fonction acide est estérifiée par action d'un alcool en présence d'un acide fort, pour conduire aux composés de formule (XX): 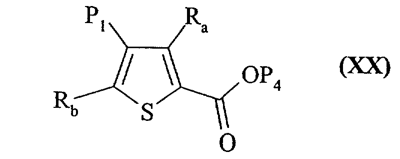 dans laquelle R_{a,} R_{b} et P₁ sont tels que définis précédemment, et P₄ représente un groupement (C₁-C₄)alkyle linéaire ou ramifié,
composés de formule (XX) qui sont :
- soit mis à réagir, dans des conditions basiques de couplage au palladium, avec un composé de formule (VII) : 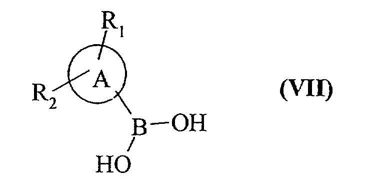
dans laquelle A, R₁ et R₂ ont les mêmes significations que dans les composés de formule (I),
pour conduire aux composés de formule (I/g), cas particulier des composés de formule (I): 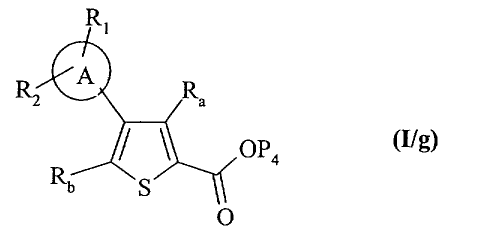 dans laquelle Rₐ, R_{b,} R_{1,} R_{2,} A et P₄ sont tels que définis précédemment,
- soit traités avec de l'hexaméthyldiétain, en présence d'un catalyseur au palladium, pour conduire aux composés de formule (XXII) : 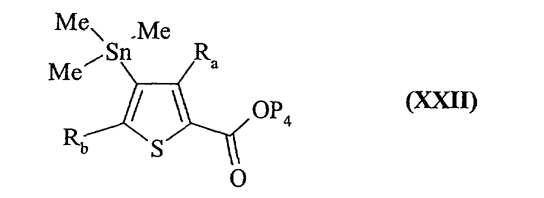
dans laquelle R_{a,} R_{b} et P₄ sont tels que définis précédemment,
composés de formule (XXII) qui sont mis à réagir :
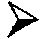 avec un composé de formule (IX): 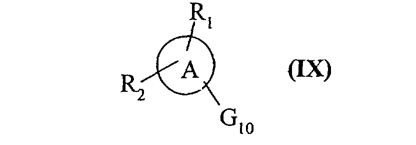
dans laquelle R₁, R₂ et A ont les mêmes significations que dans la formule (I) et G₁₀ représentent un atome d'halogène choisi parmi chlore et brome ou un groupement triflate,
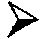 soit en présence de triphénylphosphine arsenic et d'un catalyseur au palladium, dans le cas où G₁₀ représente un groupement triflate,
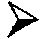 soit en présence d'un composé halogénocuivrique tel que CuBr₂ et d'un catalyseur au palladium, dans des conditions de solvant polaire, dans le cas où G₁₀ représente un atome d'halogène,
pour conduire également aux composés de formule (I/g) tels que décrits précédemment,
- soit traités par du bis(pinacolato)di-borane, suivi par une réaction d'oxydation, pour conduire aux composés de formule (XXI) : 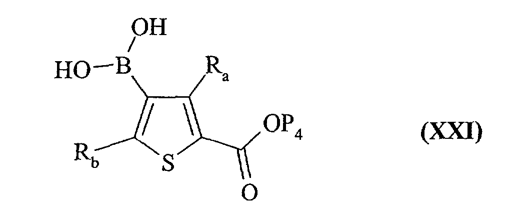
dans laquelle R_{a,} R_{b} et P₄ sont tels que définis précédemment,
composés de formule (XXI) qui sont mis à réagir dans des conditions basiques de couplage au palladium avec un composé de formule (IX) : 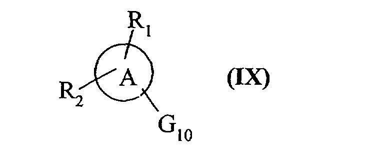 dans laquelle R₁, R₂, G₁₀ et A sont tels que définis précédemment,
pour conduire également aux composés de formule (I/g), tel que défini précédemment : 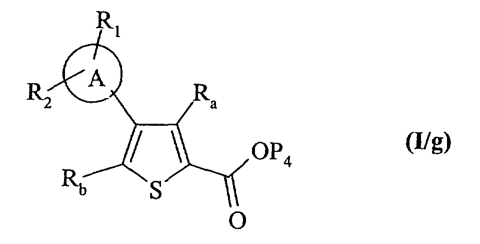 dans laquelle Rₐ, R_{b}, R₁, R₂ et P₄ sont tels que définis précédemment,
- soit mis à réagir avec un composé de formule (IXa), (lesdits composés de formule (IXa) étant obtenus par traitement des composés de formule (IX) tels que définis précédemment, avec de l'hexaméthyldiétain en présence d'un catalyseur au palladium) : 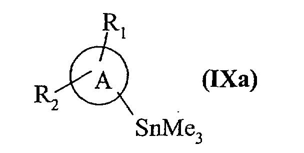
dans laquelle A, R₁, et R₂ sont tels que définis dans la formule (I),
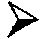 soit en présence de triphénylphosphine arsenic et d'un catalyseur au palladium, dans le cas où P₁ dans les composés de formule (XX) représente un groupement triflate,
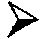 soit en présence d'un composé halogénocuivrique tel que CuBr₂ et d'un catalyseur au palladium, dans des conditions de solvant polaire, dans le cas où P₁ dans les composés de formule (XX) représente un atome d'halogène,
pour conduire également aux composés de formule (I/g) tels que décrits précédemment,
composés de formule (I/g) dans le cas particulier où ils représentent des composés de formule (I/h) dans lesquels A représente un groupement phényle, R₂ représente un atome d'hydrogène, R₁ représente un groupement hydroxy ou un atome d'halogène (Hal) et R_{a,} R_{b} et P₄ ont la même signification que dans la formule (I): 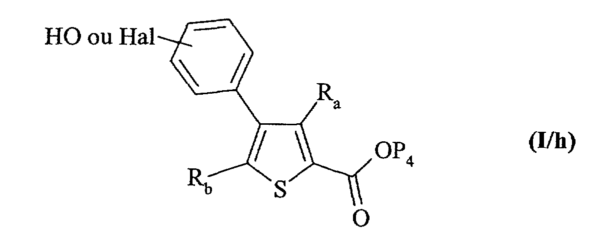 composés de formule (I/h) qui peuvent alors être traités préalablement par de l'anhydride trifluorométhanesulfonique en présence d'une base forte, dans le cas où R₁ représente un groupement hydroxy, pour obtenir le dérivé activé phényltriflate,
lesdits composés portant un groupement R₁ prenant la définition halogène ou triflate pouvant alors :
o soit être mis à réagir en condition basique et en présence d'un catalyseur au palladium avec un composé de formule (X) : 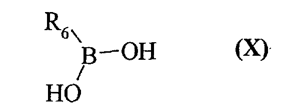
dans laquelle R₆ est tel que défini dans la formule (I), c'est à dire qu'il représente un groupement choisi parmi aryle, cycloalkyle, et un hétérocycle, et dans le cas présent R₆ représente préférentiellement un groupement aryle,
pour conduire aux composés de formule (I/i), cas particulier des composés de formule (I): 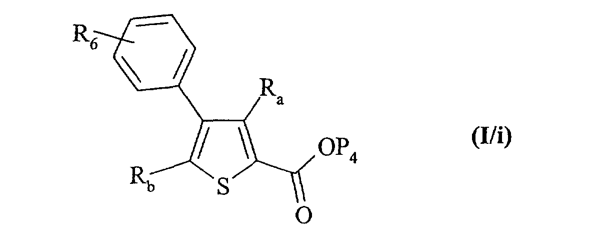 dans lesquels R_{6,}R_{a,} R_{b} et P₄ sont tels que définis précédemment,
o soit être traités par du bis(pinacolato)di-borane suivi par une réaction d'oxydation pour conduire aux composés de formule (XXIII) : 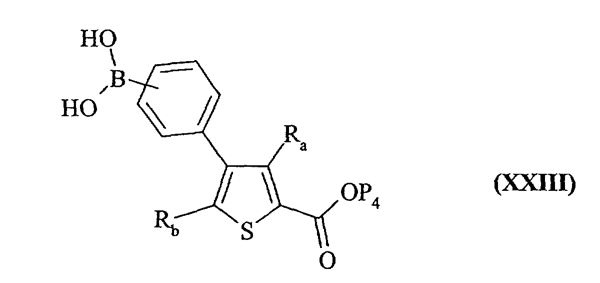
dans lesquels Rₐ, R_{b} et P₄ sont tels que définis précédemment,
composés de formule (XXIII) qui sont mis à réagir en condition basique et en présence d'un catalyseur au palladium avec un composé de formule (Xa):

R₆―P₂ (Xa)

dans laquelle R₆ est tel que défini dans la formule (I), c'est à dire qu'il représente un groupement choisi parmi aryle, cycloalkyle, et un hétérocycle, et dans le cas présent R₆ représente préférentiellement un groupement aryle, et P₂ représente un atome d'halogène ou un groupement triflate,
pour conduire également aux composés de formule (I/i), tels que définis précédemment : 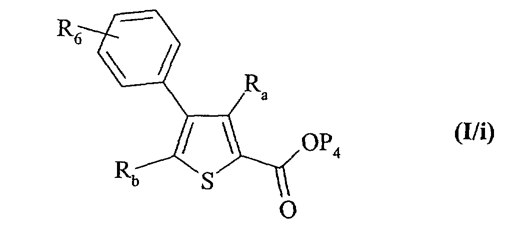 dans lesquels R₆,Rₐ, R_{b} et P₄ sont tels que définis précédemment,
o soit être traités avec de l'hexaméthyldiétain, en présence d'un catalyseur au palladium, pour conduire aux composés de formule (XXIV) : 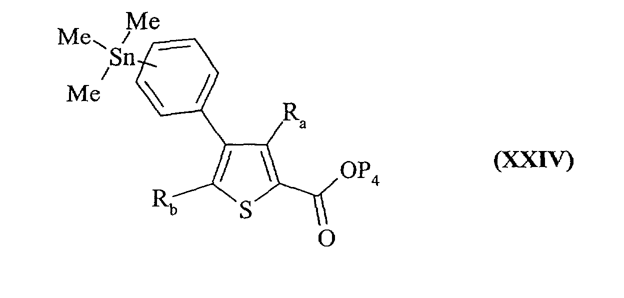
dans laquelle Rₐ, R_{b} et P₄ sont tels que définis précédemment,
composés de formule (XXIV) qui sont mis à réagir avec un composé de formule (Xa) tel que défini précédemment :

R₆―P₂ (Xa)

dans laquelle R₆ est tel que défini dans la formule (I), P₂ représente un atome d'halogène ou un groupement triflate,
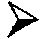 soit en présence de triphénylphosphine arsenic et d'un catalyseur au palladium, dans le cas où P₂ représente un groupement triflate,
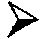 soit en présence d'un composé halogénocuivrique tel que CuBr₂ et d'un catalyseur au palladium, dans des conditions de solvant polaire, dans le cas où P₂ représente un atome d'halogène,
pour conduire également aux composés de formule (I/i), tels que définis précédemment : 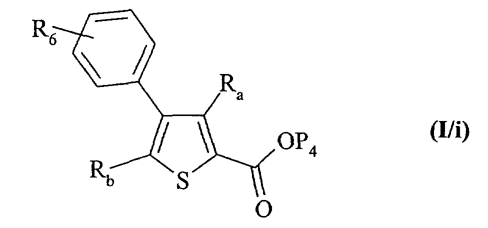 dans lesquels R₆,Rₐ, R_{b} et P₄ sont tels que définis précédemment,
o soit être mis à réagir avec un composé de formule (Xb) (lesdits composés de formule (Xb) étant obtenus par traitement des composés de formule (Xa) tels que définis précédemment, avec de l'hexaméthyldiétain en présence d'un catalyseur au palladium) :

   R₆―SnMe₃ (Xb)
dans laquelle R₆ est tel que défini précédemment,
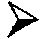 soit en présence de triphénylphosphine arsenic et d'un catalyseur au palladium, dans le cas où les composés de formule (I/h) comportent un groupement triflate,
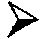 soit en présence d'un composé halogénocuivrique tel que CuBr₂ et d'un catalyseur au palladium, dans des conditions de solvant polaire, dans le cas où les composés de formule (I/h) comportent un atome d'halogène,
pour conduire également aux composés de formule (I/i) tels que décrits précédemment, 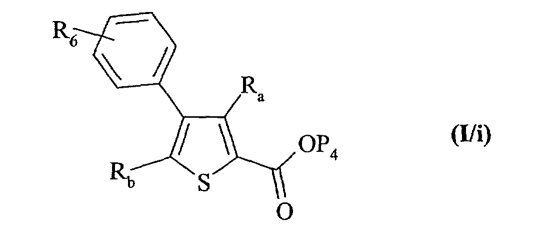 dans lesquels R₆,Rₐ, R_{b} et P₄ sont tels que définis précédemment,
composés de formule (I/g), (I/h) et (I/i) formant ensemble les composés de formule(I/j) 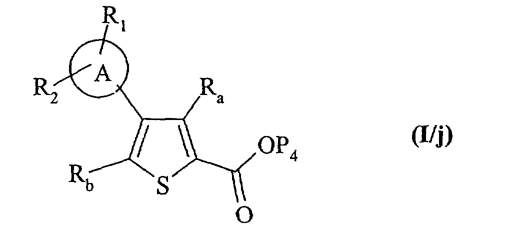 dans lesquels Rₐ, R_{b}, R₁, R₂, A et P₄ sont tels que définis précédemment,
composés de formule (I/i) qui sont saponifiés dans des conditions d'hydrolyse basique, pour conduire aux composés de formule (I/k), cas particulier des composés de formule (I): 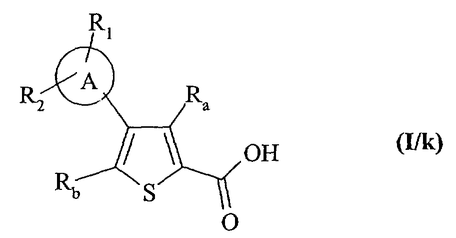 dans lesquels Rₐ, R_{b}, R₁, R₂ et A sont tels que définis dans la formule (I),
composés de formule (I/k) qui sont traités directement, dans des conditions de couplage peptidique en présence par exemple d'en agent de couplage et dans un milieu basique et apolaire, avec un composé de formule (V)

HY-R₃ (V)

dans laquelle Y et R₃ ont les mêmes significations que dans les composés de formule (I), pour conduire aux composés de (I/1), cas particulier des composés de formule (I): 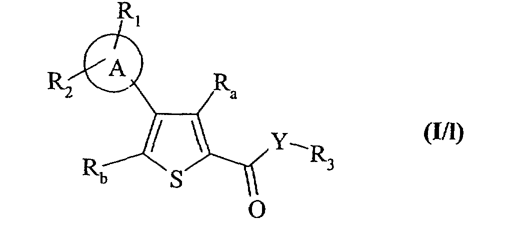 dans laquelle R_{1,} R_{2,} R_{a,} R_{b,} R₃ et Y sont tels que définis précédemment,
composés de formule (I/l) qui peuvent être traités par exemple par du [2,4-bis(4-méthoxyphényl) 1,3-dithia-2,4-diphosphetane-2,4-disulfide] pour conduire aux composés de formule (I/m), cas particulier des composés de formule (I): 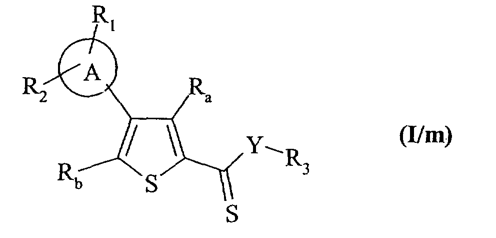 dans lesquels Rₐ, R_{b}, R₁, R₂, A, Y et R₃ sont tels que définis dans la formule (I),
les composés (I/g) à (I/m) formant ensemble des composés de l'invention, qui sont purifiés, le cas échéant, selon une technique classique de purification, qui peuvent, si on le désire, être séparés en leurs différents isomères selon une technique classique de séparation, et que l'on transforme, le cas échéant, en leurs sels d'addition à un acide ou à une base pharmaceutiquement acceptable.

Les composés de la présente invention, de par leurs propriétés pharmacologiques d'inhibiteurs de la MMP-12, sont utiles dans la prévention et le traitement des pathologies respiratoires telles que les maladies pulmonaires obstructives chroniques (BPCO), l'emphysème, les bronchites chroniques, les inflammations pulmonaires chroniques, l'asthme, la mucoviscidose, la détresse respiratoire aigüe (ARDS), les allergies respiratoires dont la rhinite allergique, ainsi que les maladies liées à la production de TNFα incluant les maladies pulmonaires fibrotiques sévères, la sarcoïdose pulmonaire, et la silicose.

D'une façon avantageuse les composés de la présente invention sont utiles pour la prévention et le traitement des maladies pulmonaires obstructives chroniques, de l'emphysème et des bronchites chromiques.

Plus particulièrement les composés de la présente invention sont utiles pour le traitement de l'emphysème lié au tabagisme.

Selon une variante de l'invention, les composés de formule (I) sont utiles pour la prévention et le traitement de l'asthme.

La présente invention a également pour objet les compositions pharmaceutiques renfermant comme principe actif au moins un composé de formule (I), un de ses isomères ou un de ses sels d'addition à un acide ou à une base pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per- ou trans-cutanée, intravaginale, rectale, nasale, perlinguale ou respiratoire.

Les compositions pharmaceutiques selon l'invention pour les injections parentérales comprennent notamment les solutions stériles aqueuses et non aqueuses, les dispersions, les suspensions ou émulsions ainsi que les poudres stériles pour la reconstitution des solutions ou dispersions injectables.

Les compositions pharmaceutiques selon l'invention, pour les administrations orales solides, comprennent notamment les comprimés simples ou dragéifiés, les comprimés sublinguaux, les sachets, les gélules, les granules, et pour les administrations liquides orales, nasales ou buccales, comprennent notamment les émulsions, les solutions, les suspensions, les gouttes, les sirops et les aérosols.

Les compositions pharmaceutiques selon l'invention, pour les administrations par voie respiratoire, comprennent notamment des compositions sous formes de solutions pour aérosols ou de poudres pour inhalateurs. Lorsque les compositions sont des aérosols, pour l'usage d'aérosols liquides, les compositions peuvent être des solutions stériles stables ou des compositions solides dissoutes au moment de l'emploi dans de l'eau stérile apyrogène, dans du sérum physiologique ou tout autre véhicule pharmaceutiquement acceptable. Pour l'usage sous forme d'aérosols secs destinés à être directement inhalés, le principe actif est éventuellement finement divisé ou micronisé, et associé à un diluant ou véhicule inerte solide hydrosoluble.

Les compositions pharmaceutiques pour l'administration rectale sont préférentiellement des suppositoires, et celles pour l'administration per- ou trans-cutanée comprennent notamment les poudres, les aérosols, les crèmes, les pommades, les gels, et les patchs.

Les compositions pharmaceutiques citées précédemment illustrent l'invention mais ne la limitent en aucune façon.

Parmi les excipients ou véhicules inertes, non toxiques, pharmaceutiquement acceptables, on peut citer à titre indicatif et non limitatif les diluants, les solvants, les conservateurs, les agents mouillants, les émulsifiants, les agents dispersants, les liants, les agents gonflants, les agents désintégrants, les retardants, les lubrifiants, les absorbants, les agents de suspension, les colorants, les aromatisants, etc...

La posologie utile varie selon l'âge et le poids du patient, la voie d'administration, la composition pharmaceutique utilisée, la nature et la sévérité de l'affection, et la prise de traitements associés éventuels. La posologie s'échelonne de 1 mg à 1000 mg en une ou plusieurs prises par jour.

Les exemples suivants illustrent l'invention mais ne la limitent en aucune façon.

Les produits de départ utilisés sont des produits commerciaux ou des produits préparés selon des modes opératoires connus à partir de composés commerciaux ou connus par l'homme du métier. Les différentes préparations conduisent à des intermédiaires de synthèse utiles pour la préparation des composés de l'invention.

Les structures des composés décrits dans les exemples et dans les préparations ont été déterminées selon les techniques spectrophotométriques usuelles (infrarouge (IR), résonance magnétique nucléaire (RMN), spectrométrie de masse (SM) dont électrospray (ES), ...) et la pureté a été déterminée par chromatographie liquide à haute performance (HPLC).

### Préparation 1 : 4-Bromo-N-(2-morpholin-4-yléthyl)thiophène-2-carboxamide

### Stade 1 : Acide 4-bromothiophène-2-carboxylique

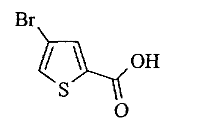

A une solution de 10 g de 4-bromothiophène-2-carboxaldehyde dans 280 ml d'éthanol et 418 ml d'hydroxyde de sodium à 1 mol/l (8 équivalents) sont ajoutés 35,56 g de nitrate d'argent (4 équivalents). Le milieu réactionnel est agité à 40°C pendant 3 heures, puis filtré sur célite et concentré sous pression réduite. Le solide blanc obtenu est solubilisé dans l'eau (300 ml). La phase aqueuse est lavée avec de l'acétate d'éthyle (2x200 ml), acidifiée jusqu'à pH 1 avec une solution d'acide chlorhydrique 1 mol/1, puis extraite avec de l'acétate d'éthyle (2x250 ml). La phase organique est alors séchée sur sulfate de sodium, filtrée puis concentrée sous pression réduite, permettant d'obtenir 9,62 g d'une poudre blanche correspondant au produit attendu.
Rendement : 93%
SM : MH⁻ : 206

### Stade 2: 4-Bromo-N-(2-morpholin-4-vléthvl)thiophène-2-carboxamide

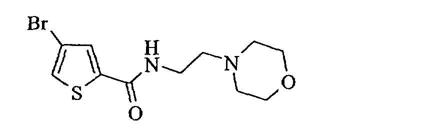

A une solution de 9,62 g du composé obtenu au stade 1 dans 150 ml de dichlorométhane anhydre additionnés de quelques gouttes de diméthylformamide sont ajoutés goutte à goutte 69,7 ml (3 équivalents) de chlorure d'oxalyle. Après 1 heure de réaction à température ambiante et sous azote, le milieu réactionnel est concentré sous pression réduite permettant d'obtenir une huile jaune qui est immédiatement solubilisée dans 150 ml de dichlorométhane anhydre. Le milieu réactionnel est refroidi à 0°C puis sont ajoutés goutte à goutte 6,4 ml de 2-morpholin-4-yléthanamine (1,05 équivalents). Le milieu réactionnel est agité à 0°C pendant 1 heure, puis sont additionnés 7,1 ml de N,N,N-triéthylamine. Le milieu réactionnel est ensuite dilué avec 500 ml de dichlorométhane, lavé avec de l'eau (3x250 ml), séché sur sulfate de sodium, filtré puis concentré sous pression réduite. Une chromatographie sur gel de silice (dichlorométhane/méthanol : 96/4) du résidu permet d'isoler 14,52 g du produit attendu.
Rendement : 98%
RMN ¹H (CDCl₃) δ (ppm) : 2,50 (m, 4H), 2,60 (m, 2H), 3,55 (m, 2H), 3,75 (m, 4H), 6,60 (bs, 1H), 7,35 (s, 1H), 7,40 (s, 1H);
SM : MH⁺ : 320

### Préparation 2 : 4-(Triméthylstannyl)-N-(2-morpholin-4-yléthyl)thiophène-2-carboxamide

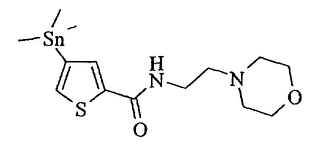

A une solution sous atmosphère inerte de 5,0 g du composé de la préparation 1 dans 80 ml de diméthyl éther éthylène glycol dégazé sont ajoutés 0,905 g de tetrakis(triphénylphosphine) palladium(0) (0,05 équivalent), puis 6,16 g d'hexaméthyldistannate (1,2 équivalents). Le milieu réactionnel est agité pendant 3 heures à 80°C, puis dilué avec de l'acétate d'éthyle (200 ml), lavé avec de l'eau (3x100 ml), séché sur sulfate de sodium, filtré puis concentré à froid sous pression réduite. Une chromatographie sur gel de silice (dichlorométhane/méthanol : 98/2) du résidu permet d'isoler 3,91 g du produit attendu.
Rendement : 60%
RMN ¹H (CDCl₃) δ (ppm) : 0,25 (s, 9H), 2,45 (m, 4H), 2,55 (m, 2H), 3,45 (m, 2H), 3,65 (m, 4H), 6,50 (bs, 1H), 7,375 (s, 1H), 7,50 (s, 1H).

### Préparation 3 : 4-({[4-(Triméthylstannyl)thièn-2-yl]carboxamido}méthyl) cyclohexane carboxylate d' éthyle

### Stade 1: 4-{[(4-Bromothièn-2-yl)carboxamido]méthyl}cyclohexane carboxylate d'éthyle

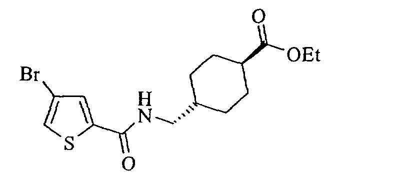

A une solution de 7,56 g du composé obtenu au stade 1 de la préparation 1 dans 150 ml de dichlorométhane sont additionnés 8,09g du produit décrit en préparation 10 stade 3 et, goutte à goutte, 10,16 ml (2.0 éq) de triéthylamine. Le milieu réactionnel est agité 17 heures à température ambiante puis repris dans du dichlorométhane et lavé de façon successive à l'eau, à l'acide chlorhydrique 1,0 M, par une solution saturée d'hydrogénocarbonate de sodium et une solution saturée de chlorure de sodium. Le produit est séché sur sulfate de sodium, filtré et concentré sous pression réduite. Une chromatographie sur gel de silice du résidu (dichlorométhane/acétate d'éthyle: 9/1) permet d'isoler 6,5g de produit attendu
Rendement : 50%
RMN¹H (CDCl₃) δ (ppm): 7,40 (s, 1H), 7,38 (s, 1H), 6,30 (bs, 1H), 4,10 (q, 2H), 3,30 (m, 2H), 2,20 (m, 1H), 2,0 (dd, 2H), 1,90 (dd, 2H), 1,6 (m, 1H), 1,40 (q, 2H), 1,25 (t, 3H), 1,0 (q, 2H);

### Stade 2: 4-({[4-(Triméthylstannyl)thien-2-yl]carboxamido}méthyl)cyclohexane carboxylate d'éthyle

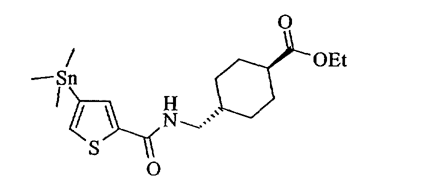

Le produit (0,367 g) est obtenu selon le procédé de la préparation 2, en utilisant comme substrat le produit obtenu dans le stade 1 précédent.
Rendement : 74%
RMN ¹H (CDCl₃) δ (ppm) : 0,325 (s, 9H), 1,05 (q, 2H), 1,25 (t, 3H), 1,425 (q, 2H), 1,575 (bs, 1H), 1,90 (d, 2H), 2,05 (d, 2H), 2,25 (t, 1H), 3,30 (t, 2H), 4,125 (q, 2H), 6,00 (bs, 1H), 7,425 (s, 1H), 7,55 (s, 1H).

### Préparation 4 : 4-Phénylcyclohex-1-ènyl trifluorométhanesulfonate

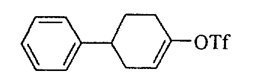

A une solution de 0,5 g de 4-phénylcyclohexanone et 0,648 g de 2,6-di-tert-butyl-4-méthylpyridine (1,1 équivalents) dans 8,0 ml de dichlorométhane anhydre est ajouté goutte à goutte 0,579 ml d'anhydride trifluorométhanesulfonique (1,2 équivalents). Le milieu réactionnel est ensuite agité pendant 1 heure à 40°C, dilué avec 25 ml de dichlorométhane, lavé avec de l'eau (2x15 ml), séché sur sulfate de sodium, filtré puis concentré sous pression réduite. Une chromatographie sur gel de silice (cyclohexane/acétate d'éthyle : 80/20) du résidu permet d'isoler 0,788 g du produit attendu.
Rendement : 89%
RMN ¹H (CDCl₃) δ (ppm) : 1,925 (m, 1H), 2,05 (m, 1H), 2,35 (m, 4H), 2,80 (m, 1H), 5,80 (m, 1H), 7,15 (m, 3H), 7,225 (m, 2H).

### Préparation 5 : Chlorure de 4-[4-(trifluorométhoxy)phényl]thiophèn-2-carbonyl

### Stade 1: 4-(4-(Trifluorométhoxy)phényl]thiophèn-2-carbaldéhyde

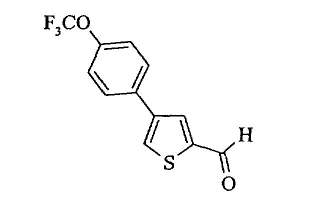

A une solution de 12,3 g de 4-bromothiophène-2-carbaldehyde et 20,0 g d'acide 4-(trifluorométhoxy)phényl-boronique (1,2 équivalent) dans 70 ml de DME dégazé sont additionnés 84,9 ml (2,1 éq) d'une solution 2.0 M de phosphate de potassium et 2,8 g (0,03 équivalent) de tetrakis(triphénylphosphine)palladium(0). Le milieu réactionnel est agité 3 heures à 80°C puis concentré sous pression réduite. Le résidu obtenu est repris par de l'acétate d'éthyle. La solution est alors filtrée sur célite, extraite à l'eau, séchée sur sulfate de sodium, filtrée puis concentrée sous pression réduite. Une chromatographie sur gel de silice du résidu (cyclohexane/acétate d'éthyle : 9/1) permet d'isoler 15,05 g du produit attendu.
Rendement : 68%
RMN ¹H (CDCl₃) δ (ppm) : 10,0 (s, 1H), 8 (s, 1H), 7,8 (s, 1H), 7,55 (m,2H), 7,25 (m, 2H);

### Stade 2 : Acide 4-[4-(tr(fluorométhoxy}phényljthiophèn-2-carboxyilque

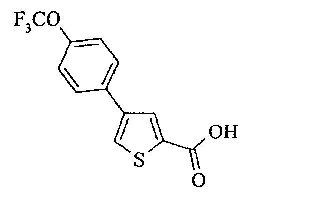

A une solution de 15,05 g du composé obtenu au stade 1 dans 200 ml d'éthanol sont additionnés 37,6 g (4 équivalents) de nitrate d'argent et 44,2 ml (8 équivalents) d'une solution aqueuse 1N de soude. Le milieu réactionnel est agité 2 heures à 40°C, puis filtré sur célite et concentré sous pression réduite. La phase aqueuse est lavée par une solution aqueuse 1N d'acide chlorhydrique, extraite à l'acétate d'éthyle, séchée sur sulfate de sodium, filtrée et concentrée sous pression réduite, permettant d'obtenir 15,514 g (rendement : 97,4%) d'une poudre beige correspondant au produit attendu.
SM: ES- 286,9

### Stade 3 : Chlorure de 4-[4-(trifluorométhoxy)phényljthiophèn-2-carbonyl

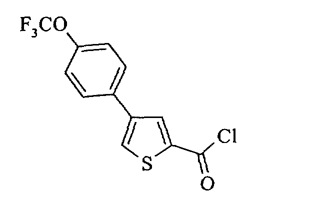

Sur une solution de 0,1 g du composé obtenu au stade 2 dans 5 ml de dichlorométhane additionnés de quelques gouttes de diméthylformamide est coulé 0,35 ml (2 équivalents) de chlorure d'oxalyle. Après 2 heures de réaction à température ambiante, le milieu réactionnel est concentré sous pression réduite permettant d'obtenir une huile jaune correspondant au produit attendu utilisé sous forme brute au cours des préparations suivantes.

### Préparation 6 : 3-(6-Aminopyridin-3-yl)propanoate d'éthyle

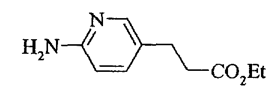

### Stade 1: 6-Aminonicotinate de méthyle

A une solution de 2,0 g d'acide méthyl 6-aminonicotinique dans un mélange (v/v :10/30) chloroforme/méthanol sont additionnés 24,6ml (3,4 éq) de triméthylsilyldiazométhane 2,0 M en solution dans le cyclohexane. Le milieu réactionnel est agité 1 heure à température ambiante puis hydrolysé avec 100 µl d'acide acétique et concentré sous pression réduite. Le produit brut est trituré dans un mélange cyclohexane/acétate d'éthyle (1/2) et filtré pour obtenir 1,459 g d'une poudre jaune correspondant au produit attendu .
Rendement : 66%
SM: ES + 153

### Stade 2 : 6-[bis(tert-Butoxycarbonyl)amino]nicotinate de méthyle

A une solution de 1,459 g de produit obtenu au stade 1 dans 50 ml de dichlorométhane sont ajoutés à 0°C, 4,18 g de di-tert-butyldicarbonate (2,0 éq), 0,117 g de diméthylaminopyridine (0,1 éq) puis sont additionnés goutte à goutte 2,7 ml (2,0 éq) de triéthylamine. Le milieu réactionnel est agité à température ambiante pendant 4 heures, concentré sous pression réduite, lavé à l'eau et puis par une solution aqueuse saturée de chlorure de sodium. Le milieu réactionnel est extrait avec de l'acétate d'éthyle et séché sur sulfate de sodium puis filtré et concentré sous pression réduite. Une chromatographie sur gel de silice du résidu (dichlorométhane/méthanol : 9/1 ) permet d'isoler 1,875 g du produit attendu.
Rendement : 55%
SM: ES+ 353

### Stade 3 : di-tert-Butyl 5-(hydroxyméthyl)pyridin-2-yl-imidodicarbonate

A une solution de 0,985 g du composé obtenu au stade 2 dans 10 ml de tétrahydrofurane est ajouté à 0°C, 0,159 g d'hydrure de lithium aluminium (1,5 éq). Le milieu réactionnel est agité à température ambiante pendant 17 heures puis hydrolysé avec une solution saturée de chlorure d'ammonium et extrait avec de l'acétate d'éthyle. La solution est lavée à l'eau, séchée sur sulfate de sodium, puis filtrée et concentrée sous pression réduite. Une chromatographie sur gel de silice du résidu (dichlorométhane/méthanol: 95/5) permet d'isoler 1,543 g du produit attendu.
Rendement : 89%
SM: ES+ 325

### Stade 4 : di-tert-Butyl 5-formylpyridin-2-yl-imidodicarbonate

A une solution de 0,894 g du composé obtenu au stade 3 dans 10 ml de dichlorométhane est ajouté à 0°C, 0,686 g de [1,1,1-tri(acétyloxy)-1,1-dihydro-1,2-benziodoxol-3(*1H*)-one] (1,5 éq). Le milieu réactionnel est agité à température ambiante pendant 2 heures puis hydrolysé à l'eau et extrait avec du dichlorométhane. La phase organique est lavée à l'eau, séchée sur sulfate de sodium puis filtrée et concentrée sous pression réduite. Une chromatographie sur gel de silice du résidu (cyclohexane/acétate d'éthyle : 80/20) permet d'isoler 0,456 g du produit attendu.
Rendement : 51%
SM: ES+ 355

### Stade 5: Acide (2E) 3-{6-[bis(tert-butoxycarbonyl)aminolpyridin-3-yl}propanoique

A une solution de 0,456 g du composé obtenu au stade 4 dans 0,3 ml de pyridine et 2,0 ml d'éthanol est ajouté 0,177 g d'acide malonique (1,2 éq) et le milieu réactionnel est agité à reflux pendant 17 heures. Le précipité obtenu est filtré et lavé avec de l'acétate d'éthyle pour isoler 0,16 g d'une poudre blanche correspondant au produit attendu.
Rendement: 31%
SM: ES+ 365, ES- 363.

### Stade 6: Acide (2E) 3-(6-aminopyridin-3-yl)propanoique

A une solution de 0,16 g du composé obtenu au stade 5 dans 3 ml d'éthanol sont additionnés 27,6 mg de palladium sur charbon (0,1 éq). Le milieu réactionnel est agité 4 heures à 40°C sous 5 bars d'hydrogène. La solution est alors filtrée sur célite et concentrée sous pression réduite pour obtenir 0,133 g d'une poudre blanche correspondant au produit attendu.
Rendement :83%
SM: ES-365

### Stade 7 : 3-(6-Aminopyridin-3-yl)propanoate d'éthyle

A une solution de 0,133 g du composé obtenu au stade 6 dans 3 ml d'éthanol est additionné 0,4 ml d'acide sulfurique concentré (10 vol). Le milieu réactionnel est agité 17 heures à reflux. La solution est alors concentrée sous pression réduite et basifiée avec une solution d'hydrogénocarbonate de sodium saturée jusqu'à pH=8. La solution obtenue est extraite avec de l'acétate d'éthyle et la phase organique est séchée sur sulfate de sodium puis filtrée pour obtenir 0,041 g du produit attendu après évaporation sous pression réduite.
Rendement :59%
SM: ES+195, ES- 193.

### Préparation 7 : (2E)-3-(4-Aminophényl)acrylate d'éthyle

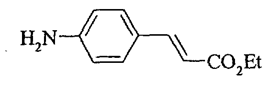

Le produit (0,153 g) est obtenu selon le procédé du stade 7 de la préparation 6, en utilisant comme substrat l'hydrochlorure de l'acide 4-aminocinnamique.
Rendement :32%
SM : ES+ 192

**Préparation 8 : 4-(Aminométhyl)phtalate de diéthyle**

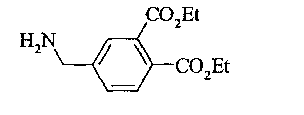

### Stade 1: 4-Bromophtalate de diéthyle

A une solution de 1,0 g d'acide 4-bromophtalique est ajouté 1,0 ml d'acide sulfurique concentré dans 20 ml d'éthanol et le milieu réactionnel est irradié à 79°C sous micro-ondes (puissance 50W) pendant 30 minutes. Le milieu réactionnel est concentré sous pression réduite et extrait avec de l'acétate d'éthyle. La solution est lavée avec de l'eau et séchée sur sulfate de sodium, puis filtrée pour obtenir 1,08 g du produit attendu après évaporation sous pression réduite.
Rendement : 88%

### Stade 2 : 4-Cyanophtalate de diéthyle

A une solution de 0,95 g du composé obtenu au stade 1 dans 10 ml de *N*-méthyl-2 pyrrolidinone est additionnés 0,508 g (1,8 éq) de cyanure de cuivre. Le milieu réactionnel est agité 17 heures à 200°C. La solution est alors hydrolysée avec une solution aqueuse d'hydroxyde d'ammonium et extraite avec de l'acétate d'éthyle. La solution est lavée à l'eau, séchée sur sulfate de sodium puis filtrée pour obtenir après évaporation sous pression réduite 1,2 g d'une huile brune. Une chromatographie sur gel de silice du résidu (cyclohexane/acétate d'éthyle : 8/2 ) permet d'isoler 0,320 g du produit attendu .
Rendement : 41%
MS : ES+ 273

### Stade 3 : 4-(Aminométhyl)phtalate de diéthyle

A une solution de 0,15 g de produit obtenu au stade 2 sont ajoutés 1 ml d'éthanol, 0,5 ml d'acide chlorhydrique à 36% et 15 mg (0,1 équivalent) de palladium sur charbon à 10%. Le milieu réactionnel est agité sous 15 bars d'hydrogène à 60°C pendant 2 heures. Le milieu est filtré sur célite et concentré sous pression réduite pour obtenir 0,3 g d'une poudre beige.
Rendement : 24%
SM : ES+ 252

### Préparation 9: 3-(4-Aminophényl)propanoate d'éthyle

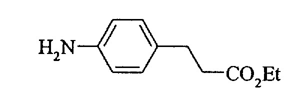

Le produit (0,503 g) est obtenu selon le procédé du stade 7 de la préparation 6, en utilisant comme substrat l'acide 3-(4-aminophényl)propionique .
Rendement : 86%
RMN ¹H (CDCl₃) δ (ppm) : 7,0 (m, 2H), 6,6 (m, 2H), 4,1 (q, 2H), 2,8 (q,2H), 1,25 (t, 3H).

### Préparation 10 : trans 4-(Aminométhyl)cyclohexanecarboxylate d'éthyle

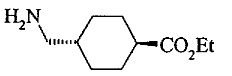

A une solution de 0,2 g d'acide trans-4-(aminométhyl)cyclohexane carboxylique dans 5 ml d'éthanol est ajouté 0,4 ml d'acide sulfurique. Le milieu réactionnel est porté à reflux pendant 17 heures, puis concentré sous pression réduite. Le résidu est repris à l'acétate d'éthyle. La solution est basifiée jusqu'à pH=9 par addition d'une solution aqueuse de soude 1N, lavée à l'eau, séchée sur sulfate de sodium puis concentrée sous pression réduite permettant d'obtenir 0,129 g d'une huile jaune correspondant au produit attendu.
Rendement : 55%
SM : ES+ 186

### Préparation 11: [4-(Aminométhyl)phényl] acétate d'éthyle

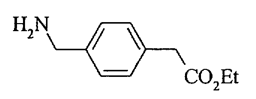

### Stade 1 : (4-Bromophényl)acétate d'éthyle

Le produit (4,208 g) est obtenu selon le procédé du stade 7 de la préparation 6, en utilisant comme substrat l'acide (4-bromophényl)acétique.
Rendement : 93%
SM : ES+243, ES- 242 .

### Stade 2 : (4-Cyanophényl)acétate d'éthyle

Le produit (0,2 g) est obtenu selon le procédé du stade 2 de la préparation 8, en utilisant comme substrat le produit obtenu dans le stade 1 précédent.
Rendement : 52%
RMN ¹H (CDCIₛ) δ (ppm): 7,6 (m, 2H), 7,5 (m, 1H), 7,4 (m, 1H), 3,8 (s,3H), 3,7 (s, 2H).

### Stade 3 : [4-(Aminométhyl)phényl]acétate d'éthyle

Le produit (0,045 g) est obtenu selon le procédé du stade 3 de la préparation 8, en utilisant comme substrat le produit obtenu dans le stade 2 précédent.
Rendement: 17%
SM : ES- 223

### Préparation 12 :3-(Aminométhyl)benzoate d'éthyle

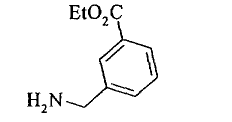

### Stade 1 : Acide 3-(aminométhyl)benzoïque

Le produit (0,213 g) est obtenu selon le procédé du stade 3 de la préparation 8, en utilisant comme substrat l'acide 3-cyanobenzoïque.
Rendement : 100%
SM : ES+ 152

### Stade 2 : 3-(Aminométhyl)benzoate d'éthyle

Le produit (0,15 g) est obtenu selon le procédé du stade 7 de la préparation 6, en utilisant comme substrat le produit obtenu dans le stade 1 précédent.
Rendement : 60%
SM : ES+ 180

### Préparation 13 : [3-(aminométhyl)phényl] acétate d'éthyle

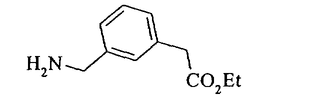

### Stade 1 : Acide (3-iodophényl)acétique

Une solution de 0,2 g de 3-iodophénylacetonitrile dans 1,0 ml d'une solution aqueuse 1,0 N de soude est placée à reflux pendant 4 heures. La solution est extraite avec du diéthyléther et la phase aqueuse est acidifiée avec une solution d'acide chlorhydrique 1,0 N. La solution extraite au diéthyléther est lavée à l'eau, séchée sur sulfate de sodium, filtrée puis évaporée sous pression réduite pour obtenir 0,17 g du produit attendu.
Rendement : 83%
RMN ¹H (DMSO) δ (ppm): 7,65 (s, 1H), 7,5 (d, 1H), 7,3 (d, 1H), 7,1 (m, 1H), 3,6 (s, 2H).

### Stade 2 : (3-Iodophényl)acétate d'éthyle

Le produit (0,164 g) est obtenu selon le procédé du stade 7 de la préparation 6, en utilisant comme substrat le produit obtenu dans le stade 1 précédent.
Rendement : 82%
RMN ¹H (CDCl₃) δ (ppm): 7,65 (s, 1H), 7,6(d, 1H), 7,3 (s, 1H), 7,1 (m, 1H), 4,2 (q, 2H), 3,5 (s, 2H), 1,25 (t, 3H).

### Stade 3 : (3-Cyanophényl)acétate d'éthyle

Le produit (0,065 g) est obtenu selon le procédé du stade 2 de la préparation 8, en utilisant comme substrat le produit obtenu dans le stade 2 précédent.
Rendement : 61 %
SM : ES-188

### Stade 4 : [3-(Aminométhyl)phényl]acétate d'éthyle

Le produit (0,071 g) est obtenu selon le procédé du stade 3 de la préparation 8, en utilisant comme substrat le produit obtenu dans le stade 3 précédent.
Rendement : 100%
SM : ES+ 194

### Préparation 14 : 4-(Aminométhyl)cyclohexanecarboxylate d'éthyle

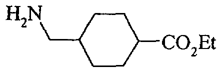

Le produit (0,239 g) est obtenu selon le procédé du stade 7 de la préparation 6, en utili comme substrat l'acide 4-(aminométhyl)cyclohexane carboxylique.
Rendement: 81%
SM : ES+ 186

### Préparation 15 : 6-(Bromométhyl)nicotinate de méthyle

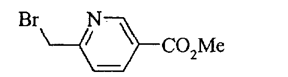

### Stade 1: 6-Méthylnicotinate de méthyle

Le produit (0,708 g) est obtenu selon le procédé du stade 1 de la préparation 6, en utilisant comme substrat 1' acide 6-méthyl nicotinique.
Rendement : 33%
SM : ES+ 152

### Stade 2 : 6-(Bromométhyl)nicotinate de méthyle

A une solution de 0,4 g du produit obtenu dans le stade 1 précédent dans 15 ml de tétrachlorure de carbone, sont ajoutés 0,471 g (1,0 éq) de *N*-bromosuccinimide et 64 mg (0,1 éq) de peroxyde de benzoyle. La solution est portée à reflux pendant 6 heures, puis filtrée et évaporée sous pression réduite. Le résidu est purifié par chromatographie sur gel de silice (dichlorométhane/acétate d'éthyle: 95/5 ) pour obtenir 0,262 g du produit attendu.
Rendement : 43%
SM : ES + 231

### Préparation 16 : 4-(Aminométhyl)benzoate d'éthyle

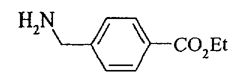

Le produit (1,03 g) est obtenu selon le procédé du stade 7 de la préparation 6, en utilisant comme substrat l'acide 4-(aminométhyl)benzoïque.
Rendement : 87%
SM : ES+ 179

### Préparation 17 :4-([(4-Bromothièn-2-yl)carboxamido]méthyl}benzoate d'éthyle

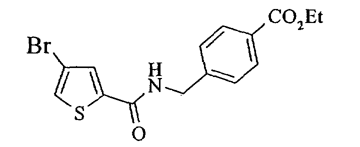

### Stade 1 : Chlorure de 4-bromothiophéne-2-carbonyl

Le produit (4,06g) est obtenu selon le procédé du stade 3 de la préparation 5, en utilisant comme substrat l'acide 4-bromothiophène-2carboxylique.

### Stade 2 : 4-{[(4-Bromothièn-2-yl)carboxamido]méthyl}bezoate d'éthyle

A une solution de 0,833 g de produit obtenu dans le stade 1 précédent dans 15 ml de dichlorométhane sont ajoutés 0,700 g (1,05 éq) du produit obtenu dans la préparation 16 et 0,77 ml de triéthylamine (1,5 éq). Le milieu réactionnel est agité 17 heures à température ambiante. La solution est hydrolysée à l'eau, lavée avec une solution aqueuse d'acide chlorhydrique 1,0 N, puis avec une solution d'hydrogénocarbonate de sodium saturée. La phase organique est séchée sur sulfate de sodium, filtrée et concentrée sous pression réduite. Le résidu est purifié par chromatographie sur gel de silice (dichlorométhane/acétate d'éthyle : 97/3) pour obtenir 0,1 g du produit attendu .
Rendement : 7%
RMN ¹H (CDCl₃) δ (ppm): 8,0 (m, 2H), 7,4 (m, 4H), 6,4 (bs, 1H), 4,6 (d, 2H), 4,3 (q, 2H), 1,40 (t, 3H).

### Préparation 18 : :{4-[(4-Bromothièn-2-yl)carboxamido]phényl}acétate d'éthyle

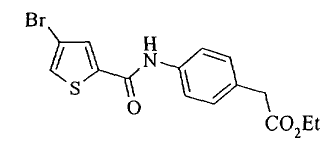

### Stade 1 : (4-Aminophényl)acétate d'éthyle

Le produit (1,686 g) est obtenu selon le procédé du stade 7 de la préparation 6 en utilisant comme substrat l'acide 4-aminophénylacétique .
Rendement : 71%
SM : ES+ 179

### Stade 2 : {4-[(4-Bromothièn-2-yl)carboxainido]phényl}acétate d'éthyle

Le produit (0,550 g) est obtenu selon le procédé du stade 2 de la préparation 17, en utilisant comme substrat le produit obtenu au stade 1 de la préparation 17, et comme co-substrat le produit décrit au stade 1 précédent.
Rendement : 32 %
SM : ES+ 369

### Préparation 19 : Chlorure de 4-(4-méthoxyphényl)thiophène-2-carbonyl

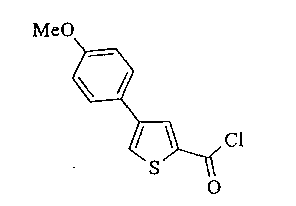

### Stade 1: 4-(4-Méthoxyphényl)thiophen-2-carbaldéhyde

A une solution de 5,24 g de 4-bromothiophène-2-carboxaldehyde et 5,0 g d'acide 4-(méthoxy)phényl-boronique (1,2 équivalents) dans 50 ml de DME dégazé sont additionnés 28,8 ml (2,1 éq) de phosphate de potassium et 0,95 g (0,03 équivalent) de tetrakis(triphénylphosphine)palladium(0). Le milieu réactionnel est agité 3 heures à 80°C puis concentré sous pression réduite. Le résidu obtenu est repris par de l'acétate d'éthyle. La solution est alors filtrée sur célite, extraite à l'eau, séchée sur sulfate de sodium, filtrée puis concentrée sous pression réduite. Une chromatographie sur gel de silice du résidu (cyclohexane/acétate d'éthyle : 9/1) permet d'isoler le produit attendu (2,594 g).
Rendement : 36%
SM : ES+ 218

### Stade 2 : Acide 4-(4-méthoxyphényl)thiophèn-2-carboxylique

Le produit (2,34 g) est obtenu selon le procédé du stade 2 de la préparation 5, en utilisant comme substrat le produit obtenu au stade 1 précédent.
Rendement : 84%
SM : ES-232

### Stade 3 : Chlorure de 4-(4-méthoxyphényl)thiophène-2-carbonyl

Le produit (0,384 g) est obtenu selon le procédé du stade 3 de la préparation 5, en utilisant comme substrat le produit obtenu au stade 2 précédent.

### Préparation 20 : 3-[4-(Aminométhyl)phényl]propanoate d'éthyle

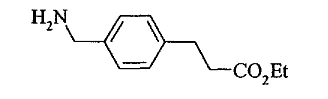

### Stade 1: 3-(4-Chlorophényl)propanoate d'éthyle

Le produit (1,046 g) est obtenu selon le procédé du stade 7 de la préparation 6, en utilisant comme substrat l'acide 3-(4-chlorophényl)propanoïque.
Rendement : 91%
SM : ES+ 276

### Stade 2 : 3-(4-Cyanophényl)propanoate d'éthyle

Le produit (0,076 g) est obtenu selon le procédé du stade 2 de la préparation 8, en utilisant comme substrat le produit obtenu au stade 1 précédent.
Rendement : 16%
SM: ES+204

### Stade 3 : 3-[4-(Aminométhyl)phényl]propanoate d'éthyle

Le produit (0,131 g) est obtenu selon le procédé du stade 3 de la préparation 8, en utilisant comme substrat le produit obtenu au stade 2 précédent.
Rendement : 77%
SM : ES+ 208

### Préparation 21 : 3-(3-Aminophényl)propanoate d'éthyle

### Stade 1 : 3-(3-Bromophényl)propanoate d'éthyle

Le produit (1,778 g) est obtenu selon le procédé du stade 7 de la préparation 6, en utilisant comme substrat l'acide 3-(3-bromophényl)propionique.
Rendement : 80%
RMN ¹H (CDCl₃) δ (ppm): 7,35 (m, 2H), 7,1 (m, 2H), 4,1 (q, 2H), 2,9 (q, 2H), 2,6 (q, 2H), 1,20 (t,3H).

### Stade 2 : 3-(3-Cyanophényl)propanoate d'éthyle

Le produit (1,277 g) est obtenu selon le procédé du stade 2 de la préparation 8, en utilisant comme substrat le produit obtenu au stade 1 précédent.
Rendement : 91%
RMN ¹H (CDCl₃) δ (ppm): 7,55 (s, 2H), 7,4 (m, 1H), 7,35 (m, 1H), 4,1 (q, 2H), 2,9 (q, 2H), 2,6 (q, 2H), 1,20 (t, 3H).

### Stade 3 : 3-(3-Aminophényl)propanoate d'éthyle

Le produit (0,619 g) est obtenu selon le procédé du stade 3 de la préparation 8, en utilisant comme substrat le produit obtenu au stade 2 précédent.
Rendement : 40%
SM : ES+ 208

### Préparation 22 : 7-Aminoheptanoate d'éthyle

Le produit (0,470 g) est obtenu selon le procédé du stade 7 de la préparation 6, en utilisant comme substrat l'acide 6-aminocaproïque.
Rendement : 78%
SM : ES + 159,9

### Exemple 1 ; 4-(4-Isopropylphényl)-N-(2-morpholin-4-yléthyl)thiophène-2-carboxamide

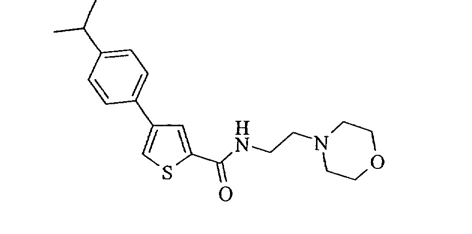

A une solution sous azote de 100 mg du composé de la préparation 1 dans 3,0 ml de DME dégazé sont ajoutés 61,6 mg d'acide (4-isopropylphényl) boronique (1,2 équivalents), 10,8 mg de tetrakis(triphénylphosphine)palladium(0) (0,03 équivalents) et 0,328 ml d'une solution de phosphate de potassium à 2,0 mol/l (2,1 équivalents). Le milieu réactionnel est ensuite agité pendant 3 heures à 80°C, dilué avec de l'acétate d'éthyle (20 ml), lavé avec de l'eau (2x15 ml), séché sur sulfate de sodium, filtré et concentré sous pression réduite. Une chromatographie sur gel de silice (dichlorométhane/méthanol : 98/2) du résidu permet d'isoler 51,4 mg du produit attendu.
Rendement : 46%
RMN ¹H (CDCl₃) δ (ppm) : 1,30 (d, 6H), 2,575 (m, 4H), 2,60 (m, 2H), 2,95 (m, 1H), 3,575 (m, 2H), 3,75 (m, 4H), 6,65 (bs, 1H), 7,30 (d, 2H), 7,50 (m, 3H), 7,75 (s, 1H);
SM : MH⁺: 359
HPLC (214nm) : 100,00%

### Exemple 2: 4-(4-Biphényl)-N-(2-morpholin-4-yléthyl)thiophène-2-carboxamide

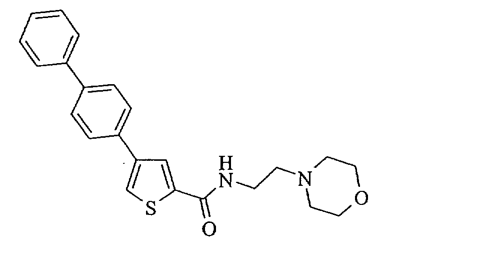

Le produit (57,7 mg) est obtenu selon le procédé de l'exemple 1 en utilisant comme co-substrat l'acide (1,1'-biphényl-4-yl)boronique.
Rendement : 47%
RMN ¹H (DMSO) δ (ppm) : 2,45 (m, 4H), 2,50 (m, 4H), 7,375 (m, 1H), 7,50 (m, 2H), 7,75 (m, 6H), 8,10 (s, 1H), 8,25 (s, 1H), 8,50 (bs, 1H);
SM : MH⁺ : 393
HPLC (214 nm) : 98,2%

### Exemple 3 : 4-(4-Ethylphényl)-N-(2-morpholin-4-yléthyl)thiophène-2-carboxamide

Le produit (15,8 mg) est obtenu selon le procédé de l'exemple 1 en utilisant comme co-substrat l'acide 4-éthylphénylboronique.
Rendement : 15%
RMN ¹H (CDCl₃) δ (ppm) : 1,25 (t, 3H), 2,50 (m, 4H), 2,60 (m, 2H), 2,70 (q, 2H), 3,55 (m, 2H), 3,75 (m, 4H), 6,625 (bs, 1H), 7,25 (d, 2H), 7,50 (m, 3H), 7,75 (s, 1H);
SM : MH⁺ : 345
HPLC (214 nm) : 99,4%

### Exemple 4 : 4-[4-(Méthylthio)phényl]-N-(2-morpholin-4-yléthyl)thiophéne-2-carboxamide

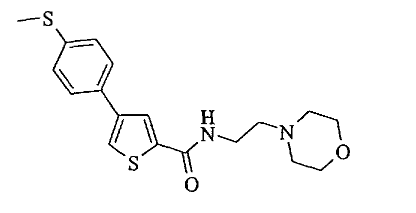

Le produit (64,4 mg) est obtenu selon le procédé de l'exemple 1 en utilisant comme co-substrat l'acide 4-(méthylthio)phénylboronique.
Rendement : 56%
RMN ¹H (CDCl₃) δ (ppm) : 2,525 (m, 7H), 2,60 (m, 2H), 3,55 (m, 2H), 3,75 (m, 4H), 6,625 (bs, 1H), 7,30 (d, 2H), 7,50 (m, 3H), 7,75 (s, 1H);
SM : MH⁺ : 363
HPLC : 94,9%

### Exemple 5 : 4-[4-(Trifluorométhyl)phényl]-N-(2-morpholin-4-yléthyl)thiophène-2-carboxamide

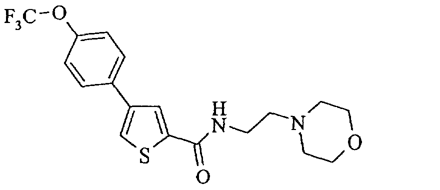

Le produit (166,2 mg) est obtenu selon le procédé de l'exemple 1 en utilisant comme co-substrat l'acide 4-(trifluorométhoxy)phénylboronique.
Rendement : 44%
RMN ¹H (CDCl₃) δ (ppm) : 2,50 (m, 4H), 2,60 (m, 2H), 3,55 (m, 2H), 3,75 (m, 4H), 6,625 (bs, 1H), 7,25 (d, 2H), 7,55 (s, 1H), 7,60 (d, 2H), 7,75 (s, 1H);
SM : MH⁺ : 401
HPLC : 98,4%

### Exemple 6: 4-[4-(tert-Butyl)phényl]-N(2-morpholin-4-yléthyl)thiophène-2-carboxamide

### Stade 1: 4-(4-tert-Butylphényl)thiophène-2-carbaldéhyde

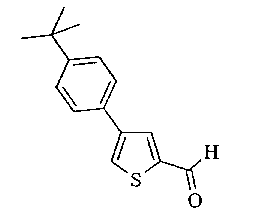

Le produit (199,8 mg) est obtenu selon le procédé du stade 1 de la préparation 9 en utilisant comme substrat le 4-bromothiophène-2-carbaldehyde et comme co-substrat l'acide 4-tert-butylphénylboronique.
Rendement : 78%

### Stade 2 : Acide 4-(4-tert-butylphényl)thiophène-2-carboxylique

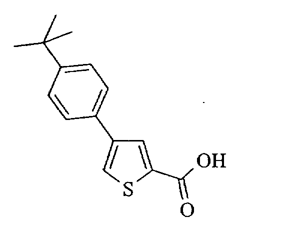

Le produit (39,3 mg) est obtenu selon le procédé du stade 2 de la préparation 5 en utilisant comme substrat le composé obtenu au stade 1 précédent.
Rendement : 37%

### Stade 3 : 4-[4-(tert-ButyI)phényq-N-(2-morpholin-4-yléthyl)thiophène-2-carboxamide

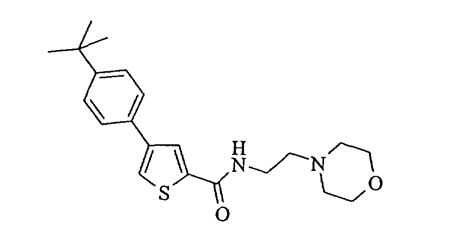

A une solution de 39,3 mg du composé obtenu au stade 2 précédent dans 1,0 ml de diméthylformamide anhydre sont ajoutés 21,8 µl de 2-morpholin-4-yléthanamine (1,1 équivalents), 63,2 mg de O-(7-azabenzotriazol-1-yl)-N,N,N',N'-tetraméthyluronium hexafluorophosphate (HATU) et 57,9 µl de N-éthyl-N,N-diisopropylamine. Le milieu réactionnel est agité pendant 17 heures à température ambiante puis concentré sous pression réduite. Le résidu obtenu est solubilisé dans l'acétate d'éthyle (15 ml), lavé avec de l'eau (2x8 ml), séché sur sulfate de sodium, filtré et concentré sous pression réduite. Une chromatographie sur gel de silice (dichlorométhane/méthanol : 98/2) du résidu permet d'isoler 9,0 mg du produit attendu.
Rendement : 16%
RMN ¹H (CDCl₃) δ (ppm) : 2,55 (m, 4H), 2,60 (m, 2H), 3,55 (m, 2H), 3,75 (m, 4H), 6,65 (bs, 1H), 7,45 (d, 2H), 7,525 (m, 3H), 7,75 (s, 1H);
SM : MH⁺ : 373
HPLC : 95,80%

### Exemple 7: 4-[4-(Trifluorométhyl)phényl}-N-(2-morpholin-4-yléthyl)thiophéne-2-carboxamide

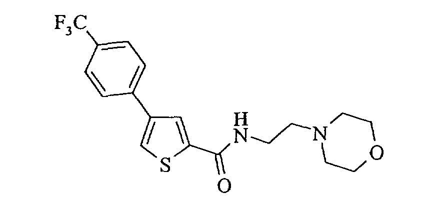

Le produit (16,3 mg) est obtenu selon le procédé de l'exemple 6 des stades 1 à 3, en utilisant au stade 1 comme co-substrat l'acide 4-(trifluorométhyl)phénylboronique au lieu de l'acide 4-tert-butylphénylboronique.
Rendement : 26%
RMN ¹H (CDCl₃) δ (ppm) : 2,55 (m, 4H), 2,65 (m, 2H), 3,675 (m, 2H), 3,75 (m, 4H), 6,725 (bs, 1H), 7,65 (s, 1H), 7,675 (s, 4H), 7;80 (s, 1H);
SM : MH⁺ : 385
HPLC : 97,16%

### Exemple 8: 4-(4-Hydroxyphényl)-N-(2-morpholin-4-yléthyl)thiophène-2-carboxamide

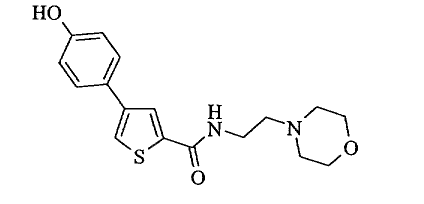

Le produit (2,73 g) est obtenu selon le procédé de l'exemple 1, en utilisant comme co-substrat l'acide (4-hydroxyphényl)boronique au lieu de l'acide (4-isopropylphényl)boronique.
Rendement : 54%
RMN ¹H (DMSO) δ (ppm) : 2,425 (m, 4H), 2,50 (m, 2H), 3,40 (m, 2H), 3,60 (m,4H), 6,80 (d, 2H), 7,50 (d, 2H), 7,85 (s, 1H), 8,10 (s, 1H), 8,425 (bs, 1H), 9,55 (s, 1H);
SM : MH⁺ : 333
HPLC : 97,8%

### Exemple 9 : 4-[4-(Pyridin-4-yl)phenyl]-N(2-morpholin-4-yléthyl)thiophène-2-carboxamide

### Stade 1: 4-(5-{[(2-Morpholin-4-yléthyl)amino]carbonyl}thien-3-yl)phényltrifluoro méthane sulfonate

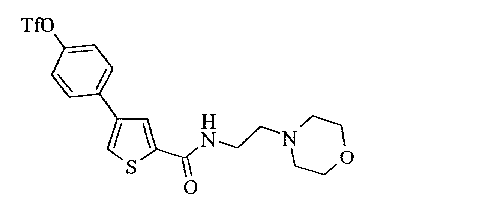

A une solution de 2,73 g du composé obtenu dans l'exemple 8 dans 50 ml de dichlorométhane anhydre sont ajoutés goutte à goutte 0,798 ml de pyridine (1,2 équivalents) et 1,45 ml d'anhydride trifluorométhanesulfonique (1,1 équivalents). Le milieu réactionnel est agité pendant 2 heures à température ambiante, dilué avec 250 ml de dichlorométhane, lavé avec de l'eau (3x100 ml), séché sur sulfate de sodium et concentré sous pression réduite. Une chromatographie sur gel de silice (dichlorométhane/méthanol : 98/2) du résidu obtenu permet d'isoler 2,62 g du produit désiré.
Rendement : 68%
RMN ¹H (CDCl₃) δ (ppm) : 2,475 (m, 4H), 2,55 (m, 2H), 3,50 (m, 2H), 3,70 (m, 4H), 6;50 (bs, 1H), 7,275 (d, 2H), 7,50 (s, 1H), 7,60 (d, 2H), 7,70 (s, 2H);
SM : MH⁺ : 465
HPLC : 98,10%

### Stade 2: 4-[4-(Pyridin-4-yl)phényl]-N-(2-morpholin-4-yléthyl)thiophène-2-carboxamide

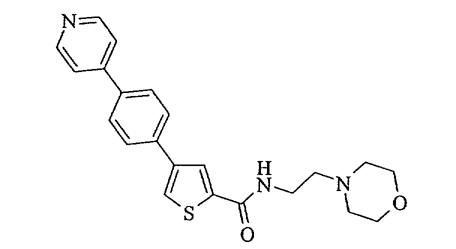

Le produit (23,7 mg) est obtenu selon le procédé de l'exemple 1, en utilisant comme substrat le produit obtenu au stade 1 précédent et comme co-substrat l'acide 4-pyridylboronique au lieu de l'acide (4-isopropylphényl)boronique.
Rendement : 17%
RMN ¹H (CDCl₃) δ (ppm) : 2,55 (m, 4H), 2,65 (m, 2H), 3,60 (m, 2H), 3,75 (m, 4H), 6,65 (bs, 1H), 7,55 (d, 2H), 7,65 (s, 1H), 7,70 (s, 4H), 7,825 (s, 1H), 8,70 (d, 2H);
SM : MH⁺ : 394
HPLC : 94,54%

### Exemple 10: 4'-[5-(2-Morpholin4-yl-éthylcarbamoyl)-thiophèn-3-yl]biphényl-3-carboxylate de méthyle

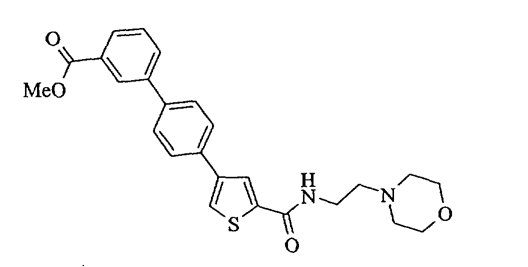

Le produit (200,2 mg) est obtenu selon le procédé de l'exemple 1, en utilisant comme substrat le produit du stade 1 de l'exemple 9 et comme co-substrat l'acide 3-(méthoxycarbonyl))phénylboronique au lieu de l'acide (4-isopropylphényl)boronique.
Rendement : 68%
RMN ¹H (CDCl₃) δ (ppm) : 2,55 (m, 4H), 2,625 (m, 2H), 3,575 (m, 2H), 3,75 (m, 4H), 3,975 (s, 3H), 6,65 (bs, 1H), 7,55 (t, 1H), 7,625 (s, 1H), 7,70 (s, 4H), 7,825 (m, 2H), 8,05 (d, 1H), 8,30 (s, 1H);
SM : MH⁺ : 451
HPLC : 95,76

### Exemple 11: 4-[4-(Pyridin-3-yl)phényl]-N-(2-morpholin-4-yléthyl)thiophène-2-carboxamide

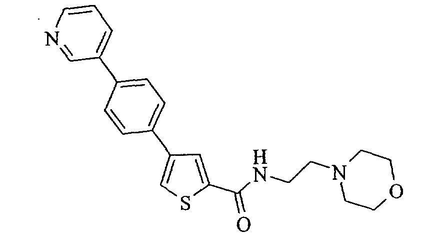

Le produit (95,2 mg) est obtenu selon le procédé de l'exemple 1, en utilisant comme substrat le produit obtenu au stade 1 de l'exemple 9 et comme co-substrat l'acide 3-pyridylboronique au lieu de l'acide (4-isopropylphényl)boronique.
Rendement : 45%
RMN ¹H (CDCl₃) δ (ppm): 2,55 (m, 4H), 2,65 (m, 2H), 3,60 (m, 2H), 3,75 (m, 4H), 6,65 (bs, 1H), 7,40 (m, 1H). 7,65 (m, 3H), 7,70 (d, 2H), 7,825 (s, 1H), 7,925 (d, 1H), 8,625 (d, 1H), 8,90 (s, 1H);
SM : MH⁺ : 394
HPLC : 100,00%

### Exemple 12 : 4-[4-(Morpholin-4-yl)phényl]-N-(2-morpholin-4-yléthyl)thiophène-2-carboxamide

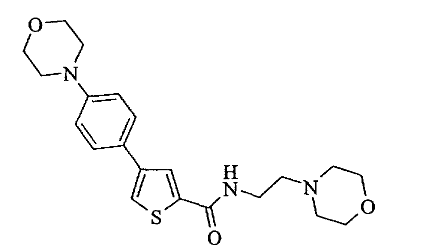

A une solution de 100 mg du composé obtenu au stade 1 de l'exemple 9 dans 2,0 ml de DME dégazé sont ajoutés sous azote 23 µl de morpholine (1,2 équivalents), 5,9 mg de tris(dibenzylideneacetone)dipalladium(0) (0,03 équivalents), 3,8 mg de 2-(di-t-butylphosphino)biphényl (0,06 équivalents) et 64 mg de phosphate de potassium. Le milieu réactionnel est agité pendant 17 heures à 80°C, dilué avec de l'acétate d'éthyle (20 ml), lavé avec de l'eau (2x10 ml), séché sur sulfate de sodium, filtré et concentré sous pression réduite. Une chromatographie sur gel de silice (dichlorométhane/méthanol : 98/2) du résidu obtenu permet d'isoler 25,2 mg du produit attendu.
Rendement : 29%
RMN ¹H (CDCl₃) δ (ppm) : 2,50 (m, 4H), 2,60 (m, 2H), 3,20 (m, 4H), 3,55 (m, 2H), 3,725 (m, 4H), 3,875 (m, 4H), 6,60 (bs, 1H), 6,975 (d, 2H), 7,45 (s, 1H), 7,50 (d, 2H), 7,75 (s, 1H);
SM : MH⁺: 402
HPLC : 100,00%

### Exemple 13 : 4-(4-Pipéridophényl)-N-(2-morpholin-4-yléthyl)thiophène-2-carboxamide

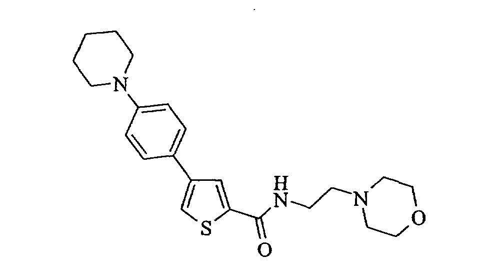

Le produit (40,3 mg) est obtenu selon le procédé de l'exemple 12, en utilisant comme co-substrat la pipéridine à la place de la morpholine.
Rendement : 50%
RMN ¹H (CDCl₃) δ (ppm) : 1,60 (m, 2H), 1,725 (m, 4H), 2,50 (m, 4H), 2,60 (m, 2H), 3,20 (m, 4H), 3,525 (m, 2H), 3,75 (4H), 6,60 (bs, 1H), 7,00 (d, 2H), 7,425 (s, 1H), 7,50 (d, 2H), 7,725 (s, 1H);
SM : MH⁺ : 400
HPLC : 98,3%

### Exemple 14: 4-[4-(Pyrrolidin-1-yl)phényl]-N-(2-morpholin-4-yléthyl)thiophène-2-carboxamide

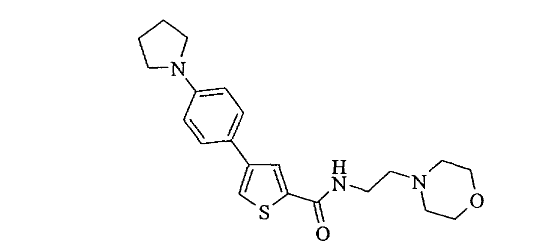

Le produit (45,7 mg) est obtenu selon le procédé de l'exemple 12, en utilisant comme co-substrat la pyrrolidine à la place de la morpholine.
Rendement : 36%
RMN ¹H (CDCl₃) δ (ppm) : 2,05 (m, 4H), 2,55 (m, 4H), 2,625 (m, 2H), 3,35 (m, 4H), 3,55 (m, 2H), 3,75 (m, 4H), 6,60 (m, 3H), 7,35 (s, 1H), 7,45 (d, 2H), 7,75 (s, 2H);
SM : MH⁺ : 386
HPLC : 99,2%

### Exemple 15: 4-[4-(1,4-Dioxa-8-azaspiro[4,5]dec-8-yl)phényl]-N-(2-morpholin-4- yléthyl)thiophène-2-carboxamide

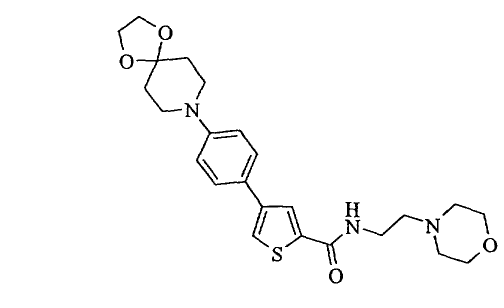

Le produit (82,1 mg) est obtenu selon le procédé de l'exemple 12, en utilisant comme co-substrat le 1,4-dioxa-8-azaspiro[4,5]decane à la place de la morpholine.
Rendement : 41%
RMN ¹H (CDCl₃) δ (ppm) : 1,875 (m, 4H), 2,525 (m, 4H), 2,625 (m, 2H), 3,40 (m, 4H), 3,55 (m, 2H), 3,75 (m, 4H), 4,00 (s, 4H), 6,625 (bs, 1H), 7,975 (d, 2H), 7,45 (s, 1H), 7,50 (d, 2H), 7,75 (s, 1H);
SM : MH⁺ : 458
HPLC : 100,00%

### Exemple 16: 4-[4-(1,2,3,6-Tétrahydropyridin-1-yl)phényl]-N-(2-morpbolin4-yléthyl)thiophène-2-carboxainide

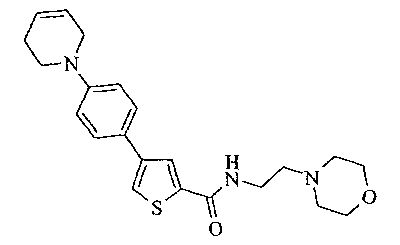

Le produit (33,4 mg) est obtenu selon le procédé de l'exemple 12, en utilisant comme co-substrat la 1,2,3,6-tetrahydropyridine à la place de la morpholine.
Rendement : 26%
RMN ¹H (CDCl₃) δ (ppm) : 2,25 (m, 2H), 2,45 (m, 4H), 2,55 (m, 2H), 3,375 (m, 2H), 3,50 (m, 2H), 3,675 (s, 6H), 5,75 (m, 1H), 5,85 (m, 1H), 6,65 (bs, 1H), 6,90 (d, 2H), 7,35 (s, 1H), 7,45 (d, 2H), 7,675 (s, 1H);
SM : MH⁺ : 398
HPLC : 94,0%

### Exemple 17: 4-[4-(3-(3R)-Hydroxy-pyrrolidin-1-yl)phényl]-N-(2-morpholin-4- yléthyl)thiophène-2-carboxamide

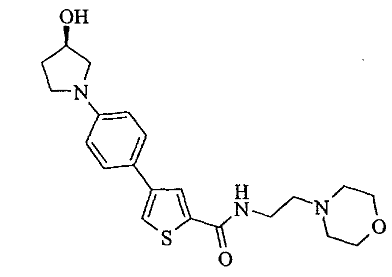

Le produit (66,5 mg) est obtenu selon le procédé de l'exemple 12, en utilisant comme co-substrat la (3R)-pyrrolidin-3-ol à la place de la morpholine.
Rendement : 38%
RMN ¹H (CDCl₃) δ (ppm) : 2,05 (m, 1H), 2,15 (m, 1H), 2,45 (m,4H), 2,55 (m, 2H), 3,25 (m, 1H), 3,35 (m, 1H), 3,50 (m, 4H), 3,65 (m, 4H), 4,55 (bs, 1H), 6,55 (d, 3H), 7,30 (s, 1H), 7,40 (d, 2H), 7,65 (s, 1H);
SM : MH⁺ : 402
HPLC : 100,0%

### Exemple 18 : 4-[4-(1H-Imidazol-1-yl)phényl]-N-(2-morpholin-4-yléthyl)thiophène- 2-carboxamide

### Stade 1 : 4-Bromothiophène-2-carboxylate de méthyle

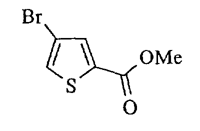

A une solution de 5,0 g du composé obtenu lors du stade 1 de la préparation 1 dans 25 ml de méthanol sont ajoutés quelques gouttes d'acide sulfurique concentré. Le milieu réactionnel est agité pendant 17 heures à 65°C puis concentré sous pression réduite. Le résidu huileux obtenu est solubilisé avec de l'acétate d'éthyle (200 ml), lavé avec de l'eau (3x100 ml), séché sur sulfate de sodium puis concentré sous pression réduite afin d'obtenir 5,24 g du produit désiré.
Rendement : 98%
RMN ¹H (CDCl₃) δ (ppm) : 3,90 (s, 3H), 7,45 (s, 1H), 7,70 (s, 1H).

### Stade 2 : 4-(4-Hydroxyphényl)thiophène-2-carboxylate de méthyle

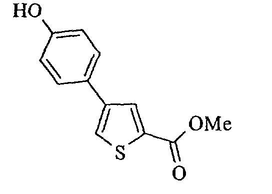

Le produit (1,43 g) est obtenu selon le procédé de l'exemple 1, en utilisant comme substrat le produit obtenu au stade 1 précédent et comme co-substrat l'acide (4-hydroxyphényl)boronique à la place de l'acide (4-isopropylphényl)boronique.
Rendement : 37%
RMN ¹H (CDCl₃) δ (ppm) : 3,825 (s, 3H), 5,25 (s, 1H), 6,80 (d, 2H), 7,40 (d, 2H), 7,475 (s, 1H), 7,95 (s, 1H);
HPLC : 98,8%

### Stade 3 : 4-(4-{[(Trifluorométhyl)sulfonyl]oxy}phényl)thiophène-2-carboxylate de méthyle

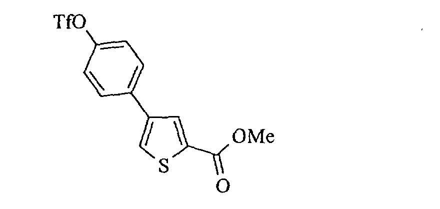

Le produit (1,74 g) est obtenu selon le procédé du stade 1 de l'exemple 9, en utilisant comme substrat le produit obtenu au stade 2 précédent.
Rendement : 78%
RMN ¹H (CDCl₃) δ (ppm) : 4,925 (s, 3H), 7,35 (d, 2H), 7,675 (m, 3H), 8,05 (s, 1H);
HPLC : 100,0%

### Stade 4 : 4-[4-(Pinacolboro)phényl]thiophène-2-carboxylate de méthyle

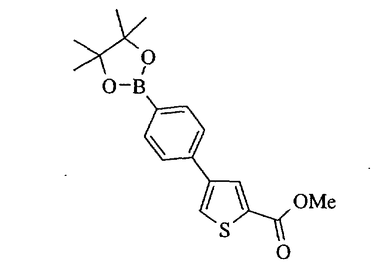

A une solution de 1,61 g du produit obtenu au stade 3 précédent dans 35 ml de 1,4-dioxane dégazé sont ajoutés sous azote 1,34 g de bis(pinacolate)di-bore (1,2 équivalents), 96,3 mg de dichloro[[1,1']-bis(diphénylphosphino)ferrocene]palladium (II) (0,03 équivalents), 146,1 mg de [1,1']-bis(diphénylphosphino)ferrocene (0,06 équivalents) et 1,29 g d'acétate de potassium (3 équivalents). Le milieu réactionnel est agité pendant 17 heures à 80°C, dilué avec de l'acétate d'éthyle (100 ml), lavé avec de l'eau (3x60 ml), séché sur sulfate de sodium et concentré sous pression réduite. Une chromatographie sur gel de silice (dichlorométhane : 100%) du résidu permet d'obtenir 1,11 g du produit désiré.
Rendement : 73%
RMN ¹H (CDCl₃) δ (ppm) : 1,30 (s, 12H), 3,825 (s, 3H), 7,525 (d, 2H), 7,650 (s, 1H), 7,80 (d, 2H), 8,05 (s, 1H);
HPLC : 89%

### Stade 5 : 4-[4-(Dihydroxyboro)phényl]thiophène-2-carboxylate de méthyle

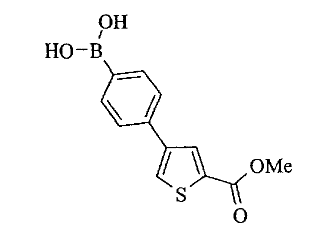

A une solution de 1,11 g du composé obtenu au stade 4 précédent dans 10 ml d'acétone sont ajoutés 10 ml d'eau et 2,07 g de sodium periodate (3,0 équivalents). Le milieu réactionnel est agité pendant 17 heures à 60°C puis concentré sous pression réduite. Le résidu obtenu est solubilisé avec de l'acétate d'éthyle (50 ml), lavé avec une solution d'acide chlorhydrique à 1,0 mol/l puis avec de l'eau (3x20 ml), séché sur sulfate de sodium, filtré puis concentré sous pression réduite. Une chromatographie sur gel de silice (dichlorométhane/méthanol : 90/10) du résidu permet d'obtenir 608 mg du produit désiré.
Rendement : 71%
RMN ¹H (DMSO) δ (ppm) : 3,85 (s, 3H), 7,75 (d, 2H), 7,85 (d, 2H), 8,225 (s, 1H), 8,30 (s, 1H);
SM : MH⁻+HCO₂H : 307

### Stade 6 : 4-[4-(1H-lmidazol-1-yl)phényl]thiophène-2-carboxylate de méthyle

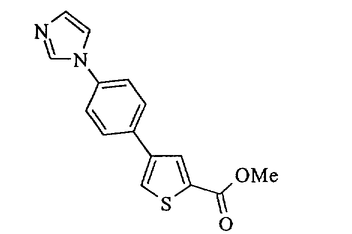

A une solution de 200 mg du composé obtenu au stade 5 dans 2,5 ml de dichlorométhane anhydre sont ajoutés 24 mg d'imidazole (0,5 équivalents), 103,5 mg d'acétate de cuivre (II) (0,75 équivalents), 61 µl de pyridine (1 équivalent) et 20 mg de tamis moléculaire à 4Â. Le milieu réactionnel est agité pendant 17 heures à température ambiante, filtré, dilué avec du dichlorométhane (20 ml), lavé avec de l'eau (2x10 ml), séché sur sulfate de sodium, filtré puis concentré sous pression réduite. Une chromatographie sur gel de silice (dichlorométhane/méthanol : 92/10) du résidu permet d'obtenir 40 mg du produit désiré.
Rendement : 37%
RMN ¹H (DMSO) δ (ppm) : 3,875 (s, 3H), 7,35 (bs, 1H), 7,775 (d, 2H), 8,00 (d, 2H), 8,30 (s, 1H), 8,40 (s, 1H), 8,725 (bs, 1H);
HPLC : 97,9%

### Stade 7 : Acide 4-[4-(1H-imidazol-1-yl)phényl]thiophène-2-carboxylique

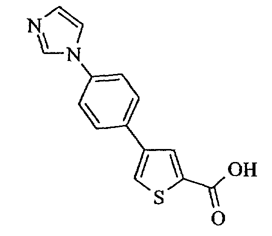

A une solution de 40 mg du composé obtenu au stade 6 dans 1,0 ml de méthanol sont ajoutés 0,5 ml d'eau et 16,8 mg d'hydroxyde de lithium (5 équivalents). Le milieu réactionnel est agité pendant 2 heures à 45°C puis concentré sous pression réduite. Le solide blanc obtenu est repris avec 5,0 ml d'une solution d'acide chlorhydrique à 1,0 mol/l. Le précipité formé est alors filtré sur un verre fritté, lavé avec de l'eau (2x3,0 ml) puis séché à l'étuve pendant 1 nuit permettant ainsi d'obtenir 31 mg du produit désiré.
Rendement : 81%
SM : MH⁺ : 271
HPLC : 97,4%

### Stade 8 : 4-[4-(1H-Imidazol-1-yl)phényl]-N-(2-morpholin-4-yléthyl)thiophène-2- carboxamide

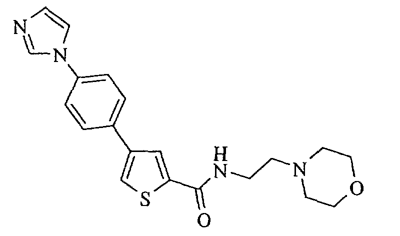

Le produit (22,1 mg) est obtenu selon le procédé du stade 3 de l'exemple 6, en utilisant comme substrat le produit obtenu au stade 7 précédent.
Rendement : 50%
RMN ¹H (CDCl₃) δ (ppm) : 2,50 (m, 4H), 2,55 (m, 2H), 3,50 (m, 2H), 3,70 (m, 2H), 6,575 (bs, 1H), 7,20 (m, 1H), 7,225 (s, 1H), 7,40 (d, 2H), 7,525 (s, 1H), 7,625 (d, 2H), 7,75 (s, 1H), 7,85 (s, 1H);
SM : MH⁺: 383
HPLC : 97,9%

### Exemple 19: N-(2-Morpholin-4-yléthyl)4-[4-(1H-pyrrol-1 -yl)phényl]-thiophène-2- carboxamide

### Stade 1: 4-(4-Nitrophényl)thiophène-2-carboxylate de méthyle

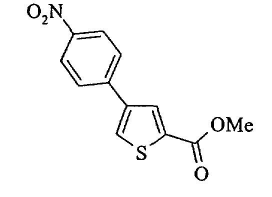

Le produit (1,94 g) est obtenu selon le procédé de l'exemple 1 en utilisant comme substrat le 4-bromothiophène-2-carboxylate de méthyle et comme co-substrat l'acide (4-nitrophényl)boronique.
Rendement : 78%
RMN ¹H (CDCl₃) δ (ppm) : 3,925 (s, 3H), 7,75 (d, 2H), 7,825 (s, 1H), 8,125 (s, 1H), 8,30 (d, 2H);

### Stade 2 : 4-(4-Aminophényl)thiophène-2-carboxylate de méthyle

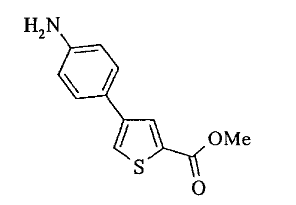

Une solution de 1,94 g du composé obtenu au stade 1 précédent dans 20 ml de méthanol contenant 194 mg de palladium sur charbon à 10% est agitée dans un autoclave pendant 6 heures à 50°C sous 10 bars d'hydrogène, après avoir purgé 3 fois avec de l'azote et 3 fois avec de l'hydrogène. Le milieu réactionnel est ensuite filtré sur célite et concentré sous pression réduite permettant d'obtenir 1,51 g du produit désiré.
Rendement : 88%
RMN ¹H (DMSO) δ (ppm) : 3,825 (s, 3H), 5,225 (s, 2H), 6,60 (d, 2H), 7,425 (d, 2H), 7,90 (s, 1H), 8,05 (s, 1H);
SM : MH⁺ : 234

### Stade 3 : 4-[4-[1H-pyrrol-1-yl)phényl]thiophène-2-carboxylate de méthyle

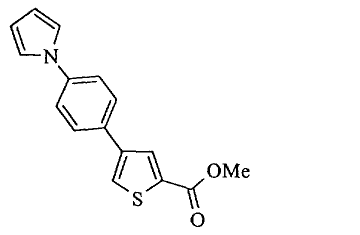

A une solution de 150 mg du composé obtenu au stade 2 précédent dans 1,0 ml d'acide acétique sont ajoutés 87 µl de 2,5-diméthoxytetrahydrofurane (1,05 équivalents). Le milieu réactionnel est agité pendant 1 heure à 110°C, dilué avec de l'acétate d'éthyle (25 ml), lavé avec une solution d'hydroxyde de sodium à 1,0 mol/l (10 ml) puis avec de l'eau (2x10 ml), séché sur sulfate de sodium, filtré puis concentré sous pression réduite. Une chromatographie sur gel de silice (dichlorométhane : 100%) du résidu permet d'obtenir 96,3 mg du produit désiré.
Rendement : 53%
RMN ¹H (CDCl₃) δ (ppm) : 3,875 (s, 3H), 6,30 (s, 2H), 7, 05 (s, 2H), 7,375 (d, 2H), 7,60 (m, 3H), 8,025 (s, 1H);
HPLC : 95,9%

### Stade 4 : Acide 4-[4-(lH-pyrrol-l-yl)phényl]thiophène-2-carboxylique

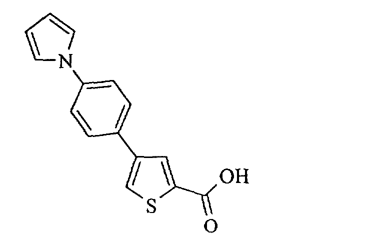

Le produit (69,5 mg) est obtenu selon le procédé du stade 7 de l'exemple 18, en utilisant comme substrat le produit obtenu dans le stade 3 précédent.
Rendement : 81%
RMN ¹H (DMSO) δ (ppm) : 6,275 (s, 2H), 7,40 (s, 2H), 7,625 (d, 2H), 7,825 (d, 2H), 8,15 (s, 1H), 8,20 (s, 1H), 13,10 (bs, 1H);
SM : MH⁻ : 268
HPLC : 93,5%

### Stade 5 : N-(2-Morpholin-4-yléthyl)-4-[4-(1H-pyrrol-1-yl)phényl]thiophène-2-carboxamide

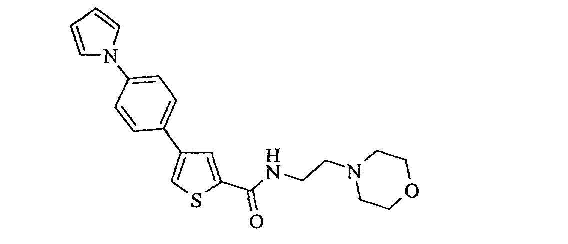

Le produit (99,1 mg) est obtenu selon le procédé du stade 3 de l'exemple 6, en utilisant comme substrat le produit obtenu dans le stade 4 précédent.
Rendement : 57%
RMN ¹H (CDCl₃) δ (ppm) : 2,525 (m, 4H), 2,60 (m, 2H), 3,575 (m, 2H), 3,725 (m, 4H), 6,35 (s, 2H), 6,60 (bs, 1H), 7,125 (s, 2H), 7,45 (d, 2H), 7,55 (s, 1H), 7,625 (d, 2H), 7,775 (s, 1H);
SM : 382
HPLC : 98,9%

### Exemple 20: 4-[4-(Isoxazol-5-yl)phényll-N-(2-morpholin-4-yléthyl)thiophène-2- carboxamide

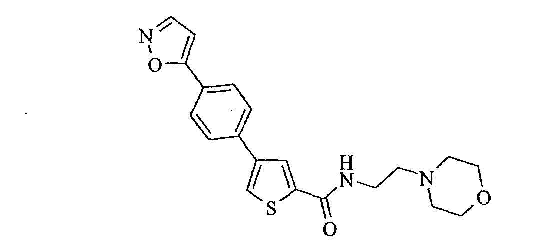

A une solution de 150 mg du composé obtenu lors de la préparation 2 dans 2,0 ml de dioxane dégazé sont ajoutés sous azote 83,3 mg de 5-(4-bromophényl)isoxazole (1,0 équivalent), 47,3 mg de chlorure de lithium (3,0 équivalents), 8,2 mg de bromure de cuivre(II) (0,1 équivalent) et 12,9 mg de tetrakis(triphénylphosphine)palladium(0) (0,03 équivalent). Le milieu réactionnel est agité pendant 4 heures à 80°C, dilué avec de l'acétate d'éthyle (20 ml), lavé avec de l'eau (10 ml), séché sur sulfate de sodium, filtré puis concentré sous pression réduite. Une chromatographie sur gel de silice (dichlorométhane/méthanol : 98/2) du résidu permet d'obtenir 64,2 mg du produit désiré.
Rendement : 45%
RMN ¹H (DMSO) δ (ppm) : 2,45 (m, 4H), 2,525 (m, 2H), 3,40 (m, 2H), 3,60 (m, 4H), 7,75 (s, 1H), 7,825 (s, 4H), 8,175 (s, 1H), 8,25 (s, 1H), 8,475 (s, 1H), 8,55 (bs, 1H);
SM : 384
HPLC : 100,0%

### Exemple 21 : 4-[4-(Cyclohexyl)phényl-N-(2-morpholin-4-yléthyl)thiophène-2-carboxamide

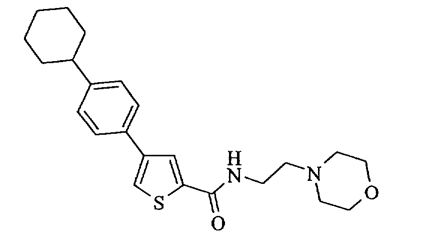

Le produit (14,8 mg) est obtenu selon le procédé de l'exemple 20, en utilisant comme co-substrat le 1-bromo-4-cyclohexylbenzene.
Rendement : 9%
RMN ¹H (CDCl₃) δ (ppm) : 1,30 (m, 1H), 1,45 (m, 4H), 1,75 (d, 1H), 1,85 (m, 4H), 2,55 (m, 5H), 2,65 (m, 2H), 3,55 (m, 2H), 3,75 (m, 4h), 6,75 (bs, 1H), 7,25 (d, 2H), 7,50 (m, 3H), 7,775 (s, 1H);
SM : 399
HPLC : 97,0%

### Exemple 22 : 4-[4-(1-Méthyl-1H-pyrazol-3-yl)phényl]-N-(2-morpholin-4-yléthyl) thiophène-2-carboxamide

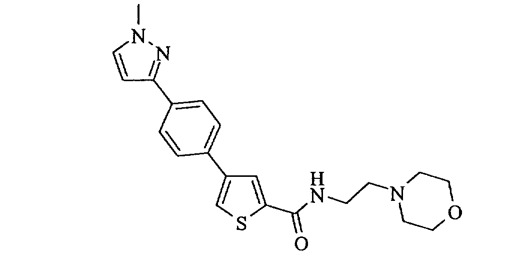

Le produit (36 mg) est obtenu selon le procédé de l'exemple 20, en utilisant comme co-substrat le 3-(4-bromophényl)-1-méthyl-1H-pyrazole.
Rendement : 23%
RMN ¹H (DMSO) δ (ppm) : 2,45 (m, 4H), 2,50 (m, 2H), 3,40 (m, 2H), 3,60 (m, 4H), 3,90 (m, 3H), 6,45 (m, 1H), 7,475 (s, 1H), 7,625 (d, 2H), 7,825 (d, 2H), 8,175 (s, 1H), 8,25 (s, 1H), 8,50 (bs, 1H);
SM : MH⁺ : 398
HPLC : 97,6%

### Exemple 23 : 4-[4-(6-Oxo-1,4,5,6-tétrahydro-pyridazin-3-yl)phényl]-N-(2-morpholin-4-yléthyl)thiophène-2-carboxamide

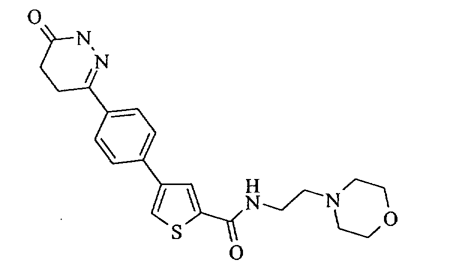

Le produit (8,1 mg) est obtenu selon le procédé de l'exemple 20, en utilisant comme co-substrat la 6-(4-bromophényl)-4,5-dihydropyridazin-3(2H)-one.
Rendement : 5%
RMN ¹H (DMSO) δ (ppm) : 2,45 (m, 4H), 2,50(m, 4H), 3,00 (t, 2H), 3,40 (m, 2H), 3,60 (m, 4H), 7,75 (d, 2H), 7,85 (d, 2H), 8,15 (s, 1H). 8,25 (s, 1H), 8,50 (bs, 1H), 10,95 (s, 1H);
SM : MH⁺ : 413
HPLC : 98,0%

### Exemple 24 : trans 4-{[4-(4-Phényl-cyclohex-1-ényl)-thiophèn-2-carboxamide] méthyl}-cyclohexane carboxylate de méthyle

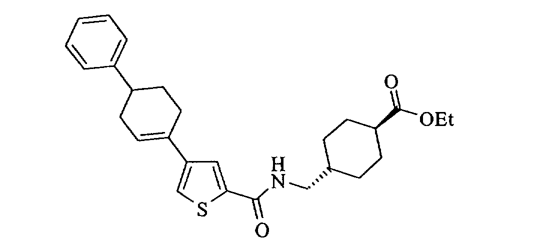

A une solution de 100 mg du produit de la préparation 3 dans 2,0 ml de diméthylformamide dégazé sont ajoutés sous azote 80 mg du composé de la préparation 4 (1,2 équivalents), 10 mg de tris(dibenzilidèneacétone)dipalladium(0) (0,05 équivalent) et 6,6 mg de triphénylarsine (0,1 équivalent). Le milieu réactionnel est agité pendant 2 heures à 45°C, dilué avec de l'acétate d'éthyle (20 ml), lavé avec de l'eau (3x25 ml), séché sur sulfate de sodium, filtré puis concentré sous pression réduite. Une chromatographie sur gel de silice (dichlorométhane/méthanol : 98/2) du résidu permet d'isoler 61,2 mg du produit attendu.
Rendement : 62%
RMN ¹H (CDCl₃) δ (ppm) : 1,05 (q, 2H), 1,25 (t, 3H), 1,45 (q, 2H), 1,60 (m, 1H), 1,90 (d, 3H), 2,05 (d, 2H), 2,125 (m, 1H), 2,25 (m, 1H), 2,35 (m, 1H), 2,525 (m, 3H), 2,85 (m, 1H), 3,30 (t, 2H), 4,10 (q, 2H), 6,00 (bs, 1H), 6,25 (bs, 1H), 7,25 (m, 4H), 7,325 (m, 2H), 7,65 (s, 1H);
HPLC : 100,0%

### Exemple 25 : trans Acide 4-{[4-(4-phényl-cyclohex-1-ényl)-thiophèn-2-carboxamide] méthyl}-cyclohexane carboxylique

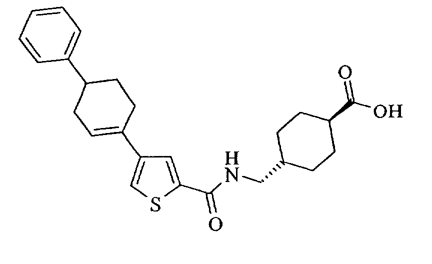

Le produit (48,2 mg) est obtenu selon le procédé du stade 7 de l'exemple 18, en utilisant comme substrat le produit obtenu lors de l'exemple 24.
Rendement : 88%
RMN ¹H (CDCl₃) δ (ppm) : 1,05 (q, 2H), 1,45 (q, 2H), 1,625 (m, 1H), 1,90 (m, 3H), 2,10 (m, 3H), 2,35 (m, 2H), 2,55 (m, 3H), 2,90( m, 1H), 3,30 (m, 2H), 6,05 (bs, 1H), 6,25 (bs, 1H), 7,25 (m, 4H), 7,35 (m, 2H), 7,65 (s, 1H);
HPLC : 99,4%

### Exemple 26 : 3-(6-{[4-(4-Tnfluorométhoxyphényl)thièn-2-yl]carboxamido} pyridin-3-yl)-propanoate d'éthyle

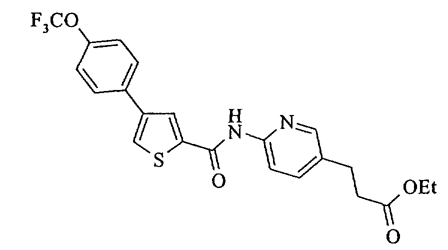

A une solution de 0,173 g du composé de la Préparation 5 dans 5 ml de dichlorométhane sont ajoutés, à 0°C, 0,1 g (1,1 équivalents) du composé de la Préparation 6, puis 0,15 ml (2,1 équivalents) de triéthylamine. Après 17 heures d'agitation à température ambiante, le milieu réactionnel est hydrolysé, lavé successivement avec une solution aqueuse 1N d'acide chlorhydrique, une solution saturée en NaHCO₃, une solution saturée en NaCl, puis séché sur sulfate de sodium, filtré et évaporé sous pression réduite. Une cristallisation dans le diisopropyléther permet d'isoler 0,093 g du produit attendu sous forme d'un solide blanc.
Rendement : 36%
RMN ¹H (CDCl₃) δ (ppm) : 8,6 (bs, 1H), 8,5( s,1H), 8,4(m, 1H), 7,8(m,2H), 7,7(m, 2H), 7,6(m,2H), 7,3(m,2H), 4,1(q,2H), 3,0(t,2H), 2,6(t,2H), 1,2(q,3H);
SM : ES+ 465
HPLC : 100%

### Exemple 27: Acide 3-(6-{[4-(4-trifluorométhoxyphényl)thièn-2-yl]carboxamido} pyridin-3-yl)-propanoïque

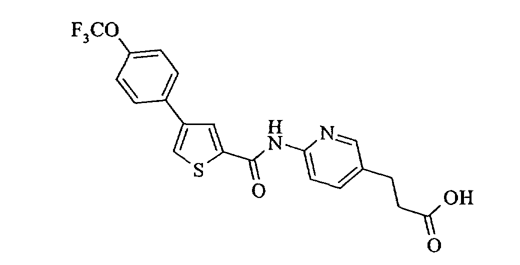

A une solution de 0,093 g du composé de l'exemple 26 dans 6,0 ml d'un mélange éthanol/eau (1/1 :v/v) est additionné 0,038 g (6 équivalents) d'hydroxyde de lithium . Le milieu réactionnel est agité 17 heures à température ambiante puis concentré sous pression réduite pour éliminer l'éthanol. La phase aqueuse est acidifiée par une solution aqueuse 1N d'acide chlorhydrique puis extraite à l'acétate d'éthyle. La phase organique est séchée sur sulfate de sodium, filtrée, puis concentrée sous pression réduite permettant d'obtenir 0,0087 g du produit attendu.
Rendement : 17%
RMN ¹H (DMSO) δ (ppm) : 12,5 (s, 1H), 8,8 (s, 1H), 8,3 (s, 1H), 8,1 (d, 1H), 7,9 (m, 1H), 7,7 (m, 2H), 7,6 (m,2H), 3,0 (t,2H), 2,6 (t,2H);
SM : ES+ 437, ES- 435
HPLC : 97,2%

### Exemple 28 : 3-(4-{[4-(4-Trifluorométhoxyphényl)thièn-2-yl]carboxamido} phényl)-propènoate d'éthyle

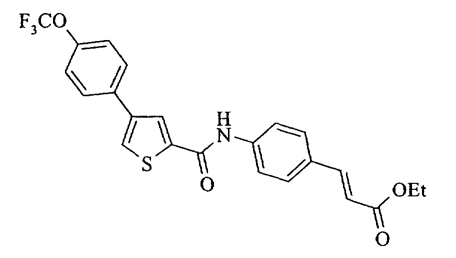

Le produit (0,146 g) est obtenu selon le procédé de l'exemple 26, en utilisant comme co-substrat le produit obtenu dans la préparation 7.
Rendement : 48%
RMN ¹H (CDCl₃) δ (ppm) : 7,9 (s, 1H), 7,7 (bs, 1H), 7,6 (m,6H), 7,5 (d, 2H), 7,25 (m,2H), 6,4 (d, 1H), 4,2 (q, 2H), 1,25 (t, 3H);
SM : ES- 459,9
HPLC : 100%

### Exemple 29: Acide 3-(4-{[4-(4-trifluorométhoxyphényl)thièn-2-yl]carboxamido} phényl)-propènoïque

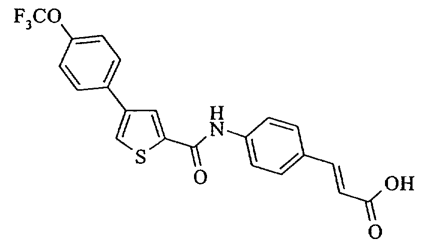

Le produit (0,094 g) est obtenu selon le procédé de l'exemple 27, en utilisant comme substrat le composé obtenu dans l'exemple 28.
Rendement : 69%
RMN ¹H (DMSO) δ (ppm): 10,45 (s, 1H), 8,5 (s, 1H), 8,3( s,1H), 7,85 (dd, 2H), 7,8 (dd, 2H), 7,7 (dd, 2H), 7,6 (d, 1H),7,5 (m, 2H), 6,4 (d, 1H);
SM: ES- 432
HPLC : 96,2%

### Exemple 30 : 4-({[4-<Trifluorométhoxyphényl)thièn-2-yl]carboxamido}méthyl) phtalate de diéthyle

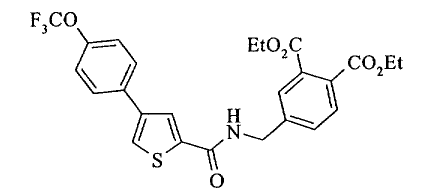

Le produit (0,106 g) est obtenu selon le procédé de l'exemple 26, en utilisant comme co-substrat le produit obtenu dans la préparation 8.
Rendement : 22%
RMN ¹H (CDCl₃) δ (ppm) : 7,7(s, 1H), 7,6 (m, 1H), 7,6( m, 1H), 7,5(m, 4H), 7,2(m, 1H), 6,4(bs, 1H), 4,8(d,2H),4,3(m,4H), 1,4(t, 6H);
HPLC : 91%

### Exemple 31: Diacide 4-({[4-(trifluorométhoxyphényl)thièn-2-yl]carboxamido} méthyl)-phtalique

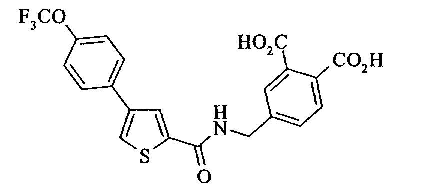

Le produit (0,0729 g) est obtenu selon le procédé de l'exemple 27, en utilisant comme substrat le composé obtenu dans l'exemple 30.
Rendement : 77,5%
RMN ¹H (DMSO) δ (ppm) : 9,2 (bs, 1H), 8,3(s, 1H), 8,2 (s, 1H), 7,85 (m, 2H), 7,7 (m, 2H), 7,5 (dd, 1H), 7,4 (dd, 2H), 4,5 (d, 2H);
SM : ES- 464
HPLC : 96,2%

### Exemple 32 : 3-(4-{[4-{4-Trifluorométhoxyphényl)thièn-2-yl]carboxamido} phényl)-propanoate d'éthyle

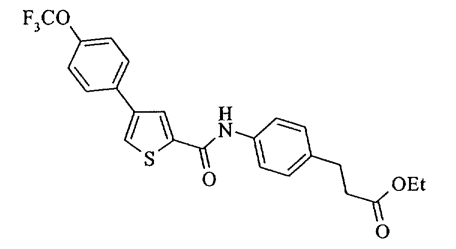

Le produit (0,483 g) est obtenu selon le procédé de l'exemple 26, en utilisant comme co-substrat le produit obtenu dans la préparation 9.
Rendement : 80%
RMN ¹H (CDCl₃) δ (ppm): 7,9 (s, 1H), 7,7 (m, 4H), 7,5 (m, 2H), 7,25 (m, 3H), 4,1 (q, 2H), 2,9 (t, 2H), 2,6 (t, 2H), 1,25 (t, 3H);
SM : ES+ 464, ES- 462
HPLC: 100%

### Exemple 33 : Acide 3-(4-{[4-(4-trifluorométhoxyphényl)thièn-2-yl]carboxamido} phényl)-propanoïque

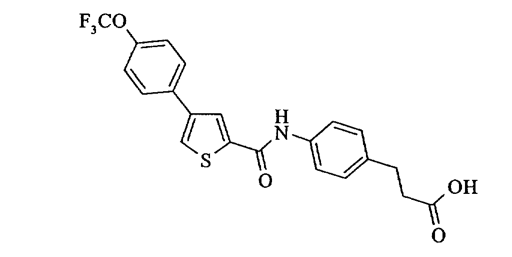

Le produit (0,279 g) est obtenu selon le procédé de l'exemple 27, en utilisant comme substrat le composé obtenu dans l'exemple 32.
Rendement : 61%
RMN ¹H (DMSO) δ (ppm) : 12,1 (s, 1H), 10,4 (s, 1H), 8,5 (s, 1H), 8,2 (s, 1H), 7,85 (dd, 2H), 7,6 (dd, 2H), 7,4 (dd, 2H), 7,2 (dd, 2H), 2,8(t, 2H), 2,5 (m, 2H);
SM : ES- 434
HPLC : 100%

### Exemple 34 : trans 4-({[4-(4-Trifluorométhoxyphényl)-thièn-2-yi]carboxamïdo} méthyl)-cyclohexane carboxylate de méthyle

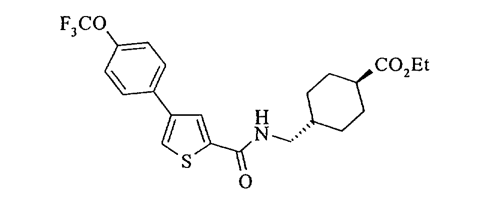

Le produit (0,120 g) est obtenu selon le procédé de l'exemple 26, en utilisant comme co-substrat le produit obtenu dans la préparation 10.
Rendement : 41%
RMN ¹H (CDCl₃) δ (ppm) : 7,7 (s, 1H), 7,6 (m, 2H), 7,5 (s, 1H), 6,0 (bs, 1H), 4,1 (q, 2H), 3,4 (t, 2H), 2,25 (m, 1H), 2,0 (m, 2H), 1,9 (m, 2H), 1,6 (m, 1H), 1,4 (m, 2H), 1,25 (t, 3H), 1,10 (q, 2H);
SM : ES+ 456, 500 (+ HCOOH)
HPLC : 98,6%

### Exemple 35 : trans Acide 4-({[4-(4-trifluorométhoxyphényl)-thièn-2-yl] carboxamide} méthyl)-cyclohexane carboxytique

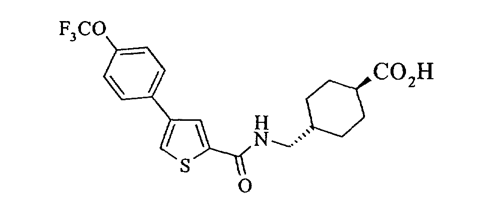

Le produit (0,112 g) est obtenu selon le procédé de l'exemple 27, en utilisant comme substrat le composé obtenu dans l'exemple 34.
Rendement : 100%
RMN ¹H (DMSO) δ (ppm) : 12,1 (s, 1H), 8,4 (bs, 1H), 8,2 (s, 1H), 8,1 (s, 1H), 7,85 (dd, 2H), 7,6 (dd, 2H), 3,1 (t, 2H), 2,1 (m, 1H), 1,8 (m, 2H), 1,75 (m, 2H), 1,5 (m, 1H), 1,2 (m, 2H), 1,0 (m, 2H);
SM :ES- 426
HPLC : 100%

### Exemple 36 : 2-[4-({l4-(4-Trinnorométhoxypbényl)thièn-2-yl]carboxamido} méthyl)phényl]-acétate d'éthyle

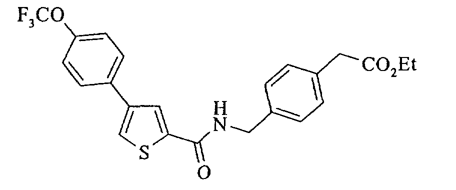

Le produit (0,036 g) est obtenu selon le procédé de l'exemple 26, en utilisant comme co-substrat le produit obtenu dans la préparation 11,
Rendement : 27%
RMN ¹H(CDCl₃) δ (ppm) : 7,8 (s, 1H), 7,55 (m, 3H), 7,25 (m, 6H), 6,45 (bs, 1H), 4,60 (d, 2H), 4,1 (q, 2H), 3,6 (s, 2H), 1,25 (t,3H);
SM: ES+ 463,7, 508 (+HCOOH)
HPLC : 94,9%

### Exemple 37 : Acide 2-[4-({[4-(4-trifluorométboxyphényl)thièn-2-yl]carboxamido} méthyl)phényl]-acétique

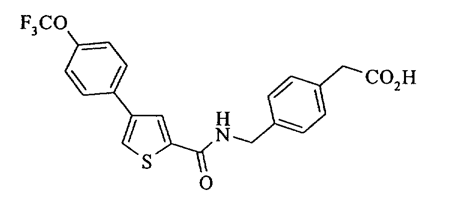

Le produit (0,0556 g) est obtenu selon le procédé de l'exemple 27, en utilisant comme substrat le composé obtenu dans l'exemple 36.
Rendement : 70%
RMN ¹H (DMSO) δ (ppm) : 12,1 (s, 1H), 9,1(bs, 1H), 8,25 (s, 1H), 8,15 (s, 1H), 7,85 (m, 2H), 7,4 (m, 2H), 7,3 (m, 1H), 7,2 (m, 2H), 7,1 (m, 1H), 4,4 (d, 2H), 3,4 (s, 2H);
SM : ES- 479,9
HPLC : 100%

### Exemple 38: 3-({[4-(4-Trilluorométhoxyphényl)thièn-2-yl]carboxamïdo}méthyl) benzoate d'éthyle

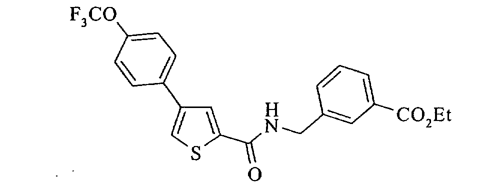

Le produit (0,070 g) est obtenu selon le procédé de l'exemple 26, en utilisant comme co-substrat le produit obtenu dans la préparation 12.
Rendement : 20%
RMN ¹H (CDCl₃) δ (ppm) : 8,05 (bs, 1H), 8,0 (d, 1H), 7,7 (s, 1H), 7,5 (m, 4H), 7,4 (t, 1H), 7,2 (m, 2H), 6,3 (bs, 1H), 4,7 (d, 2H), 4,4 (q, 2H), 1,4 (t, 3H);
SM : ES+ 450, 494 (+HCOOH)
HPLC : 97,2%

### Exemple 39 : Acide 3-({[4-(4-trifluorométhoxyphényl)thièn-2-yl]carboxamido} méthyl)benzoïque

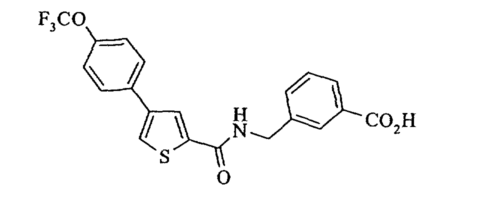

Le produit (0,0076 g) est obtenu selon le procédé de l'exemple 27, en utilisant comme substrat le composé obtenu dans l'exemple 38.
Rendement : 11%
RMN ¹H (DMSO) δ (ppm) : 12,1 (s, 1H), 9,1 (bs, 1H), 8,05 (bs, 1H), 8,0 (d, 1H), 7,7 (s, 1H), 7,5 (m, 4H), 7,4 (t, 1H), 7,2 (m, 2H), 4,7 (d, 2H);
SM : ES+ 422, ES- 420
HPLC : 100%

### Exemple 40: 2-[3-({[4-(4-Trifluorométhoxyphenyl)thièn-2-yl]carboxamido} méthyl)phényl]-acétate d'éthyle

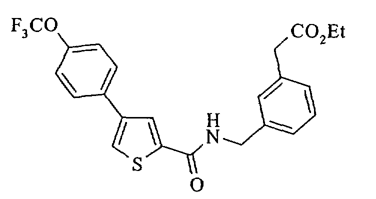

Le produit (0,038 g) est obtenu selon le procédé de l'exemple 26, en utilisant comme co-substrat le produit obtenu dans la préparation 13.
Rendement : 27%
RMN ¹H (CDCl₃) δ (ppm) : 7,8 (s, 1H), 7,6 (m, 3H), 7,3 (m, 6H), 6,45 (bs, 1H), 4,65 (d, 2H), 4,1 (q, 2H), 3,7 (s, 2H), 1,2 (t, 3H);
SM : ES+ 463,7, 508 (+HCOOH)
HPLC : 94,9%

### Exemple 41 : Acide 2-[3-({[4-(4-trifluorométhoxyphényl)thièn-2-yl]carboxamido} méthyl)phényl]-acétique

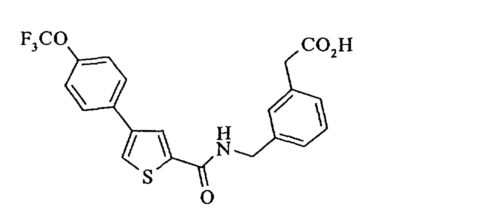

Le produit (0,023 g) est obtenu selon le procédé de l'exemple 27, en utilisant comme substrat le composé obtenu dans l'exemple 40.
Rendement : 65%
RMN ¹H (DMSO) δ (ppm) : 12,1 (s, 1H), 9,1 (bs, 1H), 8,30 (s, 1H), 8,2 (s, 1H), 7,7 (m, 2H), 7,5 (m, 2H), 7,3 (m, 1H), 7,2 (m, 2H), 7,1 (m, 1H), 4,7 (d, 2H), 3,6 (s, 2H);
SM : ES+ 436, ES- 435
HPLC : 98,4%

### Exemple 42 : 4-{(4-(4-Trifluorométhoxypheylfithiophèn-2-carboxamidelméthyl}-cyclohexane carboxylate d'éthyle

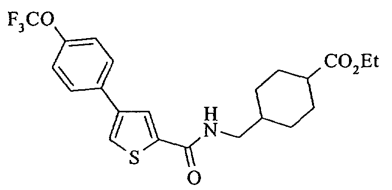

Le produit (0,116 g) est obtenu selon le procédé de l'exemple 26, en utilisant comme co-substrat le produit obtenu dans la préparation 14.
Rendement : 23%
RMN ¹H (CDCl₃) δ (ppm) : 7,8 (s, 1H), 7,6 (m, 3H), 7,3 (m, 2H), 6,20 (bs, 1H), 4,10 (m, 2H), 3,3 (m, 2H), 2,1 (m, 1H), 2,05 (m, 2H), 1,9 (m, 2H), 1,7 (m, 1H), 1,45 (m, 2H), 1,3 (t, 3H), 1,0 (m, 2H);
HPLC : 100%

### Exemple 43: Acide 4-{[4-(4-triflaorométhoxyphényl)-thiophèn-2-carboxamide] méthyl}-cyclohexane carboxylique

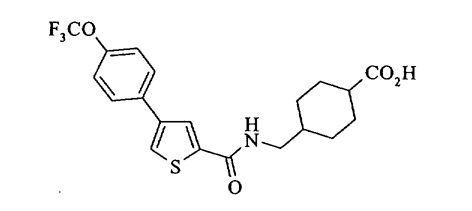

Le produit (0,082 g) est obtenu selon le procédé de l'exemple 27, en utilisant comme substrat le composé obtenu dans l'exemple 42.
Rendement : 78,5%
RMN ¹H (DMSO) δ (ppm): 12,1 (s, 1H), 8,5 (bs, 1H), 8,30 (s, 1H), 8,2 (s, 1H), 7,7 (m, 2H), 7,5 (m, 2H), 3,1 (t, 2H), 2,2 (m, 1H), 1,9 (m, 2H), 1,8 (m, 2H), 1,6 (m, 1H), 1,3 (m, 2H), 1,0 (m, 2H);
SM : ES- 472
HPLC :100%

### Exemple 44 : 6-({[4-(4-Trifluoroniéthoxyphényl)thièn-2-yl]carboxamido)méthyl)-nicotinate de méthyle

### Stade 1: 4-(4-Trifluorométhoxyphényl)thiophène-2-carboxamide

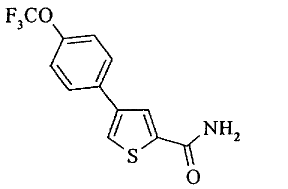

A une solution de 2,744 g du composé de la Préparation 5 dans 25 ml de tétrahydrofurane est ajouté, à -78°C, 0,34 ml d'une solution d'ammoniac dans 5 ml de tetrahydrofurane. Après 2 heures d'agitation à température ambiante, le milieu réactionnel est concentré sous pression réduite, hydrolysé à l'eau et extrait au dichlorométhane. La phase organique est lavée avec une solution saturée en NaCl, séchée sur sulfate de sodium, filtrée et évaporée sous pression réduite. Une cristallisation permet d'isoler 1,569 g du produit attendu sous forme d'un solide blanc.
Rendement : 61,72%
RMN ¹H (DMSO) δ (ppm) : 8,2 (s, 1H), 8,1 (s, 1H), 8,0 (bs, 1H), 7,8 (m, 2H), 7,50 (m, 2H);
HPLC : 96,92%

### Stade 2 : 6-({[4-(4-Trifluorométhoxyphényl)thièn-2-yl]carboxamido}méthyl)-nicotinate de méthyle

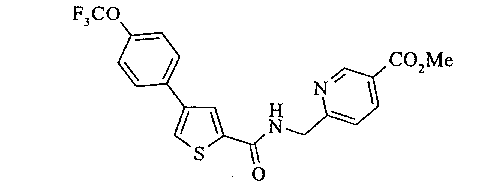

Une solution de 0,0347 g d'hydrure de sodium à 60% dans 2,0 ml de DMF à 0°C est agitée 2 heures à 40°C. Une solution de 0,249 g du composé obtenu au stade 1 précédent et de 0,2 g du composé de la préparation 15 dans 1 ml de DMF est agitée à 40°C pendant 2 heures. Le solvant est évaporé sous pression réduite. Le milieu réactionnel est repris dans de l'acétate d'éthyle, lavé à l'eau et séché sur sulfate de sodium, pour obtenir après filtration et concentration sous pression réduite 0,139 g d'une gomme verte. Une purification sur colonne semi-préparative permet d'obtenir 0,012 g d'une poudre blanche correspondant au produit attendu.
Rendement : 3%
SM : ES+ 437
HPLC : 100%

### Exemple 45 : Acide 6-({[4-(4-trifluorométboxyphényl}thièn-2-yl]carboxamido} méthyl)-nicotinique

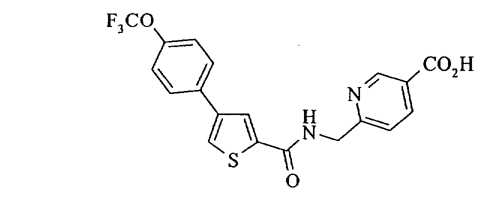

Le produit (0,006 g) est obtenu selon le procédé de l'exemple 27, en utilisant comme substrat le composé obtenu dans l'exemple 44.
Rendement : 79%
RMN ¹H (DMSO) δ (ppm) : 13,2 (s, 1H), 9,25 (bs, 1H), 9,0 (s, 1H), 8,3 (s, 1H), 8,25 (m, 1H). 8,2 (s, 1H), 7,8 (m, 2H), 7,6 (m, 3H), 4,6 (d, 2H);
SM : ES+ 423, ES- 421
HPLC : 100%

### Exemple 46 : 4-({[4-(4-Trifluorométhoxyphényl)thièn-2-yl]carboxamido}méthyl) benzoate d'éthyle

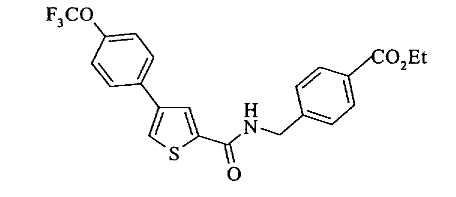

Le produit (0,051 g) est obtenu selon le procédé de l'exemple 1 en utilisant comme substrat le composé obtenu lors de la préparation 17 et comme co-substrat l'acide 4-(trifluorométhoxy)phényl-boronique.
Rendement : 42%
SM : ES+ 449,9
HPLC : 94%

### Exemple 47 : Acide 4-({[4-(4-trifluorométhoxyphényl)thièn-2-yl]carboxamido} méthyl)benzoïque

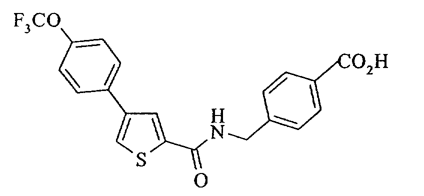

Le produit (0,008 g) est obtenu selon le procédé de l'exemple 27, en utilisant comme substrat le composé obtenu dans l'exemple 46.
Rendement : 17%
RMN ¹H (DMSO) δ (ppm) : 9,2(bs, 1H), 8,3 (s, 1H), 8,2 (s, 1H), 7,85 (m, 2H), 7,7 (m, 2H), 7,4 (m, 4H), 4,5 (d, 2H);
SM : ES- 420
HPLC : 100%

### Exemple 48 : 4-({[4-(4-Méthylthiophényl)thièn-2-yl]carboxamido}méthyl)benzoate d'éthyle

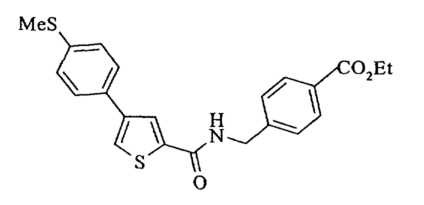

Le produit (0,1g) est obtenu selon le procédé de l'exemple 46, en utilisant comme co-substrat l'acide 4-(méthylthio)phénylboronique.
Rendement : 42%
RMN ¹H (CDCl₃) δ (ppm) : 8,1 (m, 2H), 7,8 (s, 1H), 7,6 (s, 1H), 7,40 (m, 4H), 7,2 (m, 2H), 6,3 (bs, 1H), 4,7 (m, 2H), 4,4 (m, 2H), 2,5 (s, 3H), 1,4(t, 3H);
HPLC : 90,1%

### Exemple 49 : Acide 4-({[4-(4-méthylthiophényl)thièn-2-yl]carboxamido}méthyl) benzoïque

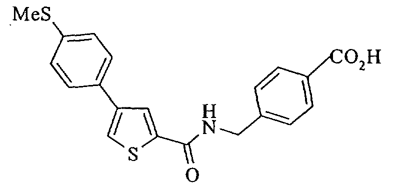

Le produit (0,0162 g) est obtenu selon le procédé de l'exemple 27, en utilisant comme substrat le composé obtenu dans l'exemple 48.
Rendement : 17%
RMN ¹H (DMSO) δ (ppm) : 12,9 (s, 1H), 9,2 (bs, 1H), 8,2 (s, 1H), 8,1 (s, 1H), 7,9 (m, 2H), 7,7 (m, 2H), 7,4 (m, 2H), 7,2 (m, 2H), 4,4 (d, 2H), 3,3 (s, 3H);
SM : ES+ 384, 428 (+HCOOH)
HPLC : 96,31%

### Exemple 50 : 4-({[4-(4-tert-Butylphényl)thièn-2-yl]carboxamido}méthyl)benzoate d'éthyle

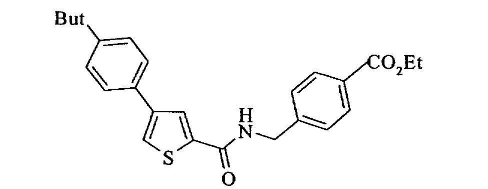

Le produit (0,16 g) est obtenu selon le procédé de l'exemple 46, en utilisant comme co-substrat l'acide 4-(t-butyl)phénylboronique.
Rendement : 67%
RMN ¹H (CDCl₃) δ (ppm) : 8,0 (m, 2H), 7,8 (s, 1H), 7,6 (s, 1H), 7,40 (m, 2H), 7,2 (m, 4H), 6,3 (bs, 1H), 4,7 (m, 2H), 4,4 (m, 2H), 1,4 (t, 3H);
SM : ES+ 422
HPLC : 100%

### Exemple 51 : Acide 4-({[4-(4-tert-butylphényl )thién-2-yl]carboxamido}méthyl) benzoïque

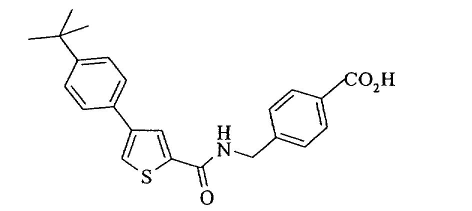

Le produit (0,152 g) est obtenu selon le procédé de l'exemple 27, en utilisant comme substrat le composé obtenu dans l'exemple 50.
Rendement : 85,4%
RMN ¹H (DMSO) δ (ppm) : 12,9 (s, 1H), 9,2 (bs, 1H), 8,2 (s, 1H), 8,1 (s, 1H), 7,85 (m, 2H), 7,6 (m, 2H), 7,4 (m, 4H), 4,5 (d, 2H), 1,3 (s, 9H);
SM : ES+ 394, ES- 392
HPLC : 100%

### Exemple 52 : (4-{[4-(4-tert-Butylphényl)thièn-2-yl]carboxamido}phényl)acétate d'éthyle

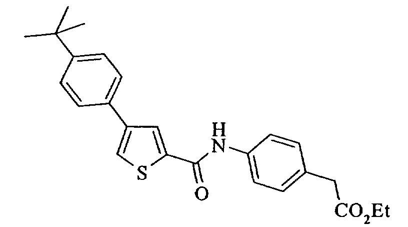

Le produit (0,141 g) est obtenu selon le procédé de l'exemple 46, en utilisant comme substrat le composé obtenu dans la préparation 18 et l'acide 4-tert-butylphénylboronique.
Rendement : 60%
RMN ¹H (CDCl₃) δ (ppm) : 7,9 (bs, 1H), 7,6 (s, 1H), 7,55 (m, 3H), 7,5 (m, 2H), 7,4 (m, 2H), 7,3 (m, 2H), 4,1 (q, 2H), 3,6 (s, 2H), 1,35 (s, 9H), 1,25 (t, 3H);
SM : ES+ 422
HPLC : 100%

### Exemple 53 : Acide (4-{[4-(4-tert-butylphényl)thièn-2-yl]carboxamido}phényl) acétique

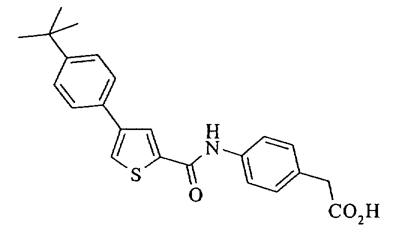

Le produit (0,113 g) est obtenu selon le procédé de l'exemple 27, en utilisant comme substrat le composé obtenu dans l'exemple 52.
Rendement : 85,4%
RMN ¹H (DMSO) δ (ppm) : 12,1 (s, 1H), 10,4 (s, 1H), 8,5 (s, 1H), 8,2 (s, 1H), 7,7 (m, 4H), 7,6 (m, 2H), 7,4 (m, 2H), 3,5 (s, 2H), 1,4 (s, 9H);
SM : ES- 392, ES+ 394
HPLC : 100%

### Exemple 54 : (4-{[4-(4-Trifluorométhoxyphényl)thièn-2-yl]carboxamido)phényl) acétate d'éthyle

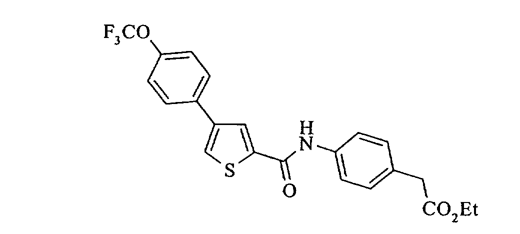

Le produit (0,0675g) est obtenu selon le procédé de l'exemple 46, en utilisant comme substrat le composé obtenu dans la préparation 18 et comme co-substrat l'acide 4-(trifluorométhoxy)phényl-boronique.
Rendement : 40%
RMN ¹H (CDCl₃) δ (ppm) : 7,8 (s, 1H), 7,7 (s, 1H), 7,5 (m, 4H), 7,25 (m, 4H), 4,2 (q, 2H), 3,6 (s, 2H), 1,3 (t, 3H);
SM : ES+ 449, 494 (+HCOOH)
HPLC : 91%

### Exemple 55 : Acide (4-{[4-(4-trifluorométhoxyphényl)thièn-2-yl]carboxamido} phényl)acétique

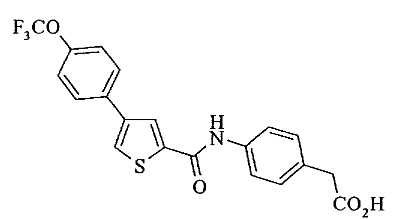

Le produit (0,080 g) est obtenu selon le procédé de l'exemple 27, en utilisant comme substrat le composé obtenu dans l'exemple 54.
Rendement : 54%
RMN ¹H (DMSO) δ (ppm) : 12,1 (s, 1H), 10,4 (s, 1H), 8,6 (s, 1H), 8,2 (s, 1H), 7,85 (m, 2H), 7,6 (m, 2H), 7,4 (m, 2H), 7,2 (m, 2H), 3,5 (s, 2H);
SM : ES- 420, ES+ 422
HPLC : 97,02%

### Exemple 56 : (4-{[4-(4-Méthylthiophényl)thièn-2-yl]carboxamido}phényl) acétate d'éthyle

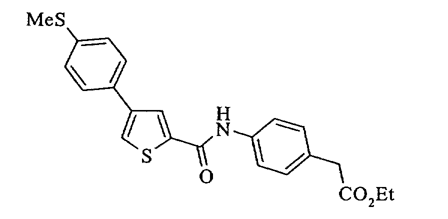

Le produit (0,119 g) est obtenu selon le procédé de l'exemple 46, en utilisant comme substrat le composé obtenu dans la préparation 18 et comme co-substrat l'acide 4-(méthylthio)phénylboronique.
Rendement : 51%
RMN ¹H (CDCl₃) δ (ppm) : 7,8 (s, 1H), 7,6 (s, 1H), 7,5 (m, 3H), 7,45 (m, 2H), 7,3(m, 4H), 4,2 (q, 2H), 3,7 (s, 2H), 2,5 (s, 3H), 1,3 (t, 3H) ;
SM : ES- 411,ES+ 456
HPLC : 97,73%

### Exemple 57: Acide (4-{[4-(4-méthylthiophényl)thièn-2-yl]carboxamido}phényl) acétique

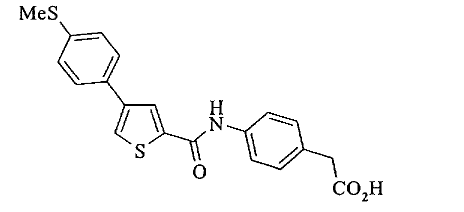

Le produit (0,083 g) est obtenu selon le procédé de l'exemple 27, en utilisant comme substrat le composé obtenu dans l'exemple 56.
Rendement : 75%
RMN ¹H (DMSO) δ (ppm) : 12,1 (s, 1H), 10,4 (s, 1H), 8,5 (s, 1H), 8,2 (s, 1H), 7,85 (m, 4H), 7,3 (m, 2H), 7,4 (m, 2H), 3,5 (s, 3H) ;
SM : ES- 382
HPLC : 100%

### Exemple 58: 4-({[4-(4-Méthoxyphényl)thièn-2-yl]carboxamido}méthyl)benzoate d'éthyle

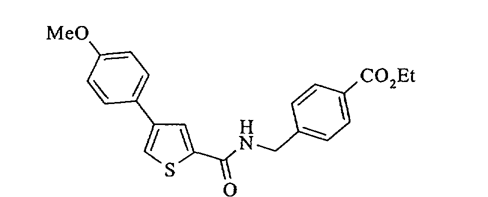

Le produit (0,300 g) est obtenu selon le procédé de l'exemple 26, en utilisant comme substrats le produit de la préparation 19 et le 4-(aminométhyl)-benzoate d'éthyle.
Rendement : 52%
RMN ¹H (DMSO) δ (ppm) : 9,1 (bs, 1H), 8,2 (s, 1H), 7,9 (m, 3H), 7,6 (m, 2H), 7,4 (m, 2H), 7,0 (m, 2H), 4,5 (d, 2H), 4,3 (q, 2H), 3,8 (s, 3H), 1,3 (t, 3H);
SM : ES+ 383
HPLC : 97,2%

### Exemple 59: Acide 4-({(4-(4-Méthoxyprhényl)thièn-2-yl]carhoxamido}méthyl) benzoïque

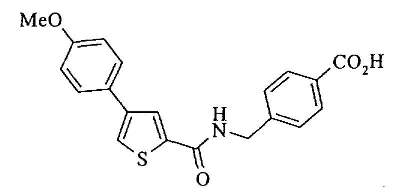

Le produit (0.060g) est obtenu selon le procédé de l'exemple 27, en utilisant comme substrat le composé obtenu dans l'exemple 58.
Rendement : 31%
RMN ¹H (DMSO) δ ppm : 12,9 (s, 1H), 9,1 (bs, 1H), 8,2 (s, 1H), 7,9 (s, 1H), 7,85 (m, 2H), 7,6 (m, 2H), 7,4 (m, 2H), 7,0 (m, 2H), 4,5 (d, 2H), 3,8 (s, 3H);
SM : ES+ 368
HPLC : 100%

### Exemple 60 : (4-{[4-(4-Méthoxyphényl)thièn-2-yl]carboxamido}phényl) acétate d'éthyle

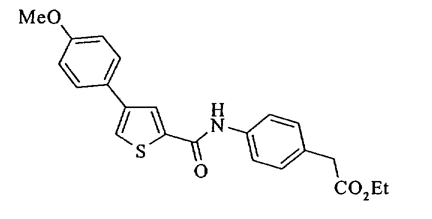

Le produit (0,16 g) est obtenu selon le procédé de l'exemple 26, en utilisant comme substrats le produit de la préparation 19 et celui de la préparation 18 stade 1.
Rendement : 93%
RMN ¹H (DMSO) δ (ppm) : 10,2 (bs, 1H), 8,4 (s, 1H), 7,9 (s, 1H), 7,6 (m, 4H), 7,3 (m, 2H), 7,0 (m, 2H), 4,1 (q, 2H), 3,8 (s, 3H), 3,6 (s, 2H), 1,3 (t, 3H);
SM : ES+ 395
HPLC : 100%

### Exemple 61: Acide (4-{[4-(4-méthoxyphényl)thièn-2-yl]carboxamido} phényl)acétique

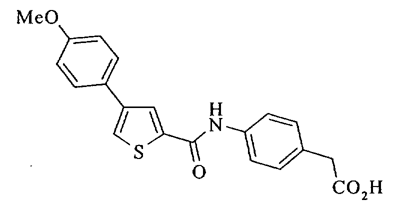

Le produit (0,0615 g) est obtenu selon le procédé de l'exemple 27, en utilisant comme substrat le composé obtenu de l'exemple 60.
Rendement : 41%
RMN ¹H (DMSO) δ (ppm) : 12,2 (s, 1H), 10,2 (s, 1H), 8,5 (s, 1H), 8,0 (s, 1H), 7,75 (m, 4H), 7,3 (m, 2H), 7,0 (m, 2H), 3,8 (s, 3H), 3,5 (s, 2H);
SM : ES- 366, ES+ 367,8
HPLC : 100%

### Exemple 62 : 3-[4-({[4-(4-Trifluorométhoxyphényl)thièn-2-yl]carboxamido} méthyl)pkényl]propanoate d'éthyle

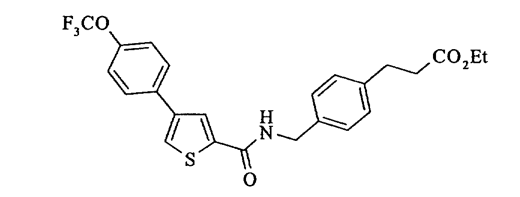

Le produit (0.084g) est obtenu selon le procédé de l'exemple 26, en utilisant comme substrats le produit de la préparation 5 et le produit de la préparation 20.
Rendement : 14%
RMN ¹H (CDCl₃) δ ppm : 7,9 (s, 1H), 7,6 (m, 3H), 7,3 (m, 2H), 7,2 (m, 4H), 6,2 (bs, 1H), 4,6 (m, 2H), 4,2 (q, 2H), 3,6 (s, 2H), 1,3 (t, 3H);
HPLC : 100%

### Exemple 63 : Acide 3-[4-({[4-(4-trifluorométhoxyphényl)thièn-2-yl]carboxamido} méthyl)phényl]propanoïque

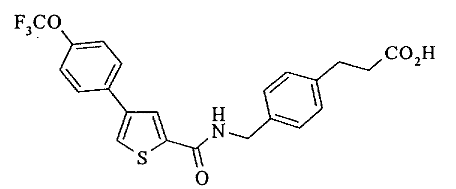

Le produit (0,0556 g) est obtenu selon le procédé de l'exemple 27, en utilisant comme substrat le composé obtenu dans l'exemple 62.
Rendement : 95%
RMN ¹H (DMSO) δ (ppm) : 12,1 (s, 1H), 9,1 (bs, 1H), 8,3 (s, 1H), 8,2 (s, 1H), 7,85 (m, 2H), 7,5 (m, 2H), 7,2 (m, 2H), 7,1 (m, 2H), 4,4 (d, 2H), 3,3 (m, 2H), 2,7 (m, 2H);
SM : ES- 494
HPLC : 100%

### Exemple 64 : 3-[3-({[4-(4-Trifluorométhoxyphényl)thièn-2-yl]carboxamido} méthyl)phényl]propanoate d'éthyle

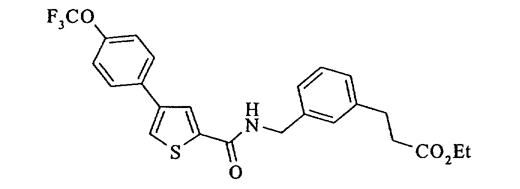

Le produit (0.243 g) est obtenu selon le procédé de l'exemple 26, en utilisant comme substrats le produit de la préparation 5 le produit de la préparation 21.
Rendement : 41.3%
RMN ¹H (CDCl₃) δ ppm : 7,8 (s, 1H), 7,5 (m, 3H), 7,2 (m, 6H), 6,2 (bs, 1H), 4,7 (d, 2H), 4,1 (q, 2H), 3,0 (m, 2H), 2,6 (m, 2H), 1,2 (t, 3H);
HPLC : 100%

### Exemple 65: Acide 3-[3-({[4-(4-trifluorométhoxyphényl)thién-2-yl]carboxamido} méthyl)phényl]propanoïque

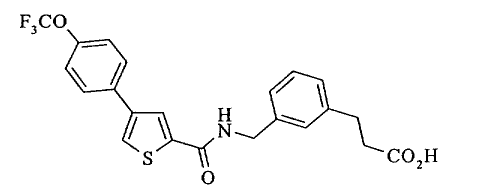

Le produit (0,1708 g) est obtenu selon le procédé de l'exemple 27, en utilisant comme substrat le composé obtenu dans l'exemple 64.
Rendement : 75%
RMN ¹H (DMSO) δ (ppm) : 12,1 (s, 1H), 9,1 (bs, 1H), 8,3 (s, 1H), 8,2 (s, 1H), 7,85 (m, 2H), 7,5 (m, 2H), 7,2 (m, 2H), 7,1 (m, 2H), 4,4 (d, 2H), 2,7 (m, 2H), 2,5( m, 2H);
SM : ES- 448
HPLC : 100%

### Exemple 66 : 7-{[4-(4-Trifluorométhoxyphényl)thièn-2-yl]carboxamido} heptanoate d'éthyle

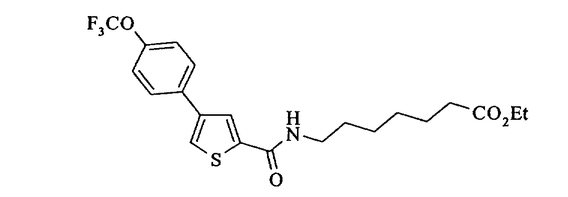

Le produit (0,363 g) est obtenu selon le procédé de l'exemple 26, en utilisant comme substrats le produit de la préparation 5 et le produit de la préparation 22.
Rendement : 32%
RMN ¹H (CDCl₃) δ ppm : 7,8 (s, 1H), 7,7 (m, 2H), 7,6 (s, 1H), 7,3 (m, 2H), 6,3 (bs, 1H), 4,1 (m, 2H), 3,5 (q, 2H), 2,4 (t, 2H), 1,7 (m, 4H), 1,5(m, 2H), 1,3 (t, 3H);
HPLC : 100%

### Exemple 67: Acide 7-{[4-(4-trifluorométhoxyphényl)thièn-2-yl]carboxamido} heptanoïque

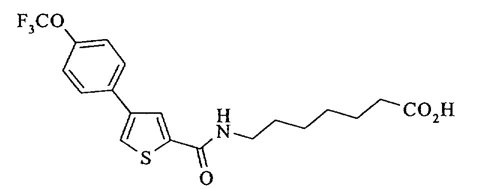

Le produit (0,284 g) est obtenu selon le procédé de l'exemple 27, en utilisant comme substrat le composé obtenu dans l'exemple 66.
Rendement : 84%
RMN ¹H (DMSO) δ (ppm) : 12,1 (s, 1H), 8,5 (bs, 1H), 8,3 (s, 1H), 8,2 (s, 1H), 7,85 (m, 2H), 7,5 (m, 2H), 3,4 (m, 2H), 2,4 (m, 2H), 1,5 (m, 4H), 1,3 (m, 2H);
SM : ES+ 402, ES- 400
HPLC : 100%

### Exemple 68 : 4-({[4-(4-Hydroxyphényl)thièn-2-yl]carboxamido}méthyl) cyclohexane carboxylate d'éthyle

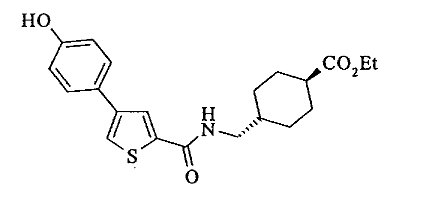

Le produit (0,974 g) est obtenu selon le procédé de la préparation 5 stade 1, en utilisant comme substrat le composé obtenu dans la préparation 3 stade 1 et l'acide (4-hydroxyphényl)boronique.
Rendement : 40%
RMN ¹H (CDCl₃) δ (ppm) : 7,7 (s, 1H), 7,4 (m, 3H), 6,8 ( m, 2H), 6,2 (bs, 1H), 4,1 (q, 2H), 3,3 (m, 2H), 2,3 (m, 1H), 2,1 (m, 2H), 1,9 (m, 2H), 1,7 (m, 1H), 1,4 (m, 2H), 1,25 (t, 3H), 1,1 (m, 2H);
SM : ES+ 387,7
HPLC : 100%

### Exemple 69: 4-[({4-[4-(Pyridin-4-yl)phényl]thièn-2-yl}carboxamido)méthyl] cyclohexane carboxylate d'éthyle

### Stade 1: 4-({[4-(4-Trifluorométhansulfonyloxyphényl)thièn-2-yl]carboxamido} méthyl)cyclohexane carboxylate d'éthyle

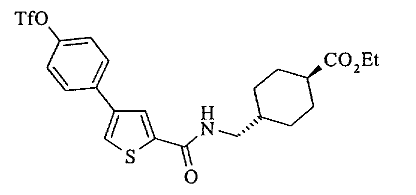

Le produit (1,136 g) est obtenu selon le procédé de l'exemple 18 stade 3, en utilisant comme substrat le produit obtenu à l'exemple 68.
Rendement : 100%
RMN ¹H (CDCl₃) δ (ppm) : 7,8 (s, 1H), 7,7 (m, 2H), 7,6 (s, 1H), 7,3 (m, 2H), 6,3 (bs, 1H), 4,1 (q, 2H), 3,3 (m, 2H), 2,4 (m, 1H), 2,0 (m, 2H), 1,9 (m, 2H), 1,7 (m, 1H), 1,6 (m, 2H), 1,5 (t, 3H), 1,0 (m, 2H);
SM : ES+ 520, 564 (+HCOOH)
HPLC : 100%

### Stade 2 : 4-[([4-[4-(Pyridin-4-yl)phényl]thièn-2-yl}carboxamido)méthyl]cyclohexane carboxylate d'éthyle

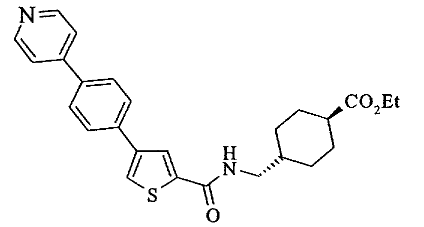

Le produit (0,133 g) est obtenu selon le procédé de la préparation 5 stade 1, en utilisant comme substrat le composé obtenu au stade 1 précédent et comme co-substrat l'acide pyridine-4-boronique.
Rendement : 75%
RMN ¹H (DMSO) δ (ppm) : 12,1 (s, 1H), 9,1 (bs, 1H), 8,3 (s, 1H), 8,2 (s, 1H), 7,85 (m, 2H), 7,5 (m, 2H), 7,2 (m, 2H), 7,1 (m, 2H), 4,4 (d, 2H), 2,7 (m, 2H), 2,5(m, 2H);
SM : ES- 448
HPLC : 100%

### Exemple 70 : Chlorhydrate d'acide 4-[({4-[4-(pyridin-4-yl)phényl]thièn-2-yl} carboxamido)méthyl] cyclohexane carboxylique

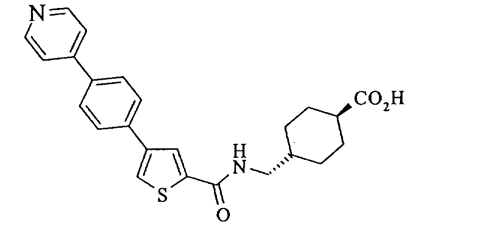

Le produit est obtenu selon le procédé de l'exemple 27, en utilisant comme substrat le composé obtenu dans l'exemple 69. Le solide est agité dans 2,0 ml de diéthyléther et 1,8 ml d'une solution 1,0 M HCl/Et₂O sont ajoutés goutte à goutte. Le précipité est filtré, lavé à l'eau et séché à 60°C sous vide pour obtenir le produit attendu (m : 0,0507 g).
Rendement : 15,2%
RMN ¹H (DMSO) δ (ppm) : 12,1 (s, 1H), 9,2 (m, 2H), 8,6 (s, 1H), 8,4 (m, 3H), 8,1 (m, 2H), 7,8 (m, 2H), 3,1 (d, 2H), 2,2 (m, 1H), 1,9 (m, 2H), 1,8 (m, 2H), 1,5(m, 1H), 1,3 (m, 2H), 1,0 (m, 2H);
SM : ES+ 421
HPLC : 97,58%

### Exemple 71: 4-(4-Bromophényl)-N-(2-morpholin-4-yléthyl)thiophène-2-carboxamide

### Stade 1: 4-(4-Bromophényl)thiophène-2-carboxylate de méthyle

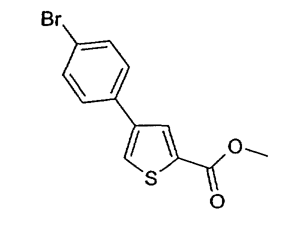

Au produit obtenu lors du stade 2 de l'exemple 19 sont ajoutés 1,5 ml d'eau et 0,6 ml d'acide bromhydrique concentré. Le milieu réactionnel est refroidi à 0°C, puis une solution de 35,5 mg de nitrite de sodium (1,1 équivalent) dans 0,5 ml d'eau est ajoutée goutte à goutte. Après 1 heure d'agitation à 0°C, une solution de 68 mg de bromure de cuivre dans 0,5 ml d'acide bromhydrique concentré est ajoutée goutte à goutte. Le milieu réactionnel est de nouveau agité pendant 1 heure à 0°C puis dilué avec de l'acétate d'éthyle (30 ml), lavé avec de l'eau (3x15 ml), lavé avec une solution saturée d'hydrogénocarbonate de sodium (15 ml) puis de nouveau avec de l'eau (15 ml). La phase organique est séchée sur sulfate de sodium, filtrée puis concentrée sous pression réduite. Une chromatographie sur gel de silice (cyclohexanelacétate d'éthyle : 95/5) du résidu permet d'isoler 52,1 mg du produit désiré.
Rendement : 40%
RMN ¹H (CDCl₃) δ (ppm) : 3,925 (s, 3H), 7,45 (d, 2H), 7,55 (d, 2H), 7,65 (s, 1H), 8,05 (s, 1H);
HPLC : 91,4%

### Stade 2 : Acide 4-(4-bromophényl)thiophène-2-carboxylique

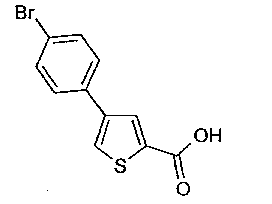

Le produit (11,32 g) est obtenu selon le procédé du stade 7 de l'exemple 19, en utilisant comme substrat le composé du stade 1 précédent.
Rendement : 99%
SM : MH⁻ : 282
HPLC : 99,3%

### Stade 3: 4-(4-Bromophényl)-N-(2-morpholin-4-yléthyl)thiophène-2-carboxamide

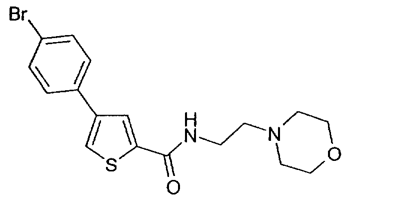

Le produit (11,58 g) est obtenu selon le procédé du stade 2 de la préparation 1, en utilisant comme substrat le composé du stade 2 précédent.
Rendement : 73%
RMN ¹H (CDCl₃) δ (ppm) : 2,45 (m, 4H), 2,55 (m, 2H), 3,50 (m, 2H), 3,675 (m, 4H), 6,60 bs, 1H), 7,35 (d, 2H), 7,45 (m, 3H), 7,675 (s, 1H);
SM : MH⁺ : 396
HPLC : 97,3%

### Exemple 72: 4-[4-(4-Acétylphényl)phényll-N-(2-morpholin-4-yléthyl) thiophène-2-carboxamide

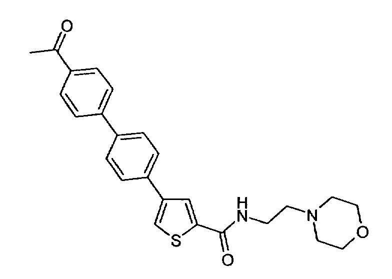

Le produit (86,8 mg) est obtenu selon le procédé du stade 1 de la préparation 5, en utilisant comme substrat le composé de l'exemple 71 et comme co-substrat l'acide 4-acétylphénylboronique.
Rendement : 78%
RMN ¹H (DMSO) δ (ppm) : 2,45 (m, 4H), 2,50 (m, 2H), 2,625 (s, 3H), 3,40 (m, 2H), 3,60 (m, 4H), 7,85 (m, 6H), 8,05 (m, 2H), 8,175 (s, 1H), 8,275 (s, 1H), 8,50 (bs, 1H);
SM : MH⁺ : 435
HPLC : 98,1%

### Exemple 73 : 4-[4-(4-Fluorophényl)phényl]-N-(2-morpholin-4-yléthyl) thiophène-2-carboxamide

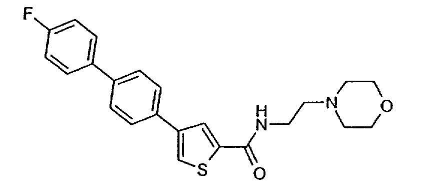

Le produit (84,5 mg) est obtenu selon le procédé de l'exemple 72, en utilisant comme co-substrat l'acide 4-fluorophénylboronique.
Rendement : 81%
RMN ¹H (DMSO) δ (ppm) : 2,45 (m, 4H), 2,50 (m, 2H), 3,40 (m, 2H), 3,60 (m, 4H), 7,30 (m, 2H), 7,80 (m, 6H), 8,125 (s, 1H), 8,25 (s, 1H), 8,50 (bs, 1H);
SM : MH⁺ : 411
HPLC : 100,0%

### Exemple 74 : 4-[4-(3-Hydroxyphényl)phényl]-N-(2-morpholin-4-yléthyl) thiophène-2-carboxamide

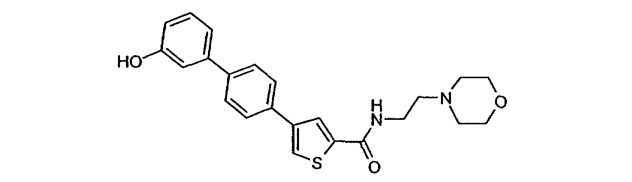

Le produit (61,5 mg) est obtenu selon le procédé de l'exemple 72, en utilisant comme co-substrat l'acide (3-hydroxyphényl)boronique.
Rendement : 59%
RMN ¹H (DMSO) δ (ppm) : 2,45 (m, 4H), 2,50 (m, 2H), 3,40 (m, 2H), 3,60 (m, 4H), 6,80 (m, 1H), 7,05 (s, 1H), 7, 15 (m, 1H), 7,275 (m, 1H), 7,70 (d, 2H), 7,775 (d, 2H), 8,125 (s, 1H), 8,25 (s, 1H), 8,50 (bs, 1H), 9,525 (s, 1H);
SM : MH⁺ : 409
HPLC : 100,0%

### Exemple 75 : 4-[4-(4-Méthylsulfenylphényl)phényl)phényl]-N-(2-morpholin-4-yléthyl)thiophène-2-carboxamide

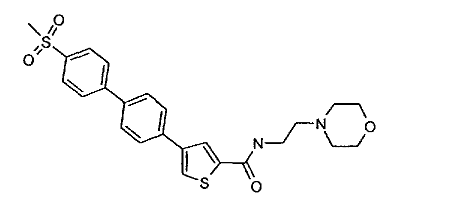

Le produit (149,5 mg) est obtenu selon le procédé de l'exemple 72, en utilisant comme co-substrat l'acide [(4-méthylsulfonyl)phényl]boronique.
Rendement : 98%
RMN ¹H (DMSO) δ (ppm) : 2,45 (m, 4H), 2,50 (m, 2H), 3,275 (s, 3H), 3,40 (m, 2H), 3,60 (m, 4H), 7,85 (m, 4H), 8,05 (s, 4H), 8,20 (s, 1H), 8,30 (s, 1H), 8,55 (bs, 1H);
SM : MH⁺ : 471
HPLC : 98,1%

### Exemple 76 : 4-[4-(3-Acétylphényl)phényl]-N-(2-morpholin-4-yléthyl) thiophène-2-carboxamide

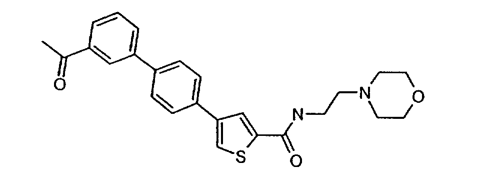

Le produit (113,6 mg) est obtenu selon le procédé de l'exemple 72, en utilisant comme co-substrat l'acide 3-acetylphénylboronique.
Rendement : 99%
RMN ¹H (DMSO) δ (ppm) : 2,45 (m, 4H), 2,50 (m, 2H), 2,70 (s, 3H), 3,40 (m, 2H), 3,60 (m, 4H), 7,675 (m, 1H), 7,85 (m, 4H), 8,00 (m, 2H), 8,15 (s, 1H), 8,30 (m, 2H), 8,55 (bs, 1H);
SM : MH⁺ : 435
HPLC : 98,1%

### Exemple 77: N-(2-Morpholin-4-yléthyl)-4-[4-(trifluorométhoxy)phényl]thiophène-2-carbothioamide

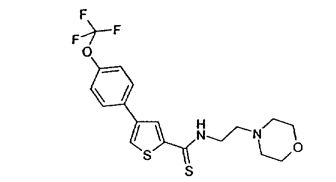

A une solution de 100 mg du produit de l'exemple 5 dans 4 ml de toluène anhydre sont additionnés 151 mg (1,5 équivalent) de réactif de Lawesson. Le milieu réactionnel est porté à 60°C pendant 24 heures. Après retour à température ambiante, le brut réactionnel est hydrolysé et extrait à l'acétate d'éthyle. Les phases organiques sont réunies, séchées sur sulfate de sodium et concentrées sous vide pour conduire à une huile jaune qui est purifiée sur silice éluée par un gradient dichlorométhane / méthanol (de 100/0 à 90/10 de 5 en 5%). La fraction la plus pure est reprise dans un système éther/pentane et conduit par précipitation à la formation d'aiguilles jaunes correspondant au produit attendu (9 mg).
Rendement : 9 %
RMN ¹H (DMSO) δ ppm : 2,45-2,64 (m, 6H), 3,58 (m, 4H), 3,86 (m, 2H), 7,47 (m, 2H), 7,80 (m, 2H), 8,15 (m, 2H), 10,17 (s, 1H)
SM : MH⁺ : 417
HPLC (214 nm) : 99,47%

### Exemple 78: N-(2-Morpholin-4-yléthyl)-4-[4-(1,2,3-thiadiazol-4-yl)phényl]thiophène-2-carboxamide

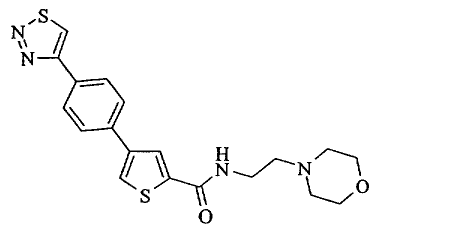

Le produit (21,7 mg) est obtenu selon le procédé de l'exemple 20, en utilisant comme co-substrat le 4-(4-bromophényl)-1,2,3-thiadiazole.
Rendement : 14,5%
RMN ¹H (DMSO) δ (ppm) : 2,425 (m, 4H), 2,50 (m, 2H), 3,40 (m, 2H), 3,60 (m, 4H), 7,90 (d, 2H), 8,25 (m, 3H), 8,30 (s, 1H), 8,55 (bs, 1H), 9,70 (s, 1H);
SM : MH⁺ : 401
HPLC : 99,3%

### Exemple 79: 4-(4-Isoxazol-5-ylphényl)-N-(2-morpholin-4-yléthyl)thiophène-2-carboxamide

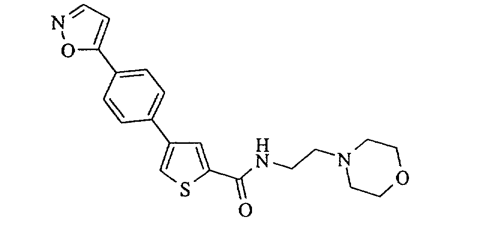

Le produit (32,1 mg) est obtenu selon le procédé de l'exemple 20, en utilisant comme co-substrat le 5-(4-bromophényl)-isoxazole.
Rendement : 22,5%
RMN ¹H (CDCl₃) δ (ppm) : 2,45 (m, 4H), 2,55 (m, 2H), 3,50 (m, 2H), 3,70 (m, 4H), 6,475 (s, 1H), 6,60 (bs, 1H), 7,55 (s, 1H), 7,625 (d, 2H), 7,725 (s, 1H), 7,80 (d, 2H), 8,25 (s, 1H);
SM : MH⁺ : 384
HPLC : 100,0%

### Exemple 80 : Acide trans-[4-([4-(4-Trifluorométhoxyphényl)thièn-2-yl]carboxamido) cyclohexyl] acétique

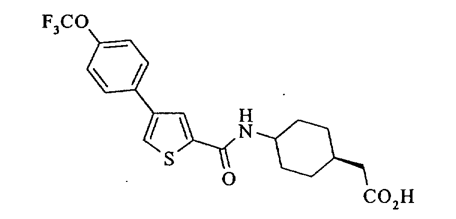

### Stade 1 : trans-[4-([4-(4-Trifluorométhoxyphényl)thièn-2 yl]carboxamido) cyclohexyl] acétate d'éthyle

A une solution de 229 mg du composé obtenu au stade 2 de la préparation 5 dans 4,0 ml de diméthylformamide anhydre sont ajoutés 0.15 g de trans(4-aminocyclohexyl)acétate d'éthyle préparé selon la méthode décrite dans *J*. *Med. Chem,* **1998**, 41, 760-771 (1,1 équivalents), 365 mg de O-[(éthoxycarbonyl)cyanométhyleneamino]-N,N,N',N'-tetraméthyluronium (TOTU) et 420 µl de N-éthyl-N,N-diisopropylamine. Le milieu réactionnel est agité pendant 17 heures à température ambiante puis concentré sous pression réduite. Le résidu obtenu est solubilisé dans l'acétate d'éthyle (50 ml), lavé avec de l'eau (2x20 ml), séché sur sulfate de sodium, filtré et concentré sous pression réduite. Une chromatographie sur gel de silice (cyclohexane/acétate d'éthyle : 70/30) du résidu permet d'isoler 62.5 mg du produit attendu.
Rendement : 17%
SM : MH⁺ : 456
HPLC : 100,0%

### Stade 2 : Acide trans-[4-(14-(4-Trifluorométhoxyphényl)thièn-2-yl]carboxamido) cyclohexyl] acétique

Le produit (62,0 mg) est obtenu selon le procédé de l'exemple 27, en utilisant comme substrat le produit obtenu au stade 1.
Rendement : 100%
RMN ¹H (DMSO) δ (ppm) : 12,0 (s, 1H), 8,35 (bs, 1H), 8,25 (m, 1H), 8,10 (s, 1H), 7,8 (m, 2H), 7,45 (m, 2H), 3,75 (m, 1H), 2,25 (m, 2H), 2,0 (m, 1H), 1,8 (m, 2H), 1,7 (m, 2H), 1,5(m, 1H), 1,40 (m, 2H), 1,10 (m, 2H) ;
SM : MH⁺ : 428; 472 (+HCOOH)
HPLC : 100,0%

### Exemple 81: Acide 2-[4-({[4-(4-phénoxyphényl)thièn-2-yl]carboxamido} méthyl)phényl]-acétique

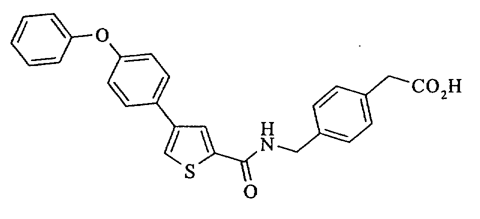

### Stade 1: 2-[4-(((4-Bromothièn-2-yl)carboxamidojméthyl)phényl]acétate d'éthyle

Le produit (135,0 mg) est obtenu selon le procédé de la préparation 17, en utilisant comme substrat le produit obtenu dans la préparation 11.
Rendement : 49%
SM : ES+383

### Stade 2: 2-[4-({4-(4-Phénoxyphényl)thièn-2-yl)carboxamido}méthyl)phényl]-acétate d'éthyle

Le produit (164,0 mg) est obtenu selon le procédé de l'exemple 1, en utilisant comme substrat le produit obtenu au stade 1 et co-substrat l'acide 4-phénoxyphénylboronique.
Rendement : 58%
HPLC : 100,0%

### Stade 3: Acide 2-[4-({4-(4-phénoxyphényl)thièn-2-yl)carboxamido}méthyl) phényl]-acétique

Le produit (122,0 mg) est obtenu selon le procédé de l'exemple 27, en utilisant comme substrat le produit obtenu au stade 2.
Rendement : 97%
RMN ¹H (DMSO) δ (ppm) : 9,1 (bs, 1H), 8,30 (s, 1H), 8,25 (m, 1H), 8,00 (s, 1H), 7,8 (m, 2H), 7,45 (m, 2H),7,3 (m, 4H), 7,15 (m, 1H), 7,0 (m, 4H), 4,5 (d, 2H), 3,5 (s, 2H);
SM : MH-: 528
HPLC : 100,0%

### Exemple 82: 4-(4-Trifuorométhoxyphényl)-N-méthylthiophène-2-carboxamide

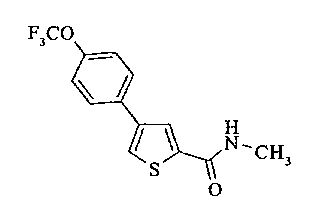

Le produit (112.9 mg) est obtenu selon le procédé du stade 1 de l'exemple 80, en utilisant comme substrat le produit obtenu au stade 2 de la préparation 5 et co-substrat la méthylamine.
Rendement : 43%
RMN ¹H (DMSO) δ (ppm) : 8,60 (bs, 1H), 8,15 (s, 1H), 8,10 (s, 1H), 7,8 (m, 2H), 7,45 (m, 2H), 2,80 (d, 3H);
SM : MH⁺ : 302; 346 (+HCOOH)
HPLC : 100,0%

### Exemple 83: Acide 2-[4-({[4-(4-benzyloxyphényl)thièn-2-yl]carboxamido} méthyl)phényl]-acétique

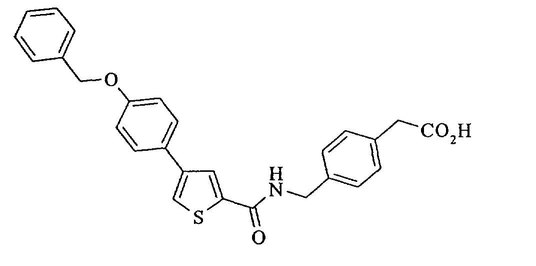

### Stade 1: 2-[4-({4-(4-Benzyloxyphényl)thièn-2-yl)carboxamido}méthyl)phényl]-acétate d'éthyle

Le produit (45,0 mg) est obtenu selon le procédé de l'exemple 1, en utilisant comme substrat le produit obtenu au stade 1 de l'exemple 81 et co-substrat l'acide 4-benzyloxyphénylboronique.
Rendement : 47%
HPLC : 100,0%

### Stade 2: Acide2-[4-({4-(4-benzyloxyphényl)thièn-2-yl)carboxamido}méthyl)phényl]- acétique

Le produit (40,0 mg) est obtenu selon le procédé de l'exemple 27, en utilisant comme substrat le produit obtenu au stade 2.
Rendement : 95%
RMN ¹H (DMSO) δ (ppm): 12,25 (m, 1H), 9,1 (bs, 1H), 8,25 (s, 1H), 8,0 (s, 1H), 7,6 (m, 2H), 7,5 (m, 2H), 7,45 (m, 2H), 7,3 (m, 1H), 7,2 (m, 2H), 7,15 (m, 2H), 7,0 (m, 2H), 5,2 (s, 2H), 4,4 (d, 2H), 3,5 (s, 2H);
HPLC : 96,6%

### Exemple 84 : N-(3,5-Difluoro-4-hydroxybenzyl)-4-(4-trifluorométhoxyphényl) thiophène-2-carboxamide

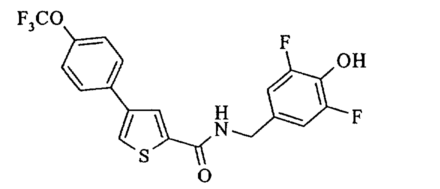

### Stade 1: 4-(Aminométhyl)-2,6-difluorophénol

Le produit (409,0 mg) est obtenu en appliquant le procédé décrit dans les stades 2 et 3 de la préparation 8, en utilisant comme substrat le 4-bromo-2,6-difluorophénol.
Rendement: 74%
SM : MH⁺ : 160
MH- : 158
HPLC : 100,0%

### Stade 2: N-(3,5 Difluoro-4-hydroxybenzyl)-4-(4-trifluorométhoxyphényl) thiophène-2-carboxamide

Le produit (49,0 mg) est obtenu selon le procédé du stade 1 de l'exemple 80, en utilisant comme substrat le produit obtenu au stade 1 et co-substrat le produit obtenu dans le stade 2 de la préparation 5.
Rendement : 42%
RMN ¹H (DMSO) δ (ppm): 10,25 (s, 1H), 9,1 (bs, 1H), 8,25 (s, 1H), 8,0 (s, 1H), 7,8 (m, 2H), 7,5 (m, 2H), 7,0 (m, 2H), 4,4 (d, 2H);
HPLC : 100%
SM : MH⁺ : 430
MH-: 428

### Exemple 85: 4-[4-(3-Nitrophényl)phényl]-N-(2-morpholin-4-yléthyl) thiophène-2-carboxamide

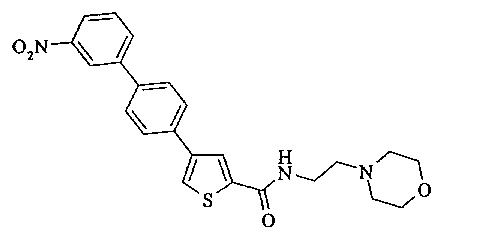

Le produit (66,2 mg) est obtenu selon le procédé de l'exemple 72, en utilisant comme co-substrat l'acide 3-nitrophénylboronique.
Rendement : 59,8%
RMN ¹H (DMSO) δ (ppm) : 2,45 (m, 4H), 2,50 (m, 2H), 3,40 (m, 2H), 3,60 (m, 4H), 7,80 (m, 1H), 7,85 (m, 2H), 7,925 (m, 2H), 8,225 (m, 3H), 8,30 (s, 1H), 8,525 (m, 2H);
SM : MH⁺ : 438
HPLC : 99,2%

### Exemple 86 : 4-[4-(2-Chlorophényl)phényl]-N-(2-morpholin-4-yléthyl) thiophène-2-carboxamide

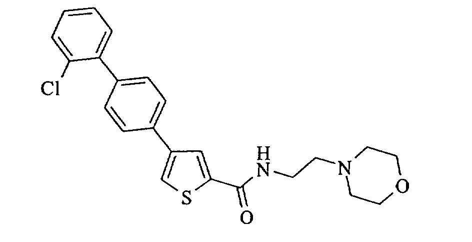

Le produit (36,3 mg) est obtenu selon le procédé de l'exemple 72, en utilisant comme co-substrat l'acide 2-chlorophénylboronique.
Rendement : 33,6%
RMN ¹H (DMSO) δ (ppm) : 2,45 (m, 4H), 2,50 (m, 2H), 3,40 (m, 2H), 3,60 (m, 4H), 7,45 (m, 3H), 7,525 (d, 2H), 7,60 (m, 1H), 7,80 (d, 2H), 8,15 (s, 1H), 8,275 (s, 1H), 8,50 (bs, 1H);
SM : MH⁺ : 427,5
HPLC : 97,7%

### Exemple 87: 4-[4-(3-Cyanophényl)phényl]-N-(2-morpholin-4-ylethyl) thiophène-2-carboxamide

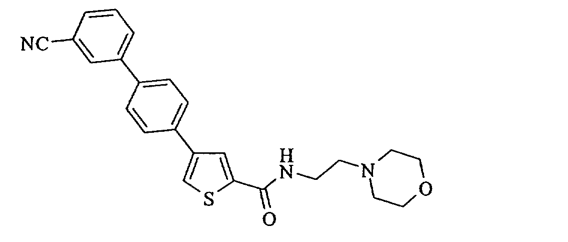

Le produit (66,4 mg) est obtenu selon le procédé de l'exemple 72, en utilisant comme co-substrat l'acide 3-cyanophénylboronique.
Rendement : 62,9%
RMN ¹H (DMSO) δ (ppm) : 2,45 (m, 4H), 2,50 (m, 2H), 3,40 (m, 2H), 3,60 (m, 4H), 7,675 (m, 1H), 7,85 (m, 5H), 8,10 (m, 1H), 8,20 (s, 1H), 8,25 (s, 1H), 8,30 (s, 1H), 8,50 (bs, 1H);
SM : MH⁺ : 418
HPLC : 98,3%

### Exemple 88 : 4-[4-(2-Formylphényl)phényl]-N-(2-morpholin-4-yléthyl) thiophène-2-carboxamide

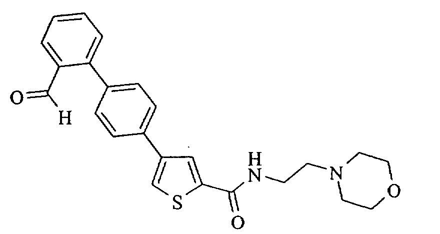

Le produit (61,9 mg) est obtenu selon le procédé de l'exemple 72, en utilisant comme co-substrat l'acide 2-formylphénylboronique.
Rendement : 58,2%
RMN ¹H (DMSO) δ (ppm) : 2,45 (m, 4H), 2,50 (m, 2H), 3,40 (m, 2H), 3,60 (m, 4H), 7,575 (m, 4H), 7,775 (m, 1H), 7,825 (d, 2H), 7,95 (m, 1H), 8,20 (s, 1H), 8,30 (s, 1H), 8,525 (bs, 1H), 9,95 (s, 1H);
SM : MH⁺ : 421
HPLC : 97,5%

### Exemple 89 : 4-[4-(3,4,5-Triméthoxyphényl)phényl]-N-(2-morpholin-4-yléthyl) thiophène-2-carboxamide

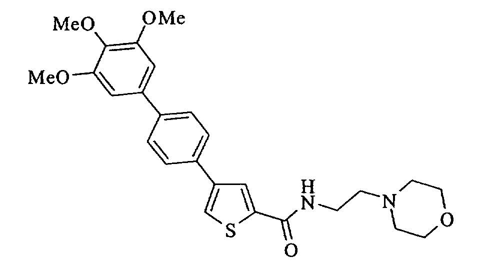

Le produit (77,4 mg) est obtenu selon le procédé de l'exemple 72, en utilisant comme co-substrat l'acide 3,4,5-triméthoxyphénylboronique.
Rendement : 63,4%
RMN ¹H (DMSO) δ (ppm) : 2,45 (m, 4H), 2,50 (m, 2H), 3,40 (m, 2H), 3,60 (m, 4H), 3,70 (s, 3H), 3,875 (s, 6H), 6,95 (s, 2H), 7,75 (s, 4H), 8,125 (s, 1H), 8,25 (s, 1H), 8,50 (bs, 1H);
SM : MH⁺ : 483
HPLC : 100,0%

### Exemple 90 : 4-[4-(4-Hydroxyméthylphényl)phényll"N-(2-morpholin-4-yléthyl) thiophène-2-carboxamide

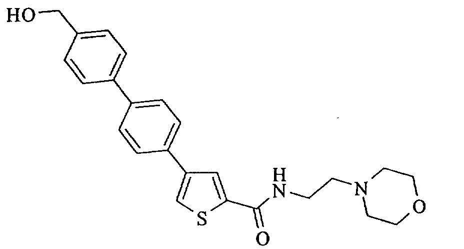

Le produit (82,9 mg) est obtenu selon le procédé de l'exemple 72, en utilisant comme co-substrat l'acide 4-(hydroxyméthyl)phénylboronique.
Rendement : 77,6%
RMN ¹H (DMSO) δ (ppm) : 2,45 (m, 4H), 2,50 (m, 2H), 3,40 (m, 2H), 3,60 (m, 4H), 4,55 (s, 2H), 5,225 (bs, 1H), 7,40 (d, 2H), 7,70 (d, 2H), 7,775 (s, 4H), 8,10 (s, 1H), 8,25 (s, 1H), 8,50 (bs, 1H);
SM : MH⁺ : 423
HPLC : 100,0%

### Exemple 91 : 4-[4-(3-Acétamidophényl)phényl]-N-(2-morpholin-4-yléthyl) thiophène-2-carboxamide

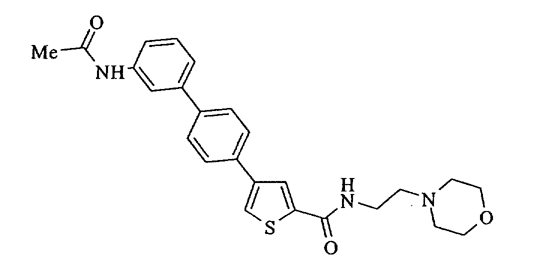

Le produit (100,3 mg) est obtenu selon le procédé de l'exemple 72, en utilisant comme co-substrat l'acide 3-acétamidophénylboronique.
Rendement : 88,2%
RMN ¹H (DMSO) δ (ppm) : 2,45 (m, 4H), 2,50 (m, 2H), 3,40 (m, 2H), 3,60 (m, 4H), 7,40 (m, 2H), 7,575 (m, 1H), 7,70 (d, 2H), 7,80 (d, 2H), 7,95 (s, 1H), 8,15 (s, 1H), 8,25 (s, 1H), 8,55 (bs, 1H), 10,05 (bs, 1H);
SM : MH⁺ : 451
HPLC : 100,0%

### Exemple 92 : 4-[4-(3,4-Méthylènedioxyphényl)phényl]-N-(2-morpholin-4-yléthyl) thiophène-2-carboxamide

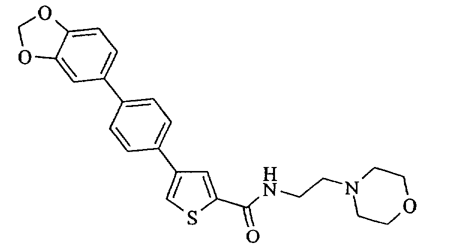

Le produit (121,4 mg) est obtenu selon le procédé de l'exemple 72, en utilisant comme co-substrat l'acide 3,4-(méthylènedioxy)phénylboronique.
Rendement : 99,2% RMN ¹H (DMSO) δ (ppm) : 2,45 (m, 4H), 2,50 (m, 2H), 3,40 (m, 2H), 3,60 (m, 4H), 6,175 (s, 2H), 7,00 (m, 1H), 7,225 (m, 1H), 7,325 (s, 1H), 7,75 (m, 4H), 8,10 (s, 1H), 8,25 (s, 1H), 8,525 (bs, 1H);
SM : MH⁺ : 437
HPLC : 96,9%

### Exemple 93 : N-(Cyclopropylméthyl)-4-(4-trifluorométhoxyphényl)thiophène-2-carboxamide

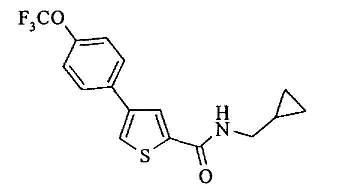

Le produit (146.0 mg) est obtenu selon le procédé de l'exemple 80 stade 1, en utilisant comme co-substrat le produit obtenu dans le stade 2 de la préparation 5 et la cyclopropylméthylamine.
Rendement : 41%
RMN ¹H (CDCl₃) δ (ppm) : 7,8 (s, 1H), 7,6 (m, 2H), 7,5 (s, 1H), 7,3 (m, 2H), 6,1 (bs, 1H), 3,3 (m, 2H), 1,2 (m, 1H), 0,8 (m, 2H), 0,3 (m,2H);
SM : MH⁺ : 342; 386 (+HCOOH)
HPLC : 100%

### Exemple 94 : N-(tert-Butyl)-4-(4-trifluorométhoxyphényl)thiophène-2-carboxamide

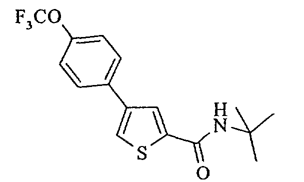

Le produit (171.0 mg) est obtenu selon le procédé de l'exemple 80 stade 1, en utilisant comme co-substrat le produit obtenu dans le stade 2 de la préparation 5 et la *tert*butylamine.
Rendement : 47.8% RMN ¹H (CDCl₃) δ (ppm) : 7,7 (s, 1H), 7,6 (m, 2H), 7,5 (s, 1H), 7,3 (m, 2H), 5,9 (bs, 1H), 1,5(s, 9H);
SM : MH⁺: 344; 388 (+HCOOH)
HPLC : 100%

### Exemple 95 : N-(Cyclopropyl)-4-(4-trifluorométhoxyphényl)thiophène-2-carboxamide

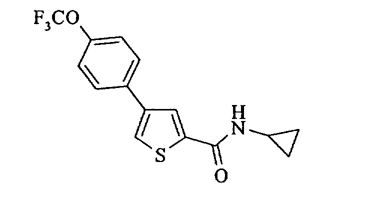

Le produit (168.0 mg) est obtenu selon le procédé de l'exemple 80 stade 1, en utilisant comme co-substrat le produit obtenu dans le stade 2 de la préparation 5 et la cyclopropylamine.
Rendement : 49.3%
RMN ¹H (CDCl₃) δ (ppm) : 7,8 (s, 1H), 7,6 (m, 3H), 7,3 (m, 1H), 6,1 (bs, 1H), 2,9 (m, 1H), 0,9 (m, 2H), 0,7 (m, 2H);
SM : MH⁺ : 328; 362 (+HCOOH)
HPLC : 100%

### Exemple 96 : N-(Cyclopentyl)-4-(4-trifluorométhoxyphényl)thiophène-2-carboxamide

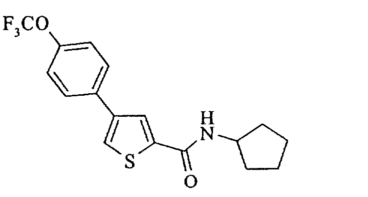

Le produit (281.0 mg) est obtenu selon le procédé de l'exemple 80 stade 1, en utilisant comme co-substrat le produit obtenu dans le stade 2 de la préparation 5 et la cyclopentylamine.
Rendement : 76% RMN ¹H (CDCl₃) δ (ppm) : 7,8 (s, 1H), 7,6 (m, 2H), 7,5 (s, 1H), 7,3 (m, 2H), 6,1 (bs, 1H), 4,5 (m, 1H), 2,2 (m, 2H), 1,8 (m, 4H), 1,7 (m,2H);
SM : MH⁺ : 356; 400 (+HCOOH)
HPLC : 100%

### Exemple 97 : N-(2-Hydroxyéthyl)-4-(4-trifluorométhoxyphényl)thiophène-2-carboxamide

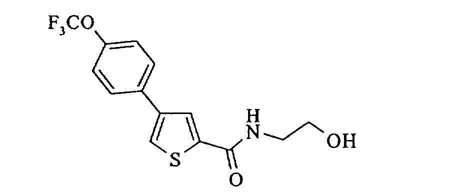

Le produit (171.0 mg) est obtenu selon le procédé de l'exemple 80 stade 1, en utilisant comme co-substrat le produit obtenu dans le stade 2 de la préparation 5 et l'éthanolamine.
Rendement : 49.5%
RMN ¹H (CDCl₃) δ (ppm) : 8,8 (bs, 1H), 8,2 (s, 1H), 8,1 (s, 1H), 7,8 (m, 2H), 7,5 (m, 2H), 4,8 (m, 1H). 3,4 (m, 2H), 3,3 (m, 2H);
SM : MH⁺ : 332; 376 (+HCOOH)
HPLC : 100%

### Exemple 98 : N-(Cyclopentylinéthyl)-4-(4-trîflnorométhoxyphényl)thiophène-2-carboxamide

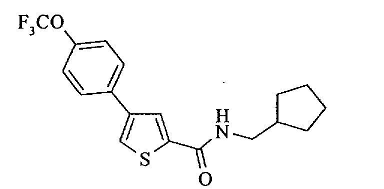

Le produit (95,6 mg) est obtenu selon le procédé de l'exemple 80 stade 1, en utilisant comme co-substrat le produit obtenu dans le stade 2 de la préparation 5 et la 1-cyclopentylméthanamine.
Rendement : 11%
RMN ¹H (CDCl₃) δ (ppm) : 7,8 (s, 1H), 7,6 (m, 2H), 7,5 (s, 1H), 7,2 (m, 2H), 6,1 (m, 1H), 3,4 (m, 2H), 2,2 (m, 1H), 1,8 (m, 2H), 1,7 (m, 2H), 1,6 (m, 2H), 1,3 (m, 2H);
SM : MH⁺ : 370; 414 (+HCOOH)
HPLC : 100%

### Exemple 99 : Etudes pharmacologiques des composés de l'invention:

### Evaluation in vitro de l'activité inhibitrice des composés de l'invention sur la MMP-12:

L'activité inhibitrice des composés de formule (I) sur la métalloprotéinase-12 est évaluée en testant la capacité des composés de l'invention à inhiber la protéolyse d'un peptide substrat de la MMP-12.

Le peptide substrat utilisé (peptide-1 fluorigénique : FP-1) dans le test présente la séquence suivante : Mca-Pro-Leu-Gly-Leu-Dap(Dnp)-Ala-Arg-NH₂

L'activité inhibitrice d'un composé de formule (I) est exprimée en valeur d'IC_{50,} qui représente la concentration en inhibiteur pour laquelle un taux d'inhibition de 50% de la métalloprotéinase est observé.

La réaction commence par l'addition séquentielle de 41 µl de substrat FP-1 (concentration finale de 10 µM) à une solution tampon de 50 mM de Tris-HCl et 10 mM de CaCl₂, et contenant 5 mM d'acide hydroxamique et 5 µl de l'enzyme diluée dans une solution tampon 0,005% Brij-35. Les microplaques sont incubées pendant 20 minutes à température ambiante. Les composés de l'invention sont testés à des concentrations variants de 0,3 à 30 µM.

La mesure de la quantité de protéolyse du substrat peptidique est suivie par une mesure d'absorbance à 405 nm en utilisant un spectrophotomètre de microplaques, à la température ambiante. Les valeurs d'IC₅₀ sont calculées à partir de courbes dans lesquelles le pourcentage de l'activité catalytique relative au control est représenté sur l'axe des X et la concentration en inhibiteur est représentée sur l'axe des Y.

Les valeurs d'IC₅₀ pour les composés des exemples 1 à 98 sont inférieures à 5 µM. Les composés de l'invention possèdent bien des propriétés inhibitrices de la métalloprotéinase-12 (MMP-12).

Le test décrit ci-dessus pour l'inhibition de la MMP-12 est ainsi adapté et utilisé pour déterminer la capacité des composés de formule (I) à inhiber les métalloprotéinases MMP-1, MMP-2, MMP-3, MMP-7, MMP-9, MMP-13 et MMP-14. Les résultats obtenus montrent que les composés de l'invention ont généralement des valeurs d'IC₅₀ pour la MMP-12 qui sont de 10 à plus de 100 fois plus faibles que les valeurs d'IC₅₀ obtenues pour le même composé avec les autres métalloprotéinases testées, prouvant ainsi leur capacité d'inhibition sélective vis-à-vis de la métalloprotéinase-12 (MMP-12).

## Revendications

**1.** Composés de formule (I): 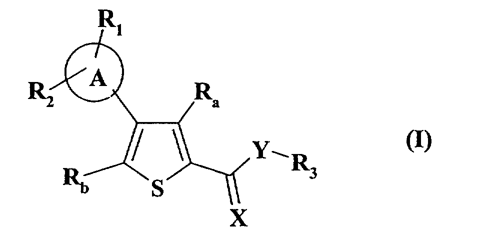 **caractérisés en ce que** :
• X représente un atome d'oxygène ou un atome de soufre,
• Y représente un atome d'oxygène, un groupement ―NH ou un groupement -N(C₁-C₆)alkyle,
• Rₐ représente un groupement choisi parmi hydrogène, halogène, (C₁-C₃)alkyle, hydroxy, et (C₁-C₃)alkoxy,
• R_{b} représente un groupement choisi parmi hydrogène, halogène, et (C₁-C₃)alkyle,
• A représente un groupement choisi parmi phényle, (C₅-C₆)cycloalkyle et (C₅-C₆)cycloalkènyle,
• R₁ et R₂, identiques ou différents, indépendamment l'un de l'autre, représentent chacun un groupement choisi parmi :
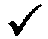 hydrogène, halogène, cyano, nitro, halogéno(C₁-C₆)alkyle, halogéno(C₁-C₆)alkoxy, (C₁-C₆)alkyle, (C₂-C₆)alkènyle, (C₂-C₆)alkynyle,
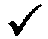 -OR₄, -NR₄R₅, -S(O)ₙR₄, -C(O)R₄, -CO₂R₄, -O-C(O)R₄, -C(O)NR₄R₅, -NR₅-C(O)R₄, - NR₅-SO₂R₄, -T-CN, -T-OR₄, -T-OCF₃, -T-NR₄R₅, -T-S(O)ₙR₄, -T-C(O)R₄, -T-CO₂R₄, -T-O-C(O)R₄, -T-C(O)NR₄R₅, -T-NR₄-C(O)R₅, -T-NR₄-SO₂R₅, -R₆, et -T-R₆ dans lesquels :
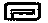 n représente un entier compris entre 0 et 2 inclus,
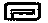 T représente une chaîne (C₁-C₆)alkylène linéaire ou ramifiée, éventuellement substituée par un groupement choisi parmi oxo, atome d'halogène, (C₁-C₆)alkoxy, hydroxy, amino, mono(C₁-C₆)alkylamino et di(C₁-C₆)alkylamino, et/ou dans laquelle éventuellement un des atomes de carbone est remplacé par un atome d'oxygène, un atome de soufre, un groupement -NH- , ou -N(C₁-C₆)alkyle-,
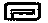 R₄ représente un atome d'hydrogène, un groupement (C₁-C₆)alkyle, aryle, cycloalkyle, ou un hétérocycle,
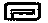 R₅ représente un atome d'hydrogène ou un groupement (C₁-C₆)alkyle,
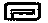 R₆ représente un groupement choisi parmi aryle, cycloalkyle, et un hétérocyle, chacun de ces groupements étant éventuellement substitués par un à cinq groupements, identiques ou différents, indépendamment l'un de l'autre choisis parmi halogène, cyano, nitro, halogéno(C₁-C₆)alkyle, halogéno(C₁-C₆)alkoxy, (C₁-C₆)alkyle, (C₁-C₆)alkènyle, -OR₄₀, -NR₄₀R₅₀, -S(O)ₙ₁R₄₀, -C(O)R₄₀, -CO₂R_{40,} -O-C(O)R₄₀, -C(O)NR₄₀R_{50,} -NR₅₀-C(O)R₄₀, -NR₅₀-SO₂R₄₀, -T₁-CN, -T₁-OR_{40,} -T₁-OCF_{3,} -T₁-NR₄₀R₅₀, -T₁-S(O)ₙR₄₀, -T₁-C(O)R₄₀, -T₁-CO₂R₄₀, -T₁-O-C(O)R_{40,} -T₁-C(O)NR₄₀R₅₀, -T₁-NR₅₀-C(O)R_{40,} -T₁-NR₅₀-SO₂R_{40,} -G₁ et -T₁-G₁ dans lesquels :
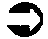 R_{40,} R_{50,} T₁ et n₁ ont respectivement les mêmes significations que R_{4,} R_{5,} T et n tels que définis précédemment,
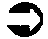 G₁ représente un groupement choisi parmi aryle, cycloalkyle, et un hétérocycle, chacun de ces groupements étant éventuellement substitués par 1 à 5 groupements, identiques ou différents, indépendamment l'un de l'autre choisis parmi halogène, cyano, nitro, halogéno (C₁-C₆)alkyle, halogéno(C₁-C₆)alkoxy, (C₁-C₆)alkyle, hydroxy, (C₁-C₆)alkoxy, phénoxy, benzyloxy, amino, mono(C₁-C₆)alkylamino, di(C₁-C₆)alkylamino, mercapto, (C₁-C₆)alkylthio, (C₁-C₇)acyle, (C₁-C₆)alkylsulfoxyde, carboxy, (C₁ -C₆)alkoxycarbonyl, phényl et un hétérocycle,
• R₃ représente un groupement -R₇ ou U-R_{11,} dans lesquels :
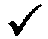 R₇ représente un groupement choisi parmi hydrogène, (C₁-C₆)alkyle, aryle, cycloalkyle et hétérocycle, chaque système cyclique étant éventuellement substitué par un à cinq groupements, identiques ou différents, indépendamment l'un de l'autre choisis parmi halogène, cyano, nitro, halogéno(C₁-C₆)alkyle, halogéno(C₁-C₆)alkoxy, (C₁-C₆)alkyle, -OR₈, -NR₈R₉, -S(O)ₘR₈, -C(O)R₈, -CO₂R₈, -O-C(O)R₈, -C(O)NR₈R₉, -NR₈-C(O)R₉, -NR₈-SO₂R₉, -V-CN, -V-OR₈, -V-NR₈R₉, -V-S(O)ₘR₈, -V-C(O)R₈, -V-CO₂R₈, -V-O-C(O)R₈, -V-C(O)NR₈R₉, -V-NR₈-C(O)R₉, -V-NR₈-SO₂R₉, -R₁₀, et -V-R₁₀ dans lesquels :
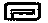 m représente un entier compris entre 0 et 2 inclus,
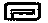 V représente une chaîne (C₁-C₆)alkylène linéaire ou ramifiée, une chaîne (C₂-C₆)alkènylène linéaire ou ramifiée, un groupement cyclopropylène ou une chaîne (C₂-C₆)alkylène linéaire ou ramifié dans laquelle un des atomes de carbone est remplacé par un atome d'oxygène, un atome de soufre, un groupement -NH-, ou -N(C₁-C₆)alkyl-,
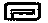 R₈ représente un atome d'hydrogène, un groupement (C₁-C₆)alkyle, aryle, cycloalkyle ou un hétérocycle,
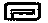 R₉ représente un atome d'hydrogène ou un groupement (C₁-C₆)alkyle,
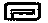 R₁₀ représente un groupement aryle, cycloalkyle, ou un hétérocyle,
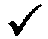 U représente une chaîne (C₁-C₆)alkylène linéaire ou ramifiée, ou (C₂-C₆)alkylène linéaire ou ramifiée dans laquelle un des atomes de carbone est remplacé par un atome d'oxygène, un atome de soufre, un groupement -NH-, ou -N(C₁-C₆)alkyle,
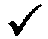 R₁₁ représente un groupement choisi parmi halogène, -OR₁₂, -NR₁₂R₁₃, -S(O)ₚR₁₂, -C(O)R₁₂, -CO₂R₁₂, -O-C(O)R₁₂, -C(O)NR₁₂R₁₃, -NR₁₃-C(O)R₁₂, -NR₁₃-SO₂R₁₂, et -R₁₄ ce dernier groupement étant éventuellement substitué par un ou deux groupements, identiques ou différents, indépendamment l'un de l'autre choisis parmi halogène, cyano, nitro, halogéno(C₁-C₆)alkyle, halogéno(C₁-C₆)alkoxy, (C₁-C₆)alkyle, (C₂-C₆)alkènyle, (C₂-C₆)alkynyle, -OR₁₅, -NR₁₅R_{16,} -S(O)_{q}R₁₅, -C(O)R₁₅, -O-C(O)R₁₅, -CO₂R₁₅, -C(O)Nk₁₅R_{16,} -NR₁₆-C(O)R_{15,} -NR₁₆-SO₂R₁₅, -R_{17,} -W-CN, - W-OR₁₅, -W-NR₁₅R₁₆, -W-S(O)_{q}R_{15,} -W-C(O)R_{15,} -W-CO₂R₁₅, -W-O-C(O)R₁₅, - -W-C(O)NR₁₅R_{16,} -W-NR₁₆-C(O)R_{15,} -W-NR₁₆-SO₂R₁₅, -W-R₁₇, et -C(O)-W₁-CO₂R_{15,} dans lesquels :
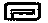 R₁₂ représente un atome d'hydrogène ou un groupement (C₁-C₆)alkyle, aryle, cycloalkyle, ou un hétérocycle
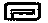 R₁₃ représente un atome d'hydrogène ou un groupement (C₁-C₆)alkyle,
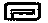 R₁₄ représente un groupement aryle, cycloalkyle ou un hétérocycle,
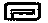 R₁₅ représente un atome d'hydrogène ou un groupement (C₁-C₆)alkyle, aryle, cycloalkyle, ou un hétérocycle,
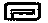 R₁₆ représente un atome d'hydrogène ou un groupement (C₁-C₆)alkyle,
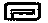 R₁₇ représente un groupement aryle, cycloalkyle ou un hétérocycle,
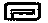 p représente un entier compris entre 0 et 2 inclus,
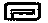 q représente un entier compris entre 0 et 2 inclus,
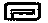 W représente une chaîne (C₁-C₆)alkylène linéaire ou ramifiée, une chaîne (C₂-C₆)alkènylène linéaire ou ramifiée, un groupement cyclopropylène, ou (C₂-C₆)alkylène linéaire ou ramifié dans laquelle un des atomes de carbone est remplacé par un atome d'oxygène, un atome de soufre, un groupement -NH-, ou -N(C₁ -C₆)alkyle-,
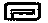 W₁ représente une chaîne (C₁-C₆)alkylène linéaire ou ramifiée,
leurs isomères, ainsi que leurs sels d'addition à un acide ou à une base pharmaceutiquement acceptable,
étant entendu que :
- par groupement aryle on comprend un système monocyclique ou bicyclique aromatique comprenant de 4 à 10 atomes de carbone, étant compris que dans le cas d'un système bicyclique l'un des cycles présente un caractère aromatique et l'autre cycle est aromatique
ou insaturé,
- par groupement cycloalkyle on comprend un système monocyclique ou bicyclique fusionné ou ponté, saturé ou partiellement insaturé, comprenant de 3 à 12 atomes de carbone,
- par un groupement hétérocycle on comprend un système monocyclique ou bicyclique fusionné ou ponté, de 3 à 12 chaînons, saturé, insaturé ou aromatique, comprenant de 1 à 4 hétéroatomes, identiques ou différents, choisis parmi oxygène, soufre et azote, et contenant éventuellement 1 ou 2 groupements oxo ou thioxo, étant compris que dans le cas d'un système bicyclique l'un des cycles présente un caractère aromatique et l'autre cycle est aromatique ou insaturé, ou les deux cycles sont saturés, ou l'un des cyles est saturé et l'autre cycle est insaturé, ou les deux cycles sont insaturés ;
- par groupement (C₁-C₆)alkyle on comprend une chaîne carbonée linéaire ou ramifiée comprenant de 1 à 6 atomes de carbone,
- par groupement halogéno(C₁-C₆)alkyle on comprend une chaîne carbonée linéaire ou ramifiée comprenant de 1 à 6 atomes de carbone et substituée par 1 à 6 atomes d'halogène,
- par groupement halogéno(C₁-C₆)alkoxy on comprend une chaîne carbonée linéaire ou ramifiée comprenant de 1 à 6 atomes de carbone et substituée par 1 à 6 atomes d'halogène, ladite chaîne étant reliée au composé de formule (I) par un atome d'oxygène,
- par atome d'halogène on comprend un atome choisi parmi brome, chlore, fluor et iode,
étant entendu également que les composés de formule (I) ne sont pas le :
5-méthyl-4-phényl-thiophèn-2-yl carboxylate d'éthyle, acide 5-méthyl-4-phényl-thiophèn-2-yl carboxylique, 3-hydroxy-4-phényl-thiophèn-2-yl-carboxylate de méthyle, 3-méthoxy-4-phényl-thiophèn-2-yl-carboxylate de méthyle, acide 3-méthoxy-4-phényl-thiophèn-2-yl-carboxylique, 4-(4-méthoxyphényl)-thiophèn-2-yl-carboxylate de méthyle, 4-phényl-thiophèn-2-yl-carboxylate de méthyle, acide 4-(4-méthoxyphényl)-thiophèn-2-yl-carboxylique, acide 4-phényl-thiophèn-2-yl-carboxylique, acide 4-(4-*tert*-butylphényl)-thiophèn-2-yl-carboxylique, 5-chloro-3-hydroxy-4-phényl-thiophèn-2-yl-carboxylate de méthyle, Acide 4-(3,5-diméthyl-phényl)-thiophèn-2-yl-carboxylique, 4-[(4-acétylamino)-phényl]-thiophèn-2-yl-carboxylate de méthyle, 4-phényl-thiophèn-2-yl-carboxylate d'éthyle, 4-phényl-thiophèn-2-yl-carboxamide, *N*-méthyl 4-phényl-thiophèn-2-yl-carboxamide, *N,N* diméthyl 4-phényl-thiophèn-2-yl-carboxamide, 5-méthyl-4-phényl-thiophèn-2-yl-carboxamide, *N*-méthyl 5-méthyl-4-phényl-thiophèn-2-yl-carboxamide, *N,N*-diméthyl 5-méthyl-4-phényl-thiophèn-2-yl-carboxamide, 2- {[4-(4-méthoxy-phényl)-thiophèn-2-yl]-carboxamido} benzoate de méthyle, et le *N*-[3-(trifluorométhyl)phényl] 4-(4-méthoxyphényl)-thiophèn-2-yl-carboxamide,
étant entendu aussi que:
- si Rₐ représente un atome d'hydrogène, A représente un groupement cyclopentèn-1-yl substitué en position 2 par un groupement R₁ prenant la définition thiényle éventuellement substitué alors R_{b} représente un groupement choisi parmi hydrogène, halogène, et (C₂-C₃)alkyle ;
- si R₃ représente un groupement R₇ prenant la définition hétérocycle alors ledit hétérocycle ne peut pas représenter un groupement 1-azabicyclo[2,2,2]oct-3-yle,
- et si R₃ représente un groupement R₇ prenant la définition phényle substitué en position *para* par un groupement R₁₀ alors ledit groupement R₁₀ ne peut pas représenter un groupement 5-méthyl-4,5-dihydro-3-oxo-2*H*-pyridazin-6-yle.

**2.** Composés de formule (I) selon la revendication 1 **caractérisés en ce qu'**ils représentent des composés de formule (IA) : 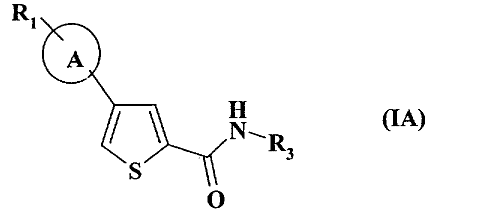 dans laquelle A, R₁, et R₃ sont tels que définis dans la formule (I),
leurs isomères, ainsi que leurs sels d'addition à un acide ou à une base pharmaceutiquement acceptable.

**3.** Composés de formule (I) selon l'une quelconque des revendications 1 ou 2 **caractérisés en ce que** A représente un groupement phényle, leurs isomères, ainsi que leurs sels d'addition à un acide ou à une base pharmaceutiquement acceptable.

**4.** Composés de formule (I) selon la revendication 3 **caractérisés en ce que** ledit groupement A prenant la définition phényle est substitué par un groupement R₁ tel que défini dans la formule (I) situé en position *para,* leurs isomères, ainsi que leurs sels d'addition à un acide ou à une base pharmaceutiquement acceptable.

**5.** Composés de formule (I) selon la revendication 3 **caractérisés en ce que** ledit groupement A prenant la définition phényle est substitué par un groupement R₁ tel que défini dans la formule (I) situé en position *ortho* ou en position *méta,* leurs isomères, ainsi que leurs sels d'addition à un acide ou à une base pharmaceutiquement acceptable.

**6.** Composés de formule (I) selon l'une quelconque des revendications 1 ou 2 **caractérisés en ce que** R₁ représente un groupement choisi parmi trifluorométhyle, trifluorométhoxy, (C₁-C₆)alkyle, -OR₄, -SR₄, -NR₄R₅, -CO₂R₄, -O-C(O)R₄, -T-CO₂R₄ et -R₆ dans lesquels :
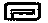 R₄ représente un atome d'hydrogène, un groupement (C₁-C₃)alkyle, aryle, cycloalkyle, ou un hétérocycle,
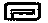 R₅ représente un atome d'hydrogène ou un groupement (C₁-C₆)alkyle,
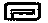 R₆ représente un groupement choisi parmi aryle, cycloalkyle, et un hétérocyle, chacun de ces groupements étant éventuellement substitués par un groupement choisi parmi halogène, cyano, nitro, halogéno(C₁-C₆)alkyle, halogéno(C₁-C₆)alkoxy, (C₁-C₆)alkyle, -OR₄₀, -NR₄₀R₅₀, -S(O)ₙ₁R₄₀, -C(O)R₄₀, -CO₂R₄₀, -O-C(O)R₄₀, -C(O)NR₄₀R₅₀, -NR₅₀-C(O)R₄₀, et -NR₅₀-SO₂R₄₀ dans lesquels R₄₀, R₅₀, et n1 sont tels que définis dans la formule (I),
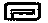 T est tel que défini dans la formule (I),
leurs isomères, ainsi que leurs sels d'addition à un acide ou à une base pharmaceutiquement acceptable.

**7.** Composés de formule (I) selon la revendication 4 **caractérisés en ce que** R₁ représente un groupement tel que défini dans la revendication 6, leurs isomères, ainsi que leurs sels d'addition à un acide ou à une base pharmaceutiquement acceptable.

**8.** Composés de formule (I) selon la revendication 5 **caractérisés en ce que** R₁ représente un groupement choisi parmi trifluorométhyle, trifluorométhoxy, (C₁-C₆)alkyle, -OR₄, et -NR₄R₅ dans lesquels :
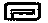 R₄ représente un atome d'hydrogène ou un groupement (C₁-C₃)alkyle,
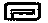 R₅ représente un atome d'hydrogène ou un groupement (C₁-C₃)alkyle,
leurs isomères, ainsi que leurs sels d'addition à un acide ou à une base pharmaceutiquement acceptable.

**9.** Composés de formule (I) selon la revendication 6 **caractérisés en ce que** R₁ représente un groupement choisi parmi (C₂-C₄)alkyle, (C₁-C₄)alkylthio, (C₁-C₄)alkoxy, trifluorométhoxy, et -R₆ dans lequel R₆ représente un groupement choisi parmi phényle, (C₅-C₆)cycloalkyle saturé ou partiellement insaturé, et un hétérocycle à 5 ou 6 chaînons comprenant de 1 à 3 hétéroatomes choisis parmi oxygène, azote et soufre, chacun desdits groupement R₆ étant éventuellement substitué par un groupement tel que défini dans la formule (I), leurs isomères, ainsi que leurs sels d'addition à un acide ou à une base pharmaceutiquement acceptable.

**10.** Composés de formule (I) selon la revendication 4 **caractérisés en ce que** R₁ représente un groupement tel que défini dans la revendication 9, leurs isomères, ainsi que leurs sels d'addition à un acide ou à une base pharmaceutiquement acceptable.

**11.** Composés de formule (I) selon la revendication 9 **caractérisés en ce que** R₁ représente un groupement choisi parmi :
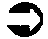 phényle éventuellement substitué par un groupement choisi parmi atome d'halogène, hydroxy, (C₁-C₄)alkoxy linéaire ou ramifié, (C₁-C₄)acyle, (C₁-C₄)alkylsulfone, -CO₂R₄₀ et ―C(O)NR₄₀R₅₀ dans lesquels R₄₀ et R₅₀ sont tels que définis dans la formule (I),
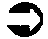 cyclohexyle,
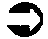 4-pyridyle, 3-pyridyle, N-pyrrolydinyle, 1-méthylpyrrol-3-yl, et 3,6-dihydro-2H-pyridin-1-yl,
leurs isomères, ainsi que leurs sels d'addition à un acide ou à une base pharmaceutiquement acceptable.

**12.** Composés de formule (I) selon la revendication 9 **caractérisés en ce que** R₁ représente un groupement cyclohexyle éventuellement substitué par un groupement choisi parmi hydroxy, (C₁-C₄)alkoxy linéaire ou ramifié, (C₁ -C₄)acyle, , (C₁-C₄)alkylsulfone, (C₁-C₄)alkyle, amino, (C₁-C₄)alkylamino et di(C₁-C₄)alkylamino, leurs isomères, ainsi que leurs sels d'addition à un acide ou à une base pharmaceutiquement acceptable.

**13.** Composés de formule (I) selon l'une quelconque des revendications 1 ou 2 **caractérisés en ce que** R₃ représente un groupement R₇ choisi parmi phényle, cyclohexyle, et pyridyle, chacun de ces groupements étant éventuellement substitué par un ou deux groupements, identiques ou différents, indépendamment l'un de l'autre choisis parmi (C₁-C₆)alkyle, -OR₈, -NR₈R₉, -CO₂R₈, -V-OR₈, -V-NR₈R₉ et -V-CO₂R₈ dans lesquels V représente une chaîne (C₁-C₄)alkylène linéaire ou ramifiée, ou une chaîne (C₂-C₄)alkènylène linéaire ou ramifiée, R₈ représente un atome d'hydrogène ou un groupement (C₁-C₆)alkyle leurs isomères, et R₉ représente un atome d'hydrogène, ainsi que leurs sels d'addition à un acide ou à une base pharmaceutiquement acceptable.

**14.** Composés de formule (I) selon l'une quelconque des revendications 1 ou 2 **caractérisés en ce que** R₃ représente un groupement -U-R₁₁ dans lequel U représente une chaîne (C₁-C₄)alkylène linéaire et R₁₁ représente un groupement choisi parmi -CO₂R₁₂ et -R₁₄ dans lesquels :
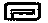 R₁₂ représente un atome d'hydrogène ou un groupement (C₁-C₆)alkyle,
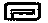 R₁₄ représente un groupement choisi parmi phényle, cyclohexyle, morpholinyle, et pyridyle chacun de ces groupements étant éventuellement substitué par un ou deux groupements, identiques ou différents, indépendamment l'un de l'autre choisis parmi halogène, (C₁-C₆)alkyle, -CO₂R_{15,} et -W-CO₂R₁₅, dans lesquels R₁₅ représente un atome d'hydrogène ou un groupement (C₁-C₆)alkyle, et W représente une chaîne (C₁-C₆)alkylène linéaire ou ramifiée, ou une chaîne (C₂-C₆)alkènylène linéaire ou ramifiée,
leurs isomères, ainsi que leurs sels d'addition à un acide ou à une base pharmaceutiquement acceptable.

**15.** Composés de formule (I) selon la revendication 1 qui sont le :
4-(4-Isopropylphényl)-N-(2-morpholin-4-yléthyl)thiophène-2-carboxamide
4-(4-Biphényl)-N-(2-morpholin-4-yléthyl)thiophène-2-carboxamide
4-(4-Ethylphényl)-N-(2-morpholin-4-yléthyl)thiophène-2-carboxamide
4-[4-(Méthylthio)phényl]-N-(2-morpholin-4-yléthyl)thiophène-2-carboxamide
4-[4-(Trifluorométhyl)phényl]-N-(2-morpholin-4-yléthyl)thiophène-2-carboxamide
4-[4-(tert-Butyl)phényl]-N-(2-morpholin-4-yléthyl)thiophène-2-carboxamide
4-[4-(Trifluorométhyl)phényl]-N-(2-morpholin-4-yléthyl)thiophène-2-carboxamide
4-(4-Hydroxyphényl)-N-(2-morpholin-4-yléthyl)thiophène-2-carboxamide
4-[4-(Pyridin-4-yl)phényl]-N-(2-morpholin-4-yléthyl)thiophène-2-carboxamide
4'-[5-(2-Morpholin-4-yl-éthylcarbamoyl)-thiophèn-3-yl]biphényl-3-carboxylate de méthyle
4-[4-(Pyridin-3-yl)phényl]-N-(2-morpholin-4-yléthyl)thiophène-2-carboxamide
4-[4-(Morpholin-4-yl)phényl]-N-(2-morpholin-4-yléthyl)thiophène-2-carboxamide
4-(4-Pipéridophényl)-N-(2-morpholin-4-yléthyl)thiophène-2-carboxamide
4-[4-(Pyrrolidin-1-yl)phényl]-N-(2-morpholin-4-yléthyl)thiophène-2-carboxamide
4-[4-(1,4-Dioxa-8-azaspiro[4,5]dec-8-yl)phényl]-N-(2-morpholin-4-yléthyl)thiophène-2-carboxamide
4-[4-(1,2,3,6-Tétrahydropyridin-1-yl)phényl]-N-(2-morpholin-4-yléthyl)thiophène-2-carboxamide
4-[4-(3-(3R)-Hydroxy-pyrrolidin-1-yl)phényl]-N-(2-morpholin-4-yléthyl)thiophène-2-carboxamide
4-[4-(1H-Imidazol-1-yl)phényl]-N-(2-morpholin-4-yléthyl)thiophène-2-carboxamide
N-(2-Morpholin-4-yléthyl)-4-[4-(1H-pyrrol-1-yl)phényl]-thiophène-2-carboxamide
4-[4-(Isoxazol-5-yl)phényl]-N-(2-morpholin-4-yléthyl)thiophène-2-carboxamide
4-[4-(Cyclohexyl)phényl]-N-(2-morpholin-4-yléthyl)thiophène-2-carboxamide
4-[4-(1-Méthyl-1H-pyrazol-3-yl)phényl]-N-(2-morpholin-4-yléthyl)thiophène-2-carboxamide
4-[4-(6-Oxo-1,4,5,6-tétrahydro-pyridazin-3-yl)phényl]-N-(2-morpholin-4-yléthyl)thiophène-2-carboxamide
trans 4-{[4-(4-Phényl-cyclohex-1-ényl)-thiophèn-2-carboxamide]méthyl}-cyclohexane carboxylate de méthyle
trans Acide 4- {[4-(4-phényl-cyclohex-1-ényl)-thiophèn-2-carboxamide]méthyl}-cyclohexane carboxylique
3-(6- {[4-(4-Trifluorométhoxyphényl)thièn-2-yl]carboxamido}pyridin-3-yl)-propanoate d'éthyle
Acide 3-(6-{[4-(4-trifluorométhoxyphényl)thièn-2-yl]carboxamido}pyridin-3-yl)-propanoïque
3-(4-{[4-(4-Trifluorométhoxyphényl)thièn-2-yl]carboxamido}phényl)-propènoate d'éthyle
Acide 3-(4-{[4-(4-trifluorométhoxyphényl)thièn-2-yl]carboxamido}phényl)-propènoïque
4-({[4-(Trifluorométhoxyphényl)thièn-2-yl]carboxamido}méthyl) phtalate de diéthyle
Diacide 4-( ([4-(trifluorométhoxyphényl)thièn-2-yl]carboxamido} méthyl)-phtalique
3-(4-{[4-(4-Trifluorométhoxyphényl)thièn-2-yl]carboxamido}phényl)-propanoate d'éthyle
Acide 3-(4-{[4-(4-trifluorométhoxyphényl)thièn-2-yl]carboxamido}phényl)-propanoïque
trans 4-({[4-(4-Trifluorométhoxyphényl)-thièn-2-yl]carboxamido}méthyl)-cyclohexane carboxylate de méthyle
trans Acide 4-({[4-(4-trifluorométhoxyphényl)-thièn-2-yl] carboxamide}méthyl)-cyclohexane carboxylique
2-[4-( {[4-(4-Trifluorométhoxyphényl)thièn-2-yl]carboxamido} méthyl)phényl]-acétate d'éthyle
Acide 2-[4-({[4-(4-trifluorométhoxyphényl)thièn-2-yl]carboxamido}méthyl)phényl]-acétique
3-({[4-(4-Trifluorométhoxyphényl)thièn-2-yl]carboxamido}méthyl)benzoate d'éthyle
Acide 3 -( {[4-(4-trifluorométhoxyphényl)thièn-2-yl]carboxamido}méthyl)benzoïque
2-[3-( {[4-(4-Trifluorométhoxyphényl)thièn-2-yl]carboxamido} méthyl)phényl] -acétate d'éthyle
Acide 2-[3-( {[4-(4-trifluorométhoxyphényl)thièn-2-yl]carboxamido} méthyl)phényl]-acétique
4- {[4-(4-Trifluorométhoxyphényl)-thiophèn-2-carboxamide]méthyl} -cyclohexane carboxylate d'éthyle
Acide 4- {[4-(4-trifluorométhoxyphényl)-thiophèn-2-carboxamide]méthyl} -cyclohexane carboxylique
6-( {[4-(4-Trifluorométhoxyphényl)thièn-2-yl]carboxamido) méthyl)-nicotinate de méthyle
Acide 6-( {[4-(4-trifluorométhoxyphényl)thièn-2-yl]carboxamido} méthyl)-nicotinique
4-({[4-(4-Trifluorométhoxyphényl)thièn-2-yl]carboxamido}méthyl)benzoate d'éthyle
Acide 4-({[4-(4-trifluorométhoxyphényl)thièn-2-yl]carboxamido}méthyl)benzoïque
4-({[4-(4-Méthylthiophényl)thièn-2-yl]carboxamido}méthyl)benzoate d'éthyle
Acide 4-( {[4-(4-méthylthiophényl)thièn-2-yl]carboxamido} méthyl)benzoïque
4-({[4-(4-tert-Butylphényl)thièn-2-yl]carboxamido}méthyl)benzoate d'éthyle
Acide 4-({[4-(4-tert-butylphényl)thièn-2-yl]carboxamido}méthyl)benzoïque
(4-{[4-(4-tert-Butylphényl)thièn-2-yI]carboxamido}phényl)acétate d'éthyle
Acide (4-{[4-(4-tert-butylphényl)thièn-2-yl]carboxamido}phényl)acétique
(4-{[4-(4-Trifluorométhoxyphényl)thièn-2-yl]carboxamido}phényl)acétate d'éthyle
Acide (4-{[4-(4-trifluorométhoxyphényl)thièn-2-yl]carboxamido}phényl)acétique
(4-{[4-(4-Méthylthiophényl)thièn-2-yl]carboxamido}phényl)acétate d'éthyle
Acide (4- {[4-(4-méthylthiophényl)thièn-2-yl]carboxamido}phényl)acétique
4-({(4-(4-Méthoxyphényl)thièn-2-yl]carboxamido}méthyl)benzoate d'éthyle
Acide 4-({[4-(4-Méthoxyphényl)thièn-2-yl]carboxamido}méthyl) benzoïque
(4-{[4-(4-Méthoxyphényl)thièn-2-yl]carboxamido}phényl)acétate d'éthyle
Acide (4-{[4-(4-méthoxyphényl)thièn-2-yl]carboxamido}phényl)acétique
3-[4-({[4-(4-Trifluorométhoxyphényl)thièn-2-yl]carboxamido} méthyl)phényl]propanoate d'éthyle
Acide 3-[4-( {[4-(4-trifluorométhoxyphényl)thièn-2-yl]carboxamido} méthyl)phényl]propanoïque
3-[3-({[4-(4-Trifluorométhoxyphényl)thièn-2-yl]carboxamido} méthyl)phényl]propanoate d'éthyle
Acide 3-[3-( ([4-(4-trifluorométhoxyphényl)thièn-2-yl]carboxamido}méthyl)phényl]propanoïque
7- {[4-(4-Trifluorométhoxyphényl)thièn-2-yl]carboxamido} heptanoate d'éthyle
Acide 7- ([4-(4-trifluorométhoxyphényl)thièn-2-yl]carboxamido}heptanoïque
4-{[4-(4-Hydroxyphényl)thièn-2-yl]carboxamido}méthyl)cyclohexane carboxylate d'éthyle
4-[( {4- [4-(Pyridin-4-yl)phényl]thièn-2-yl} carboxamido)méthyl]cyclohexane carboxylate d'éthyle
Chlorhydrate d'acide 4-(({4-(4-(pyridin-4-yl)phényl]thièn-2-yl}carboxamido)méthyl] cyclohexane carboxylique
4-[4-(4-Acétylphényl)phényl]-N-(2-morpholin-4-yléthyl)thiophène-2-carboxamide
4-[4-(4-Fluorophényl)phényl]-N-(2-morpholin-4-yléthyl)thiophène-2-carboxamide
4-[4-(3-Hydroxyphényl)phényl]-N-(2-morpholin-4-yléthyl)thiophène-2-carboxamide
4-[4-(4-Méthylsulfonylphényl)phényl)phényl]-N-(2-morpholin-4-yléthyl)thiophène-2-carboxamide
4-[4-(3-Acétylphényl)phényl]-N-(2-morpholin-4-yléthyl)thiophène-2-carboxamide
N-(2-morpholin-4-yléthyl)-4-[4-(trifluorométhoxy)phényl]thiophène-2-carbothioamide,
N-(2-Morpholin-4-yléthyl)-4-[4-(1,2,3 -thiadiazol-4-yl)phényl]thiophène-2-carboxamide,
4-(4-Isoxazol-5-ylphényl)-N-(2-morpholin-4-yléthyl)thiophène-2-carboxamide,
Acide *trans*-[4-([4-(4-Trifluorométhoxyphényl)thièn-2-yl]carboxamido)cyclohexyl] acétique,
Acide 2-[4-({[4-(4-phénoxyphényl)thièn-2-yl]carboxamido)méthyl)phényl]-acétique,
4-(4-Trifuorométhoxyphényl)-N-méthylthiophène-2-carboxamide,
Acide 2-[4-({[4-(4- benzyloxyphényl)thièn-2-yl]carboxamido} méthyl)phényl]-acétique,
N-(3,5-Difluoro-4-hydroxybenzyl)-4-(4-trifluorométhoxyphényl)thiophène-2-carboxamide,
4-[4-(3-Nitrophényl)phényl]-N-(2-morpholm-4-yléthyl)thiophène-2-carboxamide,
4-[4-(2-Chlorophényl)phényl]-N-(2-morpholin-4-yléthyl)thiophène-2-carboxamide,
4-[4-(3-Cyanophényl)phényl] -N-(2-morpholin-4-yléthyl)thiophène-2-carboxamide,
4-[4-(2-Formylphényl)phényl]-N-(2-morpholin-4-yléthyl)thiophène-2-carboxamide,
4-[4-(3,4,5-Triméthoxyphényl)phényl]-N-(2-morpholin-4-yléthyl)thiophène-2-carboxamide,
4-[4-(4-Hydroxyméthylphényl)phényl]-N-(2-morpholin-4-yléthyl)thiophène-2-carboxamide,
4-[4-(3-Acétamidophényl)phényl]-N-(2-morpholin-4-yléthyl)thiophène-2-carboxamide,
4-[4-(3,4-Méthylènedioxyphényl)phényl]-N-(2-morpholin-4-yléthyl)thiophène-2-carboxamide,
N-(Cyclopropylméthyl)-4-(4-trifluorométhoxyphényl)thiophène-2-carboxamide,
N-(*tert*-Butyl)-4-(4-trifluorométhoxyphényl)thiophène-2-carboxamide,
N-(Cyclopropyl)-4-(4-trifluorométhoxyphényl)thiophène-2-carboxamide,
N-(Cyclopentyl)-4-(4-trifluorométhoxyphényl)thiophène-2-carboxamide,
N-(2-Hydroxyéthyl)-4-(4-trifluorométhoxyphényl)thiophène-2-carboxamide,
et le N-(Cyclopentylméthyl)-4-(4-trifluorométhoxyphényl)thiophène-2-carboxamide.

**16.** Procédé de préparation des composés de formule (I) **caractérisé en ce qu'**on utilise comme produit de départ un composé de formule (II) : 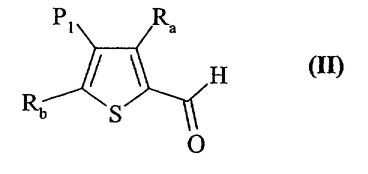 dans laquelle Rₐ et R_{b} sont tels que définis dans la formule (I) et P₁ représente un atome d'halogène ou un groupement triflate,
composés de formule (II) qui sont soumis à des conditions d'oxydation en présence, par exemple, de nitrate d'argent en milieu basique et polaire, pour donner les composés de formule (III) : 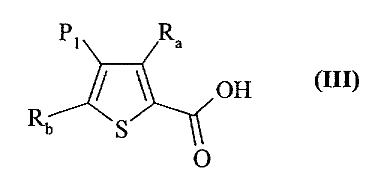 dans laquelle R_{a,} R_{b} et P₁ sont tels que définis précédemment,
composés de formule (III) qui sont éventuellement transformés en chlorures d'acide correspondant (IV) par action du chlorure d'oxalyle par exemple, 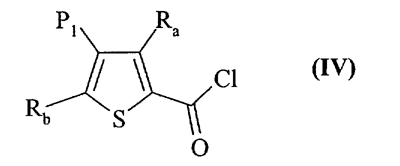 dans laquelle R_{a,} R_{b} et P₁ sont tels que définis précédemment,
ou qui sont traités directement, dans des conditions de couplage peptidique en présence d'un agent de couplage et dans un milieu basique et polaire, avec un composé de formule (V):
HY-R₃ (V)
dans laquelle Y et R₃ ont les mêmes significations que dans les composés de formule (I), pour conduire aux composés de (VI) : 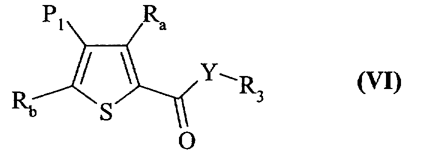 dans laquelle R_{a,} R_{b,} R_{3,} Y et P₁ sont tels que définis précédemment,
composés de formule (VI) qui sont :
• soit mis à réagir, dans des conditions basiques de couplage au palladium, avec un composé de formule (VII) : 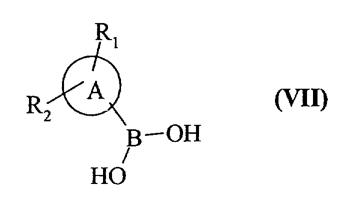
dans laquelle A, R₁ et R₂ ont les mêmes significations que dans les composés de formule (I),
pour conduire aux composés de formule (I/a), cas particulier des composés de formule (I): 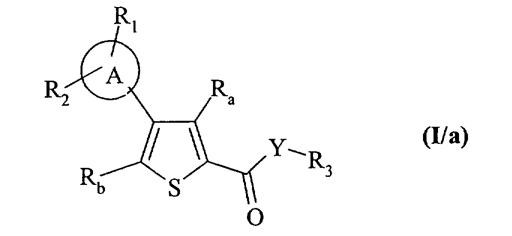 dans laquelle Rₐ, R_{b}, R₁, R₂, R₃, Y et A sont tels que définis précédemment,
• soit traités avec de l'hexaméthyldiétain, en présence d'un catalyseur au palladium, pour conduire aux composés de formule (VIII) : 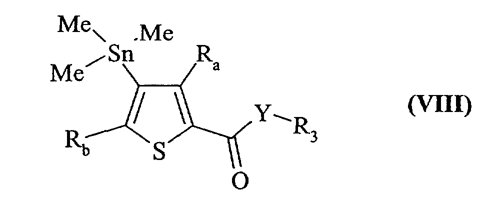
dans laquelle Rₐ, R_{b}, R₃ et Y sont tels que définis précédemment,
composés de formule (VIII) qui sont mis à réagir :
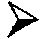 avec un composé de formule (IX) : 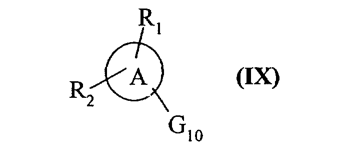
dans laquelle R_{1,} R₂ et A ont les mêmes significations que dans la formule (I) et G₁₀ représentent un atome d'halogène choisi parmi chlore et brome ou un groupement triflate,
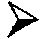 soit en présence de triphénylphosphine arsenic et d'un catalyseur au palladium, dans le cas où G₁₀ représente un groupement triflate,
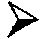 soit en présence d'un composé halogénocuivrique tel que CuBr₂ et d'un catalyseur au palladium, dans des conditions de solvant polaire, dans le cas où G₁₀ représente un atome d'halogène,
pour conduire également aux composés de formule (I/a) tels que décrits précédemment,
• soit traités par du bis(pinacolato)di-borane suivi par une réaction d'oxydation pour conduire aux composés de formule (VIa): 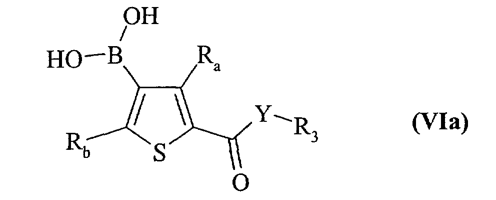
dans laquelle R_{a,} R_{b,} R₃ et Y sont tels que définis précédemment,
composés de formule (VIa) qui sont mis à réagir dans des conditions basiques de couplage au palladium avec un composé de formule (IX) : 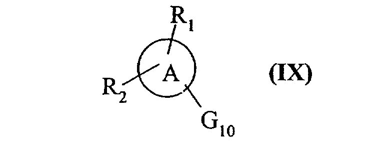 dans laquelle A, R_{1,} R₂ et G₁₀ sont tels que définis précédemment,
pour conduire également aux composés de formule (I/a), tel que défini précédemment : 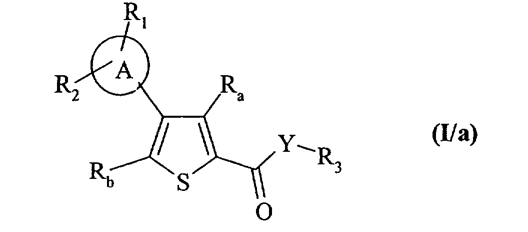 dans laquelle Rₐ, R_{b}, R1, R₂, R₃, Y et A sont tels que définis précédemment,
• soit mis à réagir avec un composé de formule (IXa): 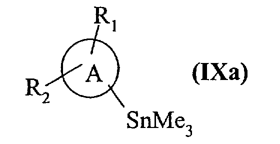
dans laquelle A, R₁, et R₂ sont tels que définis dans la formule (I),
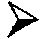 soit en présence de triphénylphosphine arsenic et d'un catalyseur au palladium, dans le cas où P₁ dans les composés de formule (VI) représente un groupement triflate,
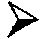 soit en présence d'un composé halogénocuivrique tel que CuBr₂ et d'un catalyseur au palladium, dans des conditions de solvant polaire, dans le cas où P₁ dans les composés de formule (VI) représente un atome d'halogène,
pour conduire également aux composés de formule (I/a) tels que décrits précédemment, 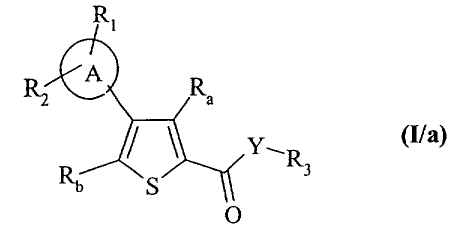 dans laquelle Rₐ, R_{b}, R₁, R₂, R₃, Y et A sont tels que définis précédemment,
composés de formule (I/a) dans le cas particulier où ils représentent des composés de formule (I/b) dans lesquels A représente un groupement phényle, R₂ représente un atome d'hydrogène, R₁ représente un groupement hydroxy ou un atome d'halogène (Hal) et Rₐ, R_{b}, Y et R₃ ont la même signification que dans la formule (I): 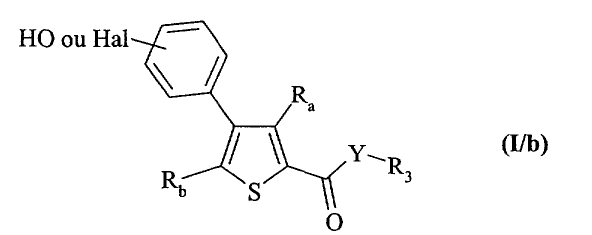 composés de formule (I/b) qui peuvent alors être traités préalablement par de l'anhydride trifluorométhanesulfonique en présence d'une base forte, dans le cas où R₁ représente un groupement hydroxy, pour obtenir le dérivé activé phényltriflate,
lesdits composés portant un groupement R₁ prenant la définition halogène ou triflate pouvant alors
o soit être mis à réagir en condition basique et en présence d'un catalyseur au palladium avec un composé de formule (X) : 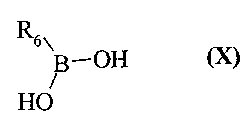
dans laquelle R₆ est tel que défini dans la formule (I), c'est à dire qu'il représente un groupement choisi parmi aryle, cycloalkyle, et un hétérocycle,
pour conduire aux composés de formule (I/c), cas particulier des composés de formule (I): 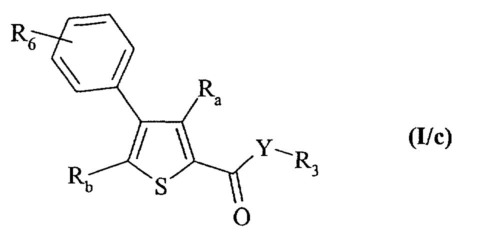 dans lesquels R₆,Rₐ, R_{b,} Y et R₃ sont tels que définis précédemment,
o soit être traités par du bis(pinacolato)di-borane suivi par une réaction d'oxydation, pour conduire aux composés de formule (XI) : 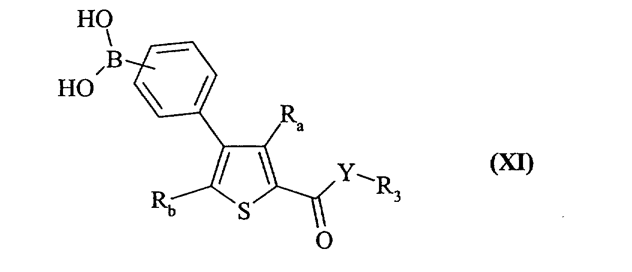
dans lesquels R_{a,} R_{b,} Y et R₃ sont tels que définis précédemment,
composés de formule (XI) qui sont mis à réagir en condition basique et en présence d'un catalyseur au palladium avec un composé de formule (Xa) :
R₆―P₂ (Xa)
dans laquelle R₆ est tel que défini dans la formule (I), c'est à dire qu'il représente un groupement choisi parmi aryle, cycloalkyle, et un hétérocycle, et P₂ représente un atome d'halogène ou un groupement triflate,
pour conduire également aux composés de formule (I/c), tels que définis précédemment : 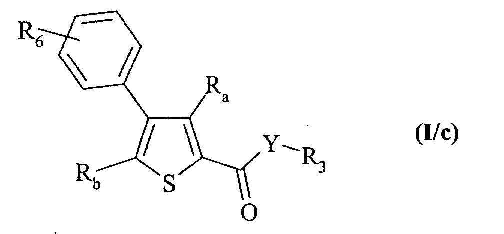 dans lesquels R_{6,}R_{a,} R_{b,} Y et R₃ sont tels que définis précédemment,
o soit être traités avec de l'hexaméthyldiétain, en présence d'un catalyseur au palladium, pour conduire aux composés de formule (XIa) : 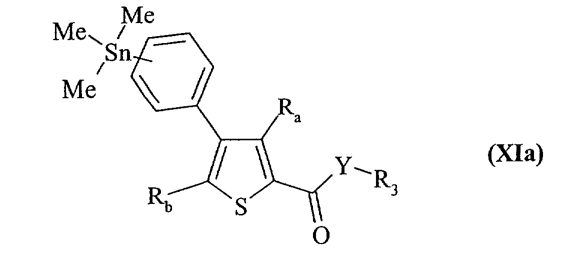
dans laquelle Rₐ, R_{b}, R₃ et Y sont tels que définis précédemment,
composés de formule (XIb) qui sont mis à réagir avec un composé de formule (Xa) tel que défini précédemment :
R₆―P₂ (Xa)
dans laquelle R₆ est tel que défini dans la formule (I), P₂ représente un atome d'halogène ou un groupement triflate,
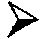 soit en présence de triphénylphosphine arsenic et d'un catalyseur au palladium, dans le cas où P₂ représente un groupement triflate,
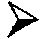 soit en présence d'un composé halogénocuivrique tel que CuBr₂ et d'un catalyseur au palladium, dans des conditions de solvant polaire, dans le cas où P₂ représente un atome d'halogène,
pour conduire également aux composés de formule (I/c), tels que définis précédemment : 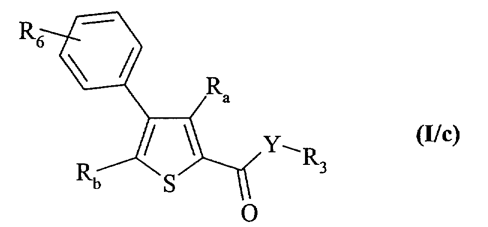 dans lesquels R₆,Rₐ, R_{b}, Y et R₃ sont tels que définis précédemment,
o soit être mis à réagir avec un composé de formule (Xb):
R₆―SnMe₃ (Xb)
dans laquelle R₆ est tel que défini précédemment,
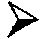 soit en présence de triphénylphosphine arsenic et d'un catalyseur au palladium, dans le cas où les composés de formule (I/b) comportent un groupement triflate,
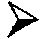 soit en présence d'un composé halogénocuivrique tel que CuBr₂ et d'un catalyseur au palladium, dans des conditions de solvant polaire, dans le cas où les composés de formule (I/b) comportent un atome d'halogène,
pour conduire également aux composés de formule (I/c) tels que décrits précédemment, 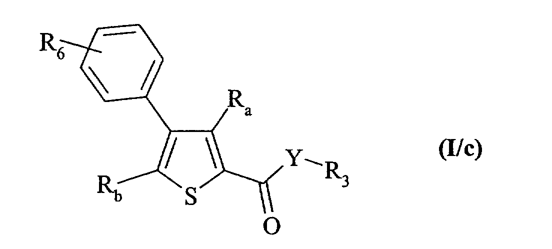 dans lesquels R₆,Rₐ, R_{b}, Y et R₃ sont tels que définis précédemment,
o soit être mis à réagir dans des conditions de couplage au palladium, en milieu basique, par un composé de formule (XII) :
R_{6'}-H (XII)
dans laquelle R_{6'} représente un groupement hétérocycle azoté éventuellement substitué par un ou plusieurs groupements tels qu'ils sont définis pour les substituants du groupement R₆ au sein des composés de formule (I),
pour conduire aux composés de formule (I/d), cas particulier des composés de formule (I): 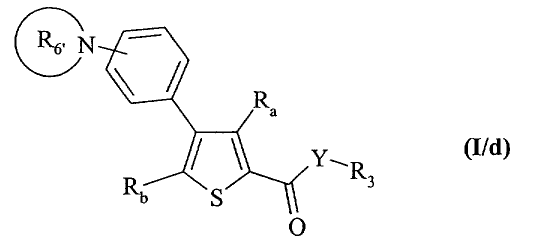 dans lesquels R_{a,} R_{b,} Y et R₃ sont tels que définis précédemment, et R_{6'} représente un hétérocycle azoté éventuellement substitué tel que défini dans la formule (I),
composés de formule (I/a), (I/b), (I/c) et (I/d) formant ensemble les composés de formule(I/e): 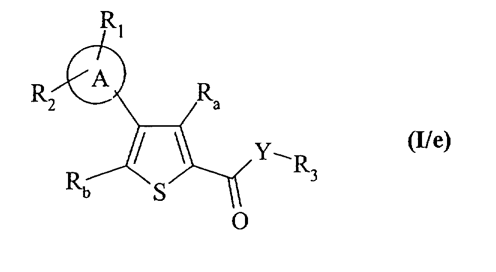 dans lesquels Rₐ, R_{b}, R₁, R₂, A, Y et R₃ sont tels que définis dans la formule (I),
composés de formule (I/e) qui peuvent être traités par exemple par du [2,4-bis(4-méthoxyphényl)1,3-dithia-2,4-diphosphetane-2,4-disulfide] pour conduire aux composés de formule (I/f), cas particulier des composés de formule (I): 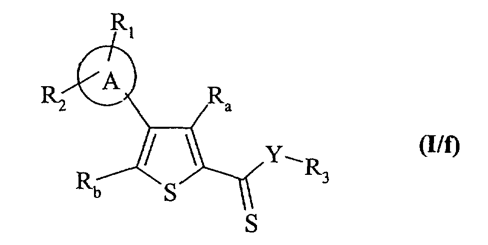 dans lesquels Rₐ, R_{b}, R₁, R_{2,} A, Y et R₃ sont tels que définis dans la formule (I),
les composés (I/a) à (I/f) formant l'ensemble des composés de l'invention, qui sont purifiés, le cas échéant, selon une technique classique de purification, qui peuvent, si on le désire, être séparés en leurs différents isomères selon une technique classique de séparation, et que l'on transforme, le cas échéant, en leurs sels d'addition à un acide ou à une base pharmaceutiquement acceptable.

**17.** Procédé de préparation des composés de formule (I) **caractérisé en ce qu'**on utilise comme produit de départ un composé de formule (II) : 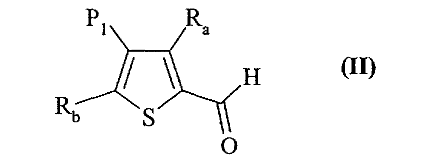 dans laquelle Rₐ et R_{b} sont tels que définis dans la formule (I) et P₁ représente un atome d'halogène ou un groupement triflate,
composés de formule (II) qui sont soumis à des conditions d'oxydations en milieu basique et polaire, pour donner les composés de formule (III): 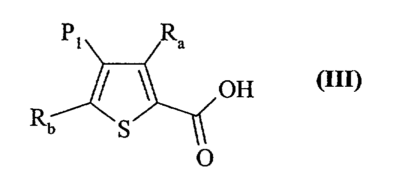 dans laquelle Rₐ, R_{b} et P₁ sont tels que définis précédemment,
composés de formule (III) dont la fonction acide est estérifiée par action d'un alcool en présence d'un acide fort, pour conduire aux composés de formule (XX): 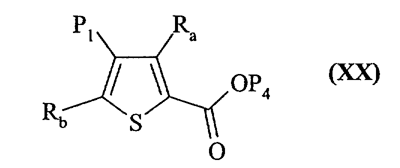 dans laquelle Rₐ, R_{b} et P₁ sont tels que définis précédemment, et P₄ représente un groupement (C₁-C₄)alkyle linéaire ou ramifié,
composés de formule (XX) qui sont :
• soit mis à réagir, dans des conditions basiques de couplage au palladium, avec un composé de formule (VII) : 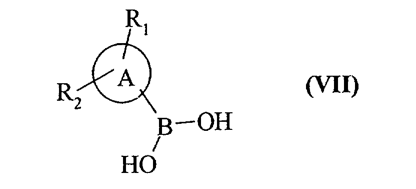
dans laquelle A, R₁ et R₂ ont les mêmes significations que dans les composés de formule (I),
pour conduire aux composés de formule (I/g), cas particulier des composés de formule (I): 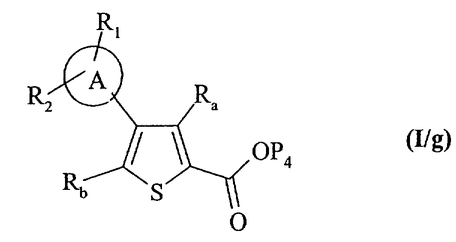 dans laquelle R_{a,} R_{b,} R₁, R_{2,} A et P₄ sont tels que définis précédemment,
• soit traités avec de l'hexaméthyldiétain, en présence d'un catalyseur au palladium, pour conduire aux composés de formule (XXII): 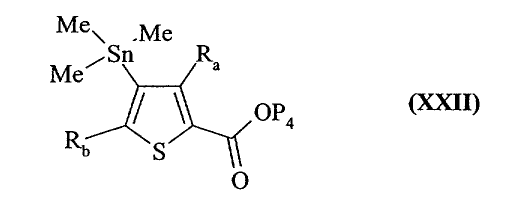
dans laquelle Rₐ, R_{b} et P₄ sont tels que définis précédemment, composés de formule (XXII) qui sont mis à réagir :
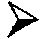 avec un composé de formule (IX) : 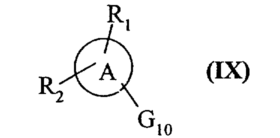
dans laquelle R₁, R₂ et A ont les mêmes significations que dans la formule (I) et G₁₀ représentent un atome d'halogène choisi parmi chlore et brome ou un groupement triflate,
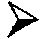 soit en présence de triphénylphosphine arsenic et d'un catalyseur au palladium, dans le cas où G₁₀ représente un groupement triflate,
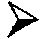 soit en présence d'un composé halogénocuivrique tel que CuBr₂ et d'un catalyseur au palladium, dans des conditions de solvant polaire, dans le cas où G₁₀ représente un atome d'halogène,
pour conduire également aux composés de formule (I/g) tels que décrits précédemment,
• soit traités par du bis(pinacolato)di-borane, suivi par une réaction d'oxydation, pour conduire aux composés de formule (XXI) : 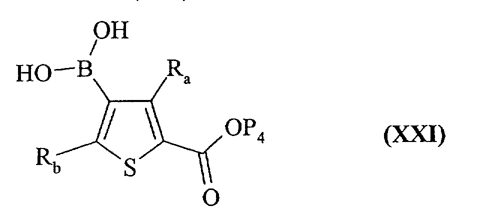
dans laquelle Rₐ, R_{b} et P₄ sont tels que définis précédemment,
composés de formule (XXI) qui sont mis à réagir dans des conditions basiques de couplage au palladium avec un composé de formule (IX) : 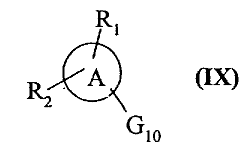 dans laquelle R₁, R₂, G₁₀ et A sont tels que définis précédemment,
pour conduire également aux composés de formule (I/g), tel que défini précédemment : 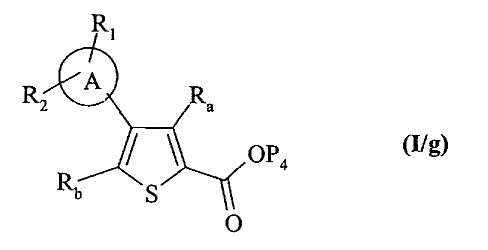 dans laquelle R_{a,} R_{b,} R_{1,} R₂ et P₄ sont tels que définis précédemment,
• soit mis à réagir avec un composé de formule (IXa): 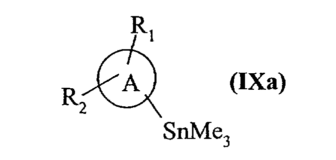
dans laquelle A, R₁, et R₂ sont tels que définis dans la formule (I),
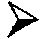 soit en présence de triphénylphosphine arsenic et d'un catalyseur au palladium, dans le cas où P₁ dans les composés de formule (XX) représente un groupement triflate,
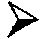 soit en présence d'un composé halogénocuivrique tel que CuBr₂ et d'un catalyseur au palladium, dans des conditions de solvant polaire, dans le cas où P₁ dans les composés de formule (XX) représente un atome d'halogène,
pour conduire également aux composés de formule (I/g) tels que décrits précédemment,
composés de formule (I/g) dans le cas particulier où ils représentent des composés de formule (I/h) dans lesquels A représente un groupement phényle, R₂ représente un atome d'hydrogène, R₁ représente un groupement hydroxy ou un atome d'halogène (Hal) et R_{a,} R_{b} et P₄ ont la même signification que dans la formule (I): 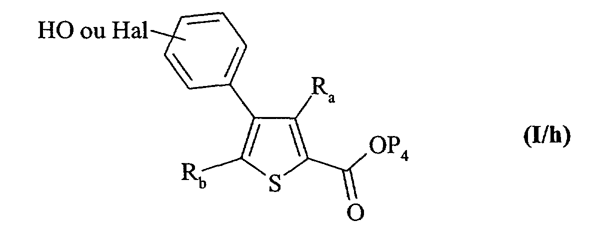 composés de formule (I/h) qui peuvent alors être traités préalablement par de l'anhydride trifluorométhanesulfonique en présence d'une base forte, dans le cas où R₁ représente un groupement hydroxy, pour obtenir le dérivé activé phényltriflate,
lesdits composés portant un groupement R₁ prenant la définition halogène ou triflate pouvant alors
o soit être mis à réagir en condition basique et en présence d'un catalyseur au palladium avec un composé de formule (X) : 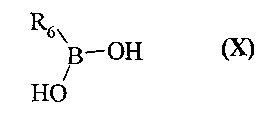
dans laquelle R₆ est tel que défini dans la formule (I), c'est à dire qu'il représente un groupement choisi parmi aryle, cycloalkyle, et un hétérocycle,
pour conduire aux composés de formule (I/i), cas particulier des composés de formule (I): 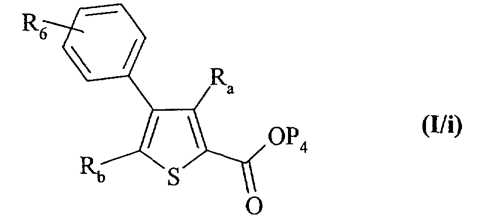 dans lesquels R_{6,} R_{a,} R_{b} et P₄ sont tels que définis précédemment,
o soit être traités par du bis(pinacolato)di-borane suivi par une réaction d'oxydation pour conduire aux composés de formule (XXIII) : 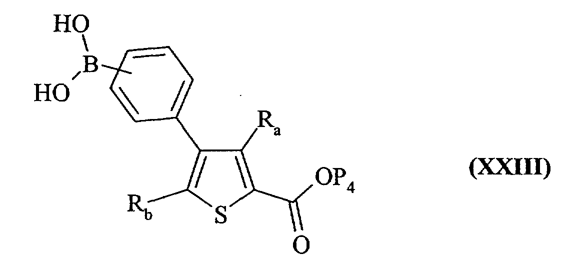
dans lesquels Rₐ, R_{b} et P₄ sont tels que définis précédemment,
composés de formule (XXIII) qui sont mis à réagir en condition basique et en présence d'un catalyseur au palladium avec un composé de formule (Xa) :
R₆―P₂ (Xa)
dans laquelle R₆ est tel que défini dans la formule (I), c'est à dire qu'il représente un groupement choisi parmi aryle, cycloalkyle, et un hétérocycle, et P₂ représente un atome d'halogène ou un groupement triflate,
pour conduire également aux composés de formule (I/i), tels que définis précédemment : 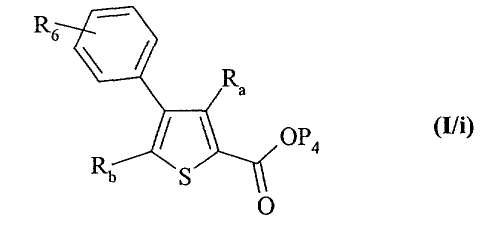 dans lesquels R_{b}, Rₐ, R_{b} et P₄ sont tels que définis précédemment,
o soit être traités avec de l'hexaméthyldiétain, en présence d'un catalyseur au palladium, pour conduire aux composés de formule (XXIV) : 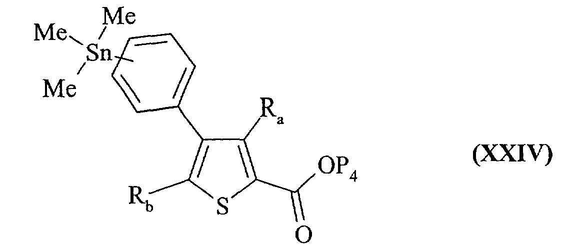
dans laquelle Rₐ, R_{b} et P₄ sont tels que définis précédemment,
composés de formule (XXIV) qui sont mis à réagir avec un composé de formule (Xa) tel que défini précédemment :
R₆―P₂ (Xa)
dans laquelle R₆ est tel que défini dans la formule (I), P₂ représente un atome d'halogène ou un groupement triflate,
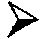 soit en présence de triphénylphosphine arsenic et d'un catalyseur au palladium, dans le cas où P₂ représente un groupement triflate,
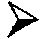 soit en présence d'un composé halogénocuivrique tel que CuBr₂ et d'un catalyseur au palladium, dans des conditions de solvant polaire, dans le cas où P₂ représente un atome d'halogène,
pour conduire également aux composés de formule (I/i), tels que définis précédemment : 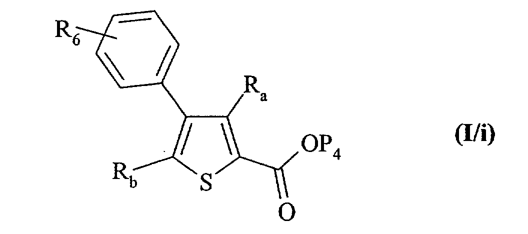 dans lesquels R_{6,}R_{a,} R_{b} et P₄ sont tels que définis précédemment,
o soit être mis à réagir avec un composé de formule (Xb):
R₆―SnMe₃ (Xb)
dans laquelle R₆ est tel que défini précédemment,
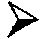 soit en présence de triphénylphosphine arsenic et d'un catalyseur au palladium, dans le cas où les composés de formule (I/h) comportent un groupement triflate,
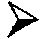 soit en présence d'un composé halogénocuivrique tel que CuBr₂ et d'un catalyseur au palladium, dans des conditions de solvant polaire, dans le cas où les composés de formule (I/h) comportent un atome d'halogène,
pour conduire également aux composés de formule (I/i) tels que décrits précédemment, 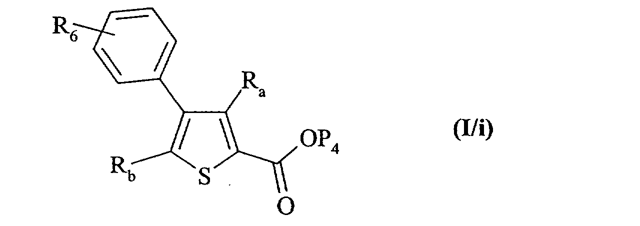 dans lesquels R_{6,}R_{a,} R_{b} et P₄ sont tels que définis précédemment,
composés de formule (I/g), (I/h) et (I/i) formant ensemble les composés de formule(I/j): 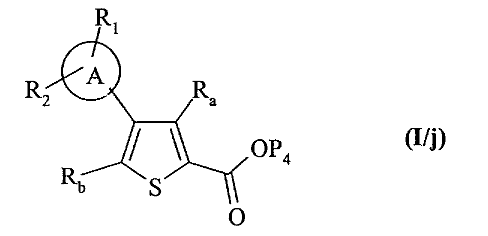 dans lesquels Rₐ, R_{b}, R₁, R₂, A et P₄ sont tels que définis précédemment,
composés de formule (I/i) qui sont saponifiés dans des conditions d'hydrolyse basique pour conduire aux composés de formule (I/k), cas particulier des composés de formule (I) 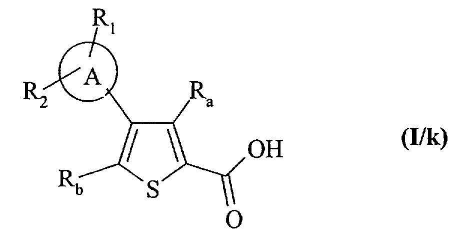 dans lesquels Rₐ, R_{b}, R₁, R₂ et A sont tels que définis dans la formule (I),
composés de formule (I/k) qui sont traités directement, dans des conditions de couplage peptidique en présence par exemple d'un agent de couplage et dans un milieu basique et apolaire, avec un composé de formule (V) :
HY-R₃ (V)
dans laquelle Y et R₃ ont les mêmes significations que dans les composés de formule (I), pour conduire aux composés de (1/l), cas particulier des composés de formule (I): 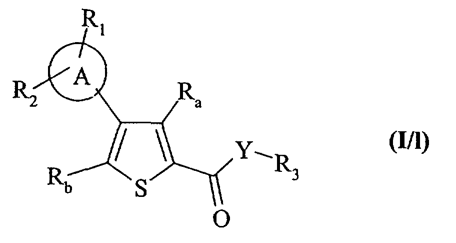 dans laquelle R₁, R₂, Rₐ, R_{b}, R₃ et Y sont tels que définis précédemment,
composés de formule (I/l) qui peuvent être traités par exemple par du [2,4-bis(4-méthoxyphényl) 1,3-dithia-2,4-diphosphetane-2,4-disulfide] pour conduire aux composés de formule (I/m), cas particulier des composés de formule (I): 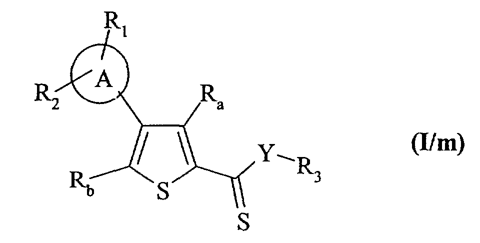 dans lesquels Rₐ, R_{b}, R₁, R₂, A, Y et R₃ sont tels que définis dans la formule (I),
les composés (I/g) à (I/m) formant ensemble des composés de l'invention, qui sont purifiés, le cas échéant, selon une technique classique de purification, qui peuvent, si on le désire, être séparés en leurs différents isomères selon une technique classique de séparation, et que l'on transforme, le cas échéant, en leurs sels d'addition à un acide ou à une base pharmaceutiquement acceptable.

**18.** Compositions pharmaceutiques contenant comme principe actif au moins un composé de formule (I) selon l'une quelconque des revendications 1 à 15, seul ou en combinaison avec un ou plusieurs excipients ou véhicules inertes, non toxiques, pharmaceutiquement acceptables

**19.** Compositions pharmaceutiques selon la revendication 18 utiles pour la prévention ou le traitement des pathologies nécessitant l'action d'un inhibiteur de métalloprotéinase-12.

**20.** Compositions pharmaceutiques selon la revendication 18 utiles pour la prévention ou le traitement des pathologies respiratoires choisies parmi les maladies pulmonaires obstructives chroniques (BPCO), l'emphysème, les bronchites chroniques, les inflammations pulmonaires chroniques, l'asthme, la mucoviscidose, la détresse respiratoire aiguë (ARDS), les allergies respiratoires dont la rhinite allergique, ainsi que les maladies liées à la production de TNFα incluant les maladies pulmonaires fibrotiques sévères, la sarcoïdose pulmonaire, et la silicose.

**21.** Compositions pharmaceutiques selon la revendication 18 utiles pour la prévention ou le traitement des maladies pulmonaires obstructives chroniques, de l'emphysème et des bronchites chroniques.

**22.** Compositions pharmaceutiques selon la revendication 18 utiles pour la prévention ou le traitement de l'emphysème lié au tabagisme.

**23.** Compositions pharmaceutiques selon la revendication 18 utiles pour la prévention ou le traitement de l'asthme.
